# EUROPEAN PATENT APPLICATION

(11) **EP 4 339 292 A1**
(43) Date of publication of application: **20.03.2024**
(21) Application number: 22807762.4
(22) Date of filing: 10.05.2022
(51) Int. Cl.: C12P 9/00, C12P 17/18, C12N 1/20, A23L 13/40, A23J 3/22, A23L 27/26

(54) **FOOD HAVING IMPROVED FLAVOR, NUTRITION, AND COLOR AND PREPARATION METHOD THEREFOR**

(30) Priority: 10.05.2021 KR 20210060113
(71) Applicant: Korea Advanced Institute of Science and Technology, Daejeon 34141 (KR)
(72) Inventor: LEE, Sang Yup, Daejeon 34141 (KR); CHOI, Kyeong Rok, Daejeon 34141 (KR); YU, Hyeeun, Daejeon 34141 (KR)
(74) Representative: Mewburn Ellis LLP
(86) International application number: PCT/KR2022/006611
(87) International publication number: WO 2022/240114

(57) **Abstract**

The present invention relates to a food having improved flavor, nutrition and color and a method for producing the same, and more specifically, to a food having a flavor, nutrition and color similar to those of meat, the food containing either a compound selected from the group consisting of heme, porphyrins, metalloporphyrins, and porphyrin analogs, or a microorganism that produces the compound, and a method for producing the same. According to the present invention, it is possible to improve the flavor, nutrition, and color of food by producing heme and various porphyrins, metalloporphyrins, and porphyrin analogs with high efficiency by fermentation of a microbial mutant, isolating/purifying the products, and adding the isolated/purified products to food.

## Description

### Technical Field

The present invention relates to a food having improved flavor, nutrition and color and a method for producing the same, and more specifically, to a food having a flavor, nutrition and color similar to those of meat, the food containing either a compound selected from the group consisting of heme, porphyrins, metalloporphyrins, and porphyrin analogs, or a microorganism that produces the compound, and a method for producing the same.

### Background Art

Porphyrins are macrocyclic compounds in which four pyrrole units are linked by methane groups to form a cyclic structure. Various types of porphyrin compounds are biosynthesized in most prokaryotes and eukaryotes, and more types of porphyrin compounds can be synthesized through chemical synthesis. In particular, porphyrins have conjugated double bonds in which alternating single and double bonds are present along the macrocycle. Therefore, they can absorb light having a wavelength in the visible wavelength range and can take on various colors. In addition, since the pair of electrons of the nitrogen atom located on the macrocycle faces inward, porphyrins can form coordinate bonds with various metal ions such as iron ions (Fe²⁺, Fe³⁺), magnesium ions (Mg²⁺), or cobalt ions (Co²⁺), thus forming metalloporphyrins such as heme, chlorophyll, and vitamin B12.

Thereamong, heme is found in most prokaryotes and eukaryotes, and is involved in various life phenomena such as transporting oxygen, removing reactive oxygen species, and generating energy by transferring electrons. Heme is used as a high-quality iron supplement with high absorption rates and for treatment of acute intermittent porphyria. In addition, in recent years, vegetable substitute meat having improved flavor and color as a result of adding heme-containing proteins or peptides has been developed and distributed (US 10863761 B2).

Meanwhile, meat substitutes are artificial meat made like real meat, and are broadly divided into meat made by culturing animal cells, and plant-based meat substitutes.

Meat made by culturing animal cells is human-edible meat made by culturing muscle stem cells of animals such as cows, pigs, and chickens, and has almost the same taste and flavor as real meat, but has the disadvantages of being time-consuming to make and expensive. Plant-based meat substitutes are based on vegetable protein and have advantages over animal cell-cultured meat in that they take less time to make and are inexpensive, but they have many shortcomings in actually giving the feeling of eating meat, because the taste, flavor, and texture thereof are very different from those of meat.

Meanwhile, the meat substitute market is expected to grow rapidly because of ecosystem destruction and global warming, which are caused by meat production, and controversy over animal cruelty. In addition, the increase in vegetarians who consume a vegetable-based diet is also a factor predicting the growth of the meat substitute market. Recently, companies that develop cell cultured meat and insect-based meat substitutes, in addition to animal cell-cultured meat and plant-based meat substitutes, have emerged one after another.

Technology to biosynthesize and extract heme-containing peptides or proteins from microorganisms or plants for use in the production of plant-based meat substitutes has been reported (EP 0,631,631; US 5,681,725; US2016-0340411). However, the ratio of the molecular weight of heme (0.6 kDa) relative to the molecular weights of heme-containing peptides or proteins (leghemoglobin, ~16 kDa; hemoglobin, ~65 kDa; and myoglobin, ~17 kDa) is very low. Thus, for use as an additive to enhance the flavor of a meat substitute, it is necessary to produce and isolate a relatively large amount of a heme-containing peptide/protein per unit mass of food. In addition, since the substance produced is in the form of a peptide or protein, the separation/purification process therefor is relatively difficult and relatively expensive.

Heme is known to have a higher absorption rate than inorganic iron compounds when ingested, and thus it is sold as a high-quality iron supplement. It has been reported that, when a heme extract or heme-producing microorganism mixed with water or feed is supplied to rats or pigs, it aids in weight gain or blood quality improvement (KR 10-1427075; and KR 10-2016-0145740).

Zinc (Zn)-protoporphyrin IX (ZnPPIX) is a reddish fluorescent compound which may be biosynthesized by competitively inserting zinc ions (Zn²⁺) instead of iron ions (Fe²⁺ or Fe³⁺) which are inserted into protoporphyrin IX by ferrochelatase (Labbe et al. Clinical Chemistry 45, 2060-2072, 1999). Therefore, zinc-protoporphyrin IX (ZnPPIX) may be used for diagnosing iron deficiency because its blood concentration increases when iron is deficient in the body (Labbe et al., Clinical Chemistry 45, 2060-2072, 1999). In addition, zinc-protoporphyrin IX is well known as an inhibitor of heme oxygenase-1 and has been reported to exhibit anticancer effects (Nowis et al., BMC Cancer 8, 97, 2008). In recent years, zinc-protoporphyrin IX has attracted attention as a natural pigment that can be used instead of nitrite or nitrate, which is known to be harmful to health, when manufacturing meat products. However, there has been no report on the production of zinc-protoporphyrin (ZnPPIX) using microorganisms, animals or plants.

Zn-coproporphyrin III (Zincphyrin) is also a fluorescent compound, and the natural biosynthesis thereof in microorganisms including *Streptomyces* sp. has been reported (Toriya et al., The Journal of Antibiotics 46, 196-200, 1993). It has been reported that Zn-coproporphyrin III can be used for anticancer treatment by photodynamic therapy because it promotes the production of reactive oxygen species when irradiated with light having a wavelength in a specific wavelength range (Yamamoto et al., BioMetals 16, 591-597, 2003) .

Until now, attempts have been made to improve the flavor of meat substitutes by adding heme bound to peptides or proteins, but there has been no attempt to produce meat substitutes having the same flavor and color as meat by adding free heme, heme-like porphyrins, metalloporphyrins, or porphyrin analogs, which are not bound to peptides or proteins, to meat substitutes.

Accordingly, the present inventors have fermented heme-producing microorganisms to obtain free heme, porphyrins, metalloporphyrins, and porphyrin analogs, which are not bound to peptides or proteins, and have found that, when the obtained products are added to meat substitutes, it is possible to obtain meat substitutes having a taste, nutrition and color similar to those of real meat, thereby completing the present invention.

### Summary of the Invention

An object of the present invention is to provide a method of producing a compound, selected from the group consisting of heme, porphyrins, metalloporphyrins, and porphyrin analogs, in a heme-producing microorganism.

Another object of the present invention is to provide a method of extracting a compound, selected from the group consisting of heme, porphyrins, metalloporphyrins, and porphyrin analogs, from heme-producing biomass.

Still another object of the present invention is to provide a method of recovering a compound, selected from the group consisting of heme, porphyrins, metalloporphyrins, and porphyrin analogs, from a culture supernatant of a heme-producing microorganism.

Yet another object of the present invention is to provide a food having a flavor similar to that of meat.

Still yet another object of the present invention is to provide a method for producing a meat substitute having a flavor similar to that of meat.

To achieve the above objects, the present invention provides a method for producing a compound selected from the group consisting of heme, porphyrins, metalloporphyrins, and porphyrin analogs, the method comprising a step of producing a compound selected from the group consisting of heme, porphyrins, metalloporphyrins, and porphyrin analogs by culturing a heme-producing microorganism having a heme synthesis pathway gene in a medium containing a carbon source.

The present invention also provides a food having a flavor similar to that of meat, the food containing a compound selected from the group consisting of heme, porphyrins, metalloporphyrins, and porphyrin analogs, obtained by fermentation of a heme-producing microorganism.

The present invention also provides a method for producing a meat substitute having a flavor similar to that of meat, the method comprising immersing a meat substitute in a solution containing: a compound selected from the group consisting of heme, porphyrins, metalloporphyrins, and porphyrin analogs, obtained by fermentation of a heme-producing microorganism; NaOH; and water.

The present invention also provides a food having a flavor similar to that of meat, the food containing an edible microorganism capable of producing a compound selected from the group consisting of heme, porphyrins, metalloporphyrins, and porphyrin analogs.

### Brief Description of Drawings

FIG. 1 shows the results of analyzing the concentrations of hemin, ZnPPIX, and PPIX after suspending the biomass of HAEM7 cells, cultured in a flask, in water, followed by extraction by adding 0.1 M NaOH-MeOH solution.
FIG. 2 shows the results of analyzing the concentrations of hemin, ZnPPIX, and PPIX after suspending the biomass of HAEM7 cells, cultured in a flask, in water, followed by extraction by adding 0.1 M NaOH-EtOH solution.
FIG. 3 shows the results of analyzing the concentrations of hemin, ZnPPIX, and PPIX after suspending the biomass of HAEM7 cells, cultured in a flask, in water, followed by extraction by adding 0.1 M KOH-EtOH.
FIG. 4 shows the results of analyzing the concentrations of hemin, ZnPPIX, and PPIX after suspending the biomass of HAEM7 cells, cultured in a flask, in water, followed by extraction by adding NH₃-EtOH solution.
FIG. 5 shows the results of analyzing the concentrations of hemin, ZnPPIX, and PPIX after suspending the biomass of HAEM7 cells, cultured in a flask, in water, followed by extraction by adding DMSO.
FIG. 6 shows the results of analyzing the concentrations of hemin, ZnPPIX, and PPIX after suspending the biomass of HAEM7 cells, cultured in a flask, in 1 M NaOH solution or water, followed by extraction by adding the same volume of 1 M NaOH solution.
FIG. 7 shows the results of analyzing the concentrations of hemin, ZnPPIX, and PPIX after suspending the biomass of HAEM7 cells, cultured in a flask, in 1 M KOH solution or water, followed by extraction by adding the same volume of 1 M KOH.
FIG. 8 shows the results of analyzing the concentrations, after extraction, of hemin, ZnPPIX, and PPIX produced by flask culture of the HAEM7 strain in MR-Fe20 medium containing glucose or glycerol as a carbon source.
FIG. 9 shows the results of analyzing the concentrations, after extraction, of hemin, ZnPPIX, and PPIX produced by flask culture of the HAEM7 strain in MR-Fe20, MR-Fe50 or MR-Fe100 medium containing glycerol as a carbon source.
FIG. 10 shows the growth curve by fed-batch culture of the HAEM7 strain and the amounts of heme, PPIX and ZnPPIX produced by the fed-batch culture, according to the present invention.

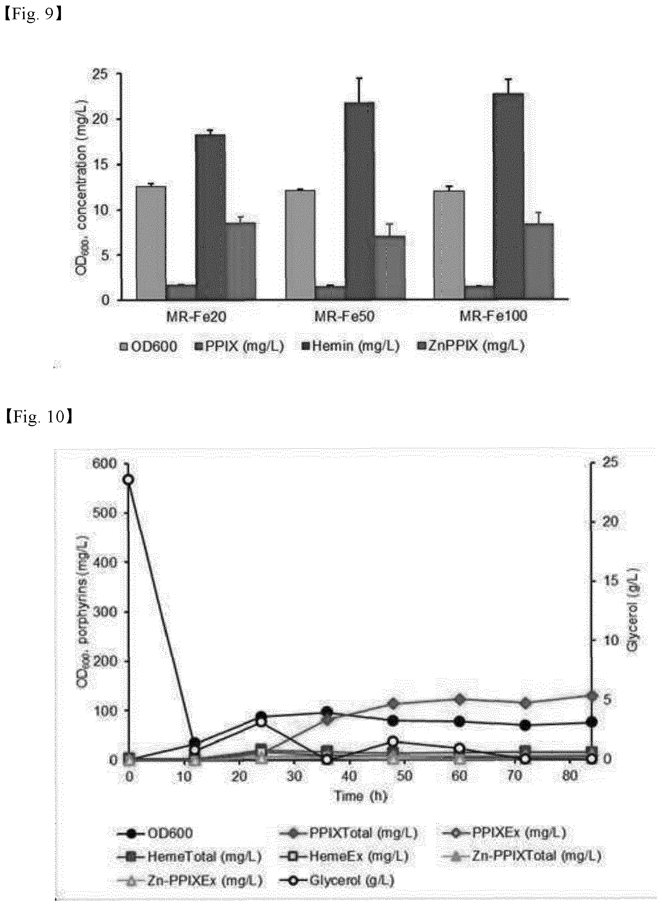

FIG. 10 shows the growth curve by fed-batch culture of the HAEM7 strain and the amounts of heme, PPIX and ZnPPIX produced by fed-batch culture, according to the present invention, and shows the results of IPTG induction when the OD₆₀₀ reached about 10 (8-hour time point) .

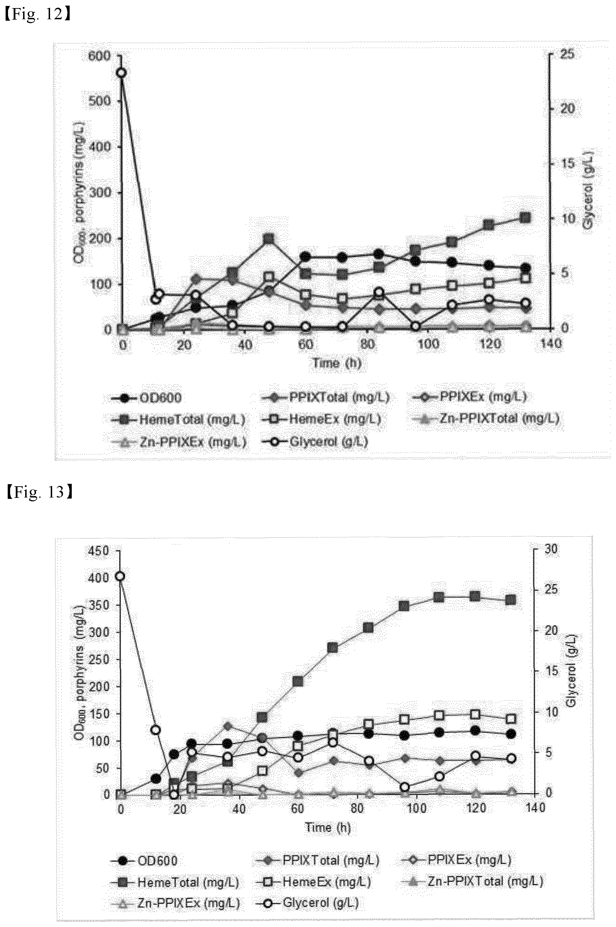

FIG. 12 shows the growth curve by fed-batch culture of the HAEM7 strain and the amounts of heme, PPIX and ZnPPIX produced by fed-batch culture, according to the present invention, and shows the results of IPTG induction when the OD₆₀₀ reached about 25 (11-hour time point) .
FIG. 13 shows the growth curve by fed-batch culture of the HAEM7 strain and the amounts of heme, PPIX and ZnPPIX produced by fed-batch culture, according to the present invention, and shows the results of IPTG induction when the OD₆₀₀ reached about 70 (18-hour time point) .

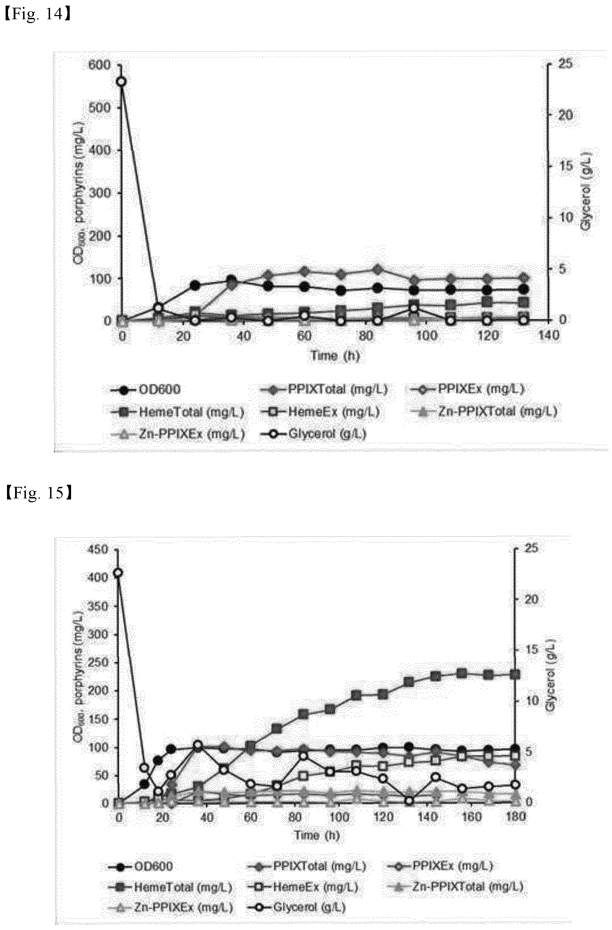

FIG. 14 shows the growth curve by fed-batch culture of the HAEM7 strain and the amounts of heme, PPIX and ZnPPIX produced by fed-batch culture, according to the present invention, and shows the results of IPTG induction when the OD₆₀₀ reached about 95 (36-hour time point) .
FIG. 15 shows the growth curve by fed-batch culture of the HAEM7 strain and the amounts of heme, PPIX and ZnPPIX produced by fed-batch culture, according to the present invention, and shows the results of fermentation performed by IPTG induction when the OD₆₀₀ reached about 70 (18-hour time point) and supplying a feeding solution (800 g/L glycerol, 8 g/L MgSO₄·7H₂O, 5 g/L (NH₄)₂SO₄, 0.5 g/L FeSO₄·7H₂O, 1 mM IPTG, 25 mg/L kanamycin, 50 mg/L ampicillin, 50 mg/L streptomycin, and 17 mg/L chloramphenicol) supplemented with 0.5 g/L FeSO₄·7H₂O.

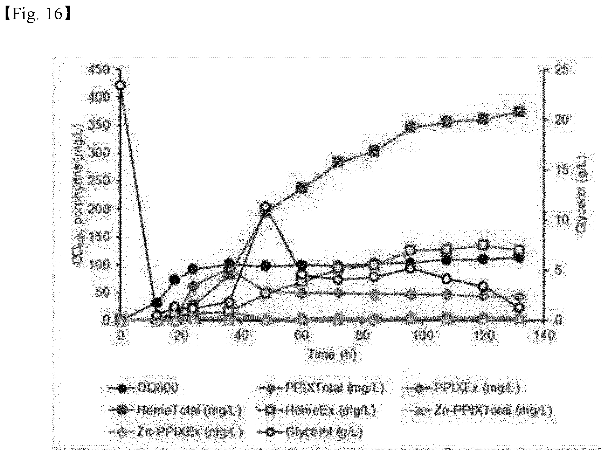

FIG. 16 shows the growth curve by fed-batch culture of the HAEM7 strain and the amounts of heme, PPIX and ZnPPIX produced by fed-batch culture, according to the present invention, and shows the results of fermentation performed by IPTG induction when the OD₆₀₀ reached about 70 (18-hour time point) and supplying a feeding solution (800 g/L glycerol, 8 g/L MgSO₄·7H₂O, 5 g/L (NH₄)₂SO₄, 2 g/L FeSO₄·7H₂O, 1 mM IPTG, 25 mg/L kanamycin, 50 mg/L ampicillin, 50 mg/L streptomycin, and 17 mg/L chloramphenicol) supplemented with 2 g/L FeSO₄·7H₂O.

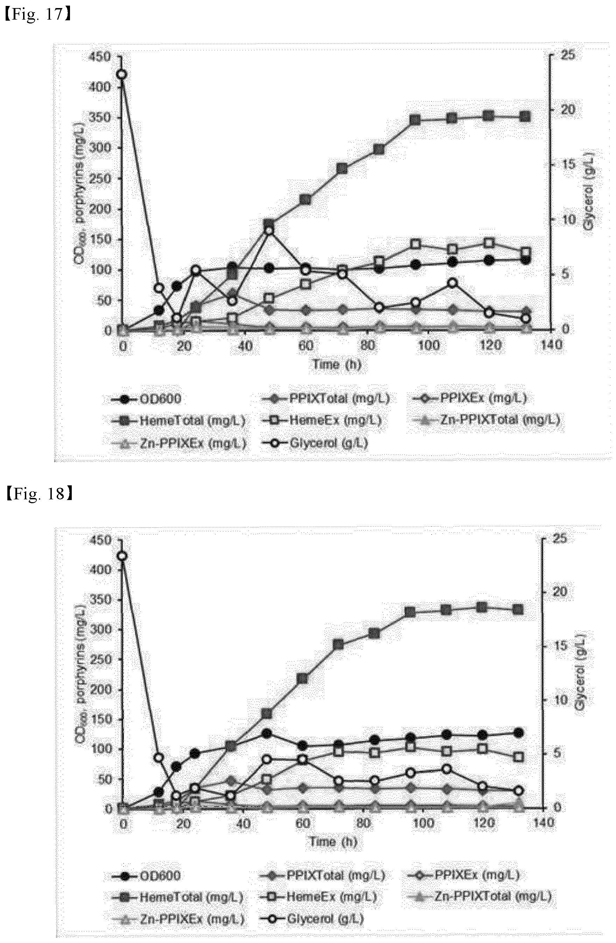

FIG. 17 shows the growth curve by fed-batch culture of the HAEM7 strain and the amounts of heme, PPIX and ZnPPIX produced by fed-batch culture, according to the present invention, and shows the results of fermentation performed by IPTG induction when the OD₆₀₀ reached about 70 (18-hour time point) and supplying a feeding solution (800 g/L glycerol, 8 g/L MgSO₄·7H₂O, 5 g/L (NH₄)₂SO₄, 4 g/L FeSO₄·7H₂O, 1 mM IPTG, 25 mg/L kanamycin, 50 mg/L ampicillin, 50 mg/L streptomycin, and 17 mg/L chloramphenicol) supplemented with 4 g/L FeSO₄·7H₂O.
FIG. 18 shows the growth curve by fed-batch culture of the HAEM7 strain and the amounts of heme, PPIX and ZnPPIX produced by fed-batch culture, according to the present invention, and shows the results of fermentation performed by IPTG induction when the OD₆₀₀ reached about 70 (18-hour time point) and supplying a feeding solution (800 g/L glycerol, 8 g/L MgSO₄·7H₂O, 5 g/L (NH₄)₂SO₄, 8 g/L FeSO₄·7H₂O, 1 mM IPTG, 25 mg/L kanamycin, 50 mg/L ampicillin, 50 mg/L streptomycin, and 17 mg/L chloramphenicol) supplemented with 8 g/L FeSO₄·7H₂O.

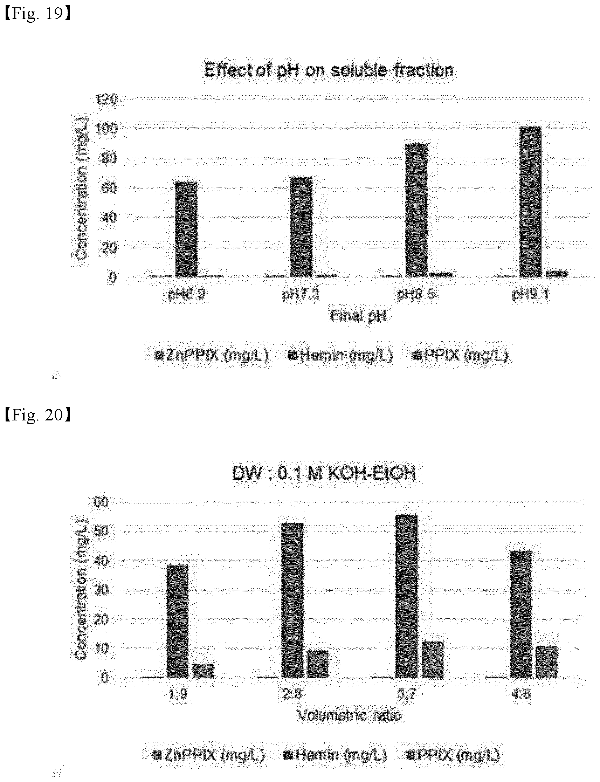

FIG. 19 shows the results of analyzing the concentrations of hemin, PPIX and ZnPPIX in a supernatant separated after adding 0 mL (pH 6.9), 0.1 mL (pH 7.3), 0.5 mL (pH 8.5) or 1 mL (pH 9.1) of 1 M NaOH per 10 mL of a HAEM7 fermentation broth.
FIG. 20 shows the results of analyzing the concentrations of hemin, PPIX and ZnPPIX recovered after suspending cells, recovered from a HAEM7 fermentation broth, in deionized water (DW), followed by extraction by adding various volumes of a solution of 0.1 M KOH in ethanol (KOH-EtOH) .

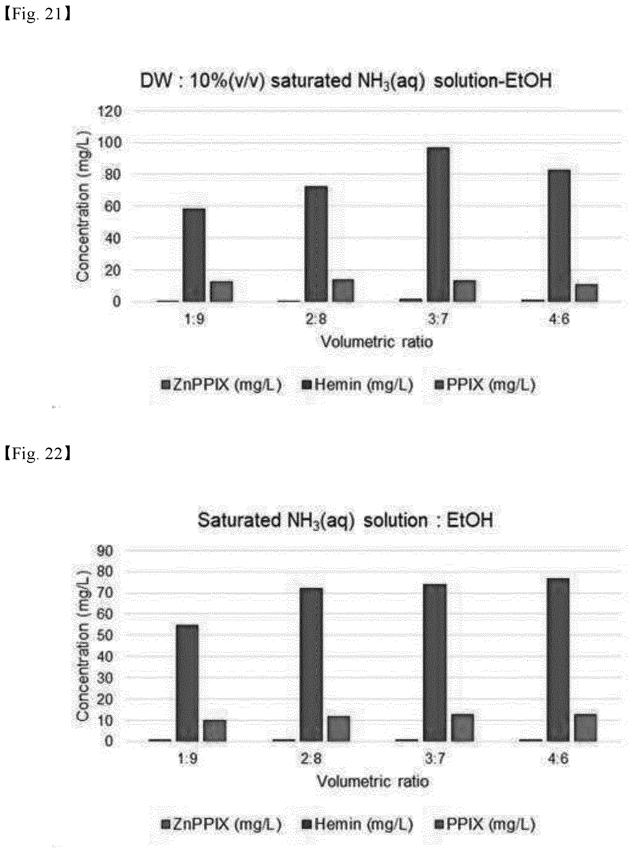

FIG. 21 shows the results of analyzing the concentrations of hemin, PPIX and ZnPPIX recovered after suspending cells, recovered from a HAEM7 fermentation broth, in deionized water (DW), followed by extraction by adding various volumes of NH₃-EtOH solution.
FIG. 22 shows the results of analyzing the concentrations of hemin, PPIX and ZnPPIX recovered after suspending cells, recovered from a HAEM7 fermentation broth, in deionized water (DW), followed by extraction by adding various volumes of NH₃-EtOH solution.

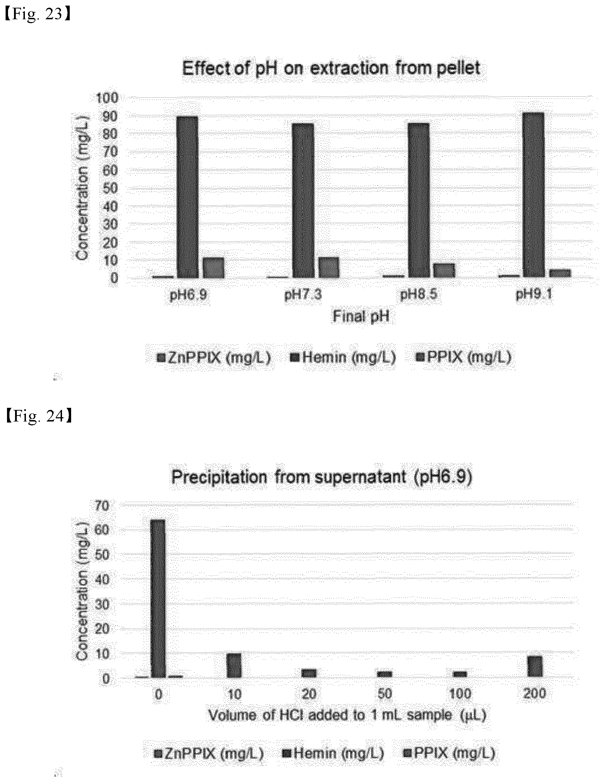

FIG. 23 shows the results of analyzing the concentrations of hemin, PPIX and ZnPPIX after adding 0 mL (pH 6.9), 0.1 mL (pH 7.3), 0.5 mL (pH 8.5) or 1 mL (pH 9.1) of 1 M NaOH per 10 mL of a HAEM7 fermentation broth, and then suspending cells, recovered from the fermentation broth, in 3 mL deionized water (DW), followed by extraction by adding 7 mL NH₃-EtOH solution.
FIG. 24 shows the results of analyzing the concentrations of hemin, PPIX and ZnPPIX in a supernatant recovered after adding 0 µL, 10 µL, 20 µL, 50 µL, 100 µL, or 200 µL of concentrated hydrochloric acid (HCl) per mL of a supernatant recovered from a HAEM7 fermentation broth.

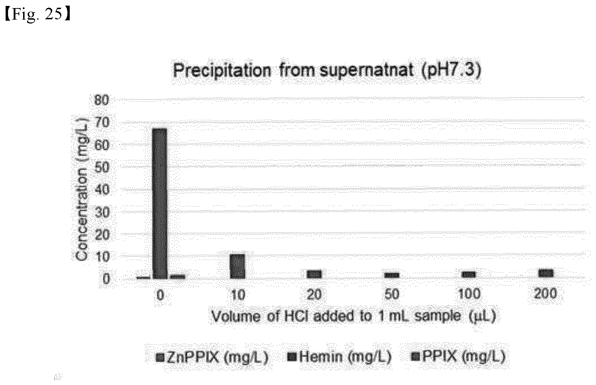

FIG. 25 shows the results of analyzing the concentrations of hemin, PPIX and ZnPPIX in a supernatant recovered after adding 0 µL, 10 µL, 20 µL, 50 µL, 100 µL, or 200 µL of concentrated hydrochloric acid (HCl) per mL of a supernatant recovered after adding 0.1 mL of 1 M NaOH aqueous solution per 10 mL of a HAEM7 fermentation broth.

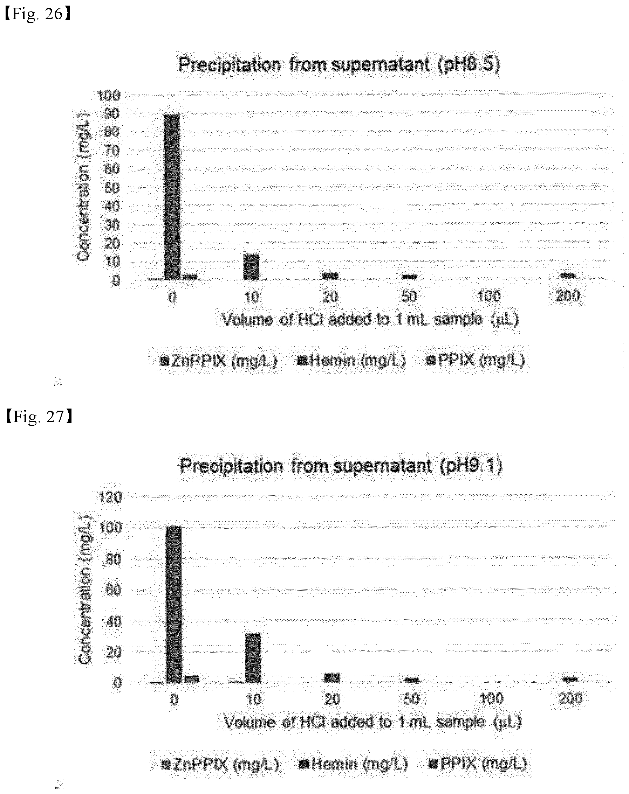

FIG. 26 shows the results of analyzing the concentrations of hemin, PPIX and ZnPPIX in a supernatant recovered after adding 0 µL, 10 µL, 20 µL, 50 µL, 100 µL, or 200 µL of concentrated hydrochloric acid (HCl) per mL of a supernatant recovered after adding 0.5 mL of 1 M NaOH aqueous solution per 10 mL of a HAEM7 fermentation broth.
FIG. 27 shows the results of analyzing the concentrations of hemin, PPIX and ZnPPIX in a supernatant recovered after adding 0 µL, 10 µL, 20 µL, 50 µL, 100 µL, or 200 µL of concentrated hydrochloric acid (HCl) per mL of a supernatant recovered after adding 1 mL of 1 M NaOH aqueous solution per 10 mL of a HAEM7 fermentation broth.

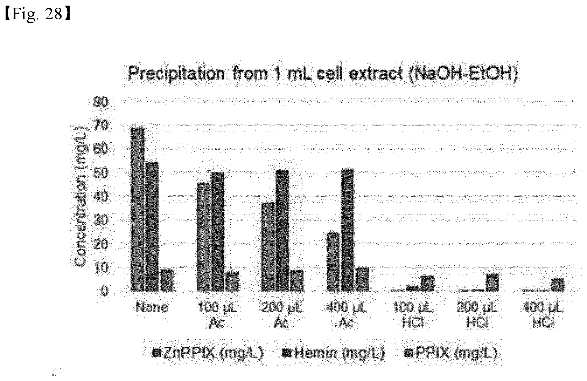

FIG. 28 shows the results of analyzing the concentrations of hemin, PPIX and ZnPPIX in a supernatant recovered after adding 100 µL, 200 µL or 400 µL of acetic acid (Ac) or concentrated hydrochloric acid (HCl) per 1 mL of an extract obtained by suspending cells, recovered after flask culture of HAEM7, in 2 mL DW and adding 8 mL NaOH-EtOH solution to the suspension.

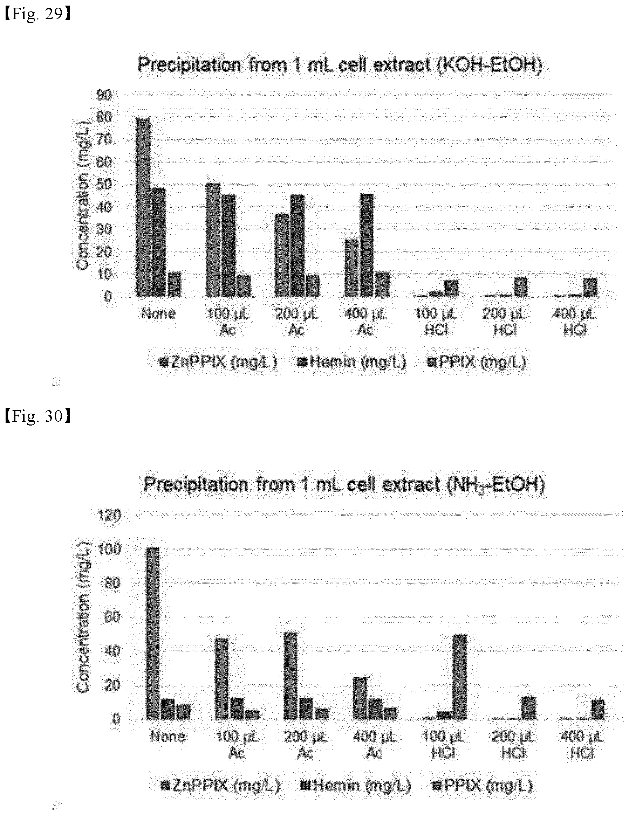

FIG. 29 shows the results of analyzing the concentrations of hemin, PPIX and ZnPPIX in a supernatant recovered after adding 100 µL, 200 µL or 400 µL of acetic acid (Ac) or concentrated hydrochloric acid (HCl) per 1 mL of an extract obtained by suspending cells, recovered after flask culture of HAEM7, in 2 mL DW and adding 8 mL KOH-EtOH solution to the suspension.
FIG. 30 shows the results of analyzing the concentrations of hemin, PPIX and ZnPPIX in a supernatant recovered after adding 100 µL, 200 µL or 400 µL of acetic acid (Ac) or concentrated hydrochloric acid (HCl) per 1 mL of an extract obtained by suspending cells, recovered after flask culture of HAEM7, in 2 mL DW and adding 8 mL NH₃-EtOH solution to the suspension.

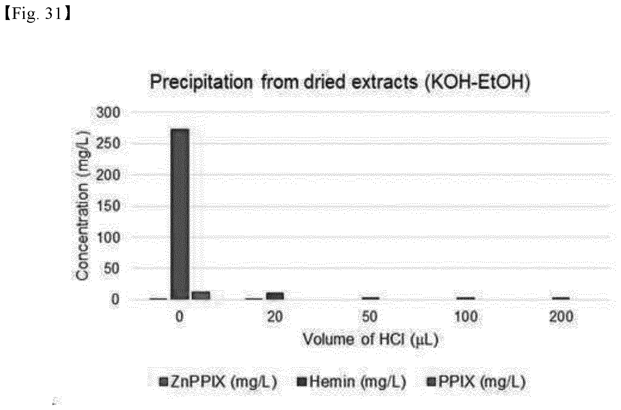

FIG. 31 shows the results of analyzing the concentrations of hemin, PPIX and ZnPPIX in a supernatant recovered after adding 0 µL, 20 µL, 50 µL, 100 µL or 200 µL of HCl per mL of a suspension obtained by suspending cells, recovered by adding 1 mL of 1 M NaOH per 10 mL of a HAEM7 fermentation broth, in 3 mL DW, and evaporating an extract obtained by adding 7 mL KOH-EtOH solution, followed by suspension in 3 mL DW.

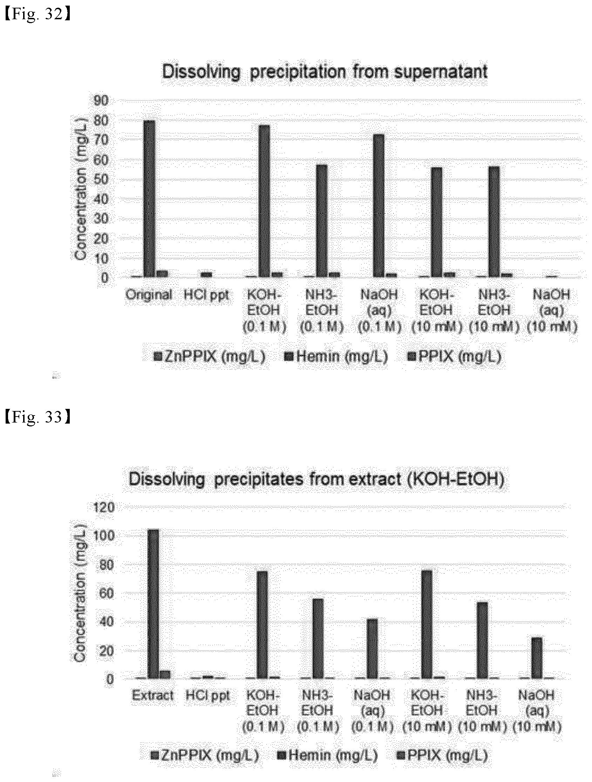

FIG. 32 shows the results of analyzing the concentrations of hemin, PPIX and ZnPPIX after adding 1 mL of 1 M NaOH aqueous solution per 10 mL of a HAEM7 fermentation broth, recovering a supernatant (Original) therefrom, adding 50 µL of concentrated hydrochloric acid (HCl) to the recovered supernatant, recovering a precipitate therefrom, and suspending the precipitate in 300 µL of 0.1 M NaOH (0.1 M) or 10 mM NaOH (10 mM), followed by extraction by adding KOH-EtOH, NH₃-EtOH, 0.1 M NaOH (aq) or 10 mM NaOH (aq). HCl ppt denotes a supernatant recovered by centrifugation after treating the supernatant (Original) of the fermentation broth, treated with 0.1 M NaOH, with HCl.
FIG. 33 shows the results of analyzing the concentrations of hemin, PPIX and ZnPPIX after adding 1 mL of 1 M NaOH aqueous solution per 10 mL of a HAEM7 fermentation broth, collecting cells therefrom, suspending the collected cells in 3 mL DW, adding 7 mL KOH-EtOH thereto to obtain an extract, 200 µL of concentrated hydrochloric acid (HCl) per 1 mL of the extract, recovering a precipitate therefrom, and suspending the precipitate in 300 µL of 0.1 M NaOH (0.1 M) or 10 mM NaOH (10 mM), followed by extraction by adding KOH-EtOH, NH₃-EtOH, 0.1 M NaOH (aq) or 10 mM NaOH (aq). HCl ppt denotes a supernatant recovered by centrifugation after treating the cell extract with HCl.

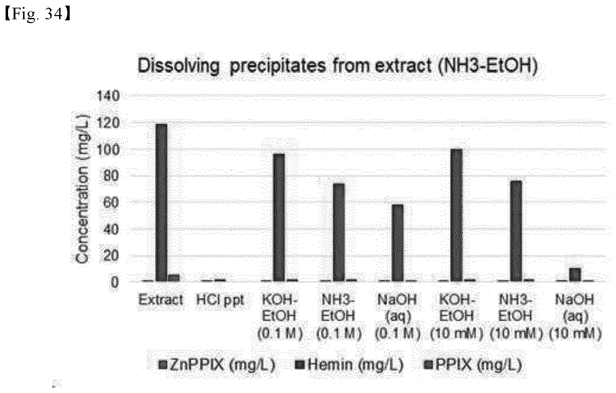

FIG. 34 shows the results of analyzing the concentrations of hemin, PPIX and ZnPPIX after adding 1 mL of 1 M NaOH aqueous solution per 10 mL of a HAEM7 fermentation broth, collecting cells therefrom, suspending the collected cells in 3 mL DW, adding 7 mL NH-₃-EtOH thereto to obtain an extract, adding 200 µL of concentrated hydrochloric acid (HCl) per 1 mL of the extract, recovering a precipitate therefrom, and suspending the precipitate in 300 µL of 0.1 M NaOH (0.1 M) or 10 mM NaOH (10 mM), followed by extraction by adding KOH-EtOH, NH₃-EtOH, 0.1 M NaOH (aq) or 10 mM NaOH (aq). HCl ppt denotes a supernatant recovered by centrifugation after treating the cell extract with HCl.

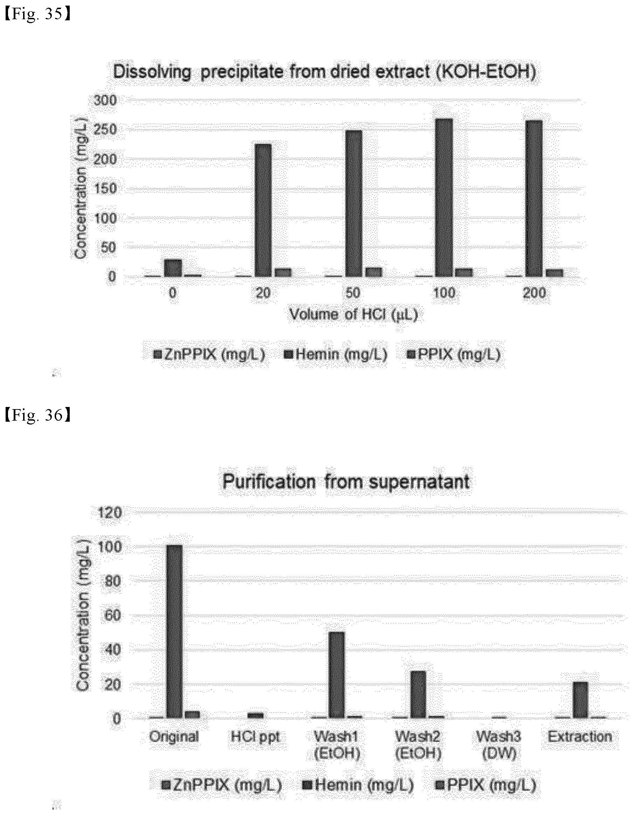

FIG. 35 shows the results of analyzing the concentrations of hemin, PPIX and ZnPPIX after adding 1 mL of 1 M NaOH aqueous solution per 10 mL of a HAEM7 fermentation broth, collecting cells therefrom, suspending the collected cells in 3 mL DW, adding 7 mL KOH-EtOH solution thereto to obtain an extract, evaporating the extract, suspending the residue in 3 mL DW, adding 0 µL, 20 µL, 50 µL, 100 µL or 200 µL of HCl to 1 mL of the suspension, recovering a precipitate therefrom, and suspending the precipitate in 3 mL DW, followed by extraction by adding 7 mL KOH-EtOH solution.
FIG. 36 shows the results of analyzing the concentrations of hemin, PPIX and ZnPPIX in a supernatant obtained by adding 1 mL of 1 M NaOH aqueous solution per 10 mL of a HAEM7 fermentation broth, collecting a supernatant (Original) therefrom, adding 50 µL of concentrated hydrochloric acid (HCl) per mL of the supernatant, recovering a precipitate therefrom, and washing the precipitate sequentially with 1 ml EtOH (Wash 1), 1 mL EtOH (Wash 2) and 1 mL DW (Wash 3). HCl ppt denotes a supernatant recovered by centrifugation after treating the supernatant (Original) of the fermentation broth, treated with 0.1 M NaOH, with HCl, and Extraction denotes a solution sample obtained by suspending the precipitate remaining after washing in 300 µL of 0.1 M NaOH and then adding 700 µL KOH-EtOH solution thereto.

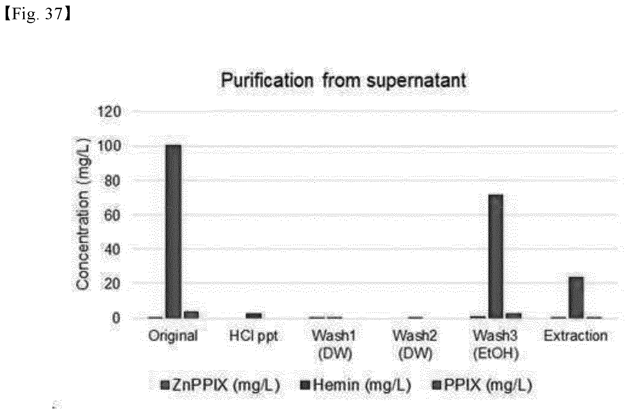

FIG. 37 shows the results of analyzing the concentrations of hemin, PPIX and ZnPPIX in a supernatant obtained by adding 1 mL of 1 M NaOH aqueous solution per 10 mL of a HAEM7 fermentation broth, collecting a supernatant (Original) therefrom, adding 50 µL of concentrated hydrochloric acid (HCl) per mL of the supernatant, recovering a precipitate therefrom, and washing the precipitate sequentially with 1 ml DW (Wash 1), 1 mL DW (Wash 2) and 1 mL EtOH (Wash 3). HCl ppt denotes a supernatant recovered by centrifugation after treating the supernatant (Original) of the fermentation broth, treated with 0.1 M NaOH, with HCl, and Extraction denotes a solution sample obtained by suspending the precipitate remaining after washing in 300 µL of 0.1 M NaOH and then adding 700 µL KOH-EtOH solution thereto.

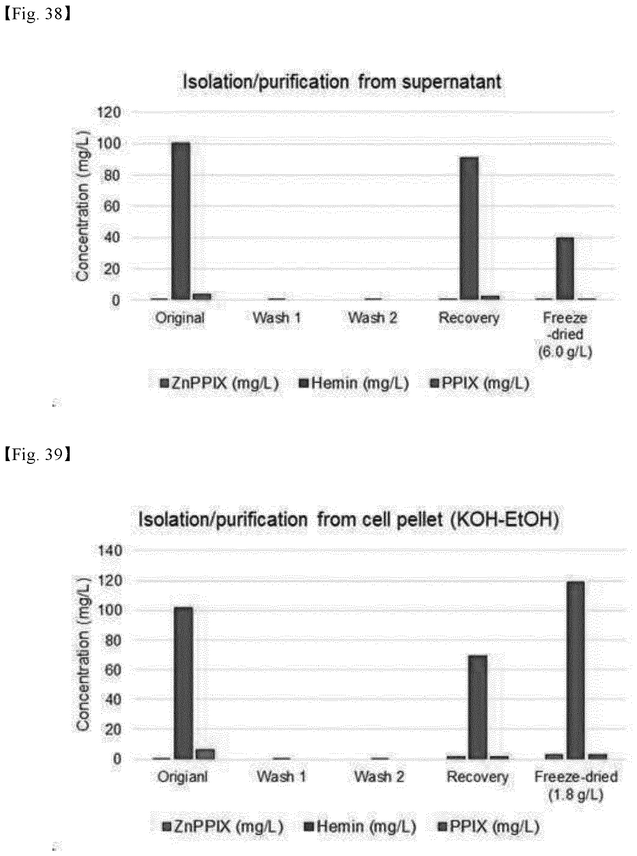

FIG. 38 shows the results of analyzing the concentrations of hemin, PPIX and ZnPPIX in the supernatants (Wash 1 and Wash 2) and precipitate (Recovery) obtained by adding 1 mL of 1 M NaOH aqueous solution to 10 mL of a HAEM7 fermentation broth, collecting a supernatant (Original) therefrom, adding 0.5 mL of concentrated hydrochloric acid (HCl) per 10 mL of the supernatant, recovering a precipitate therefrom, and washing the precipitate twice with 5 mL DW. It also includes the concentrations of hemin, PPIX, and ZnPPIX when the obtained precipitate was freeze-dried and then re-dissolved at a concentration of 6.0 g/L [Freeze-dried (6.0 g/L)].
FIG. 39 shows the results of analyzing the concentrations of hemin, PPIX and ZnPPIX in the supernatants (Wash 1 and Wash 2) and precipitate (Recovery) obtained by adding 2 mL of concentrated hydrochloric acid (HCl) to 10 mL of a KOH-EtOH-based HAEM7 cell extract (Original), recovering a precipitate therefrom, and washing the precipitate twice with 5 mL DW. It also includes the concentrations of hemin, PPIX, and ZnPPIX when the obtained precipitate was freeze-dried and then re-dissolved at a concentration of 1.8 g/L [Freeze-dried (1.8 g/L)].

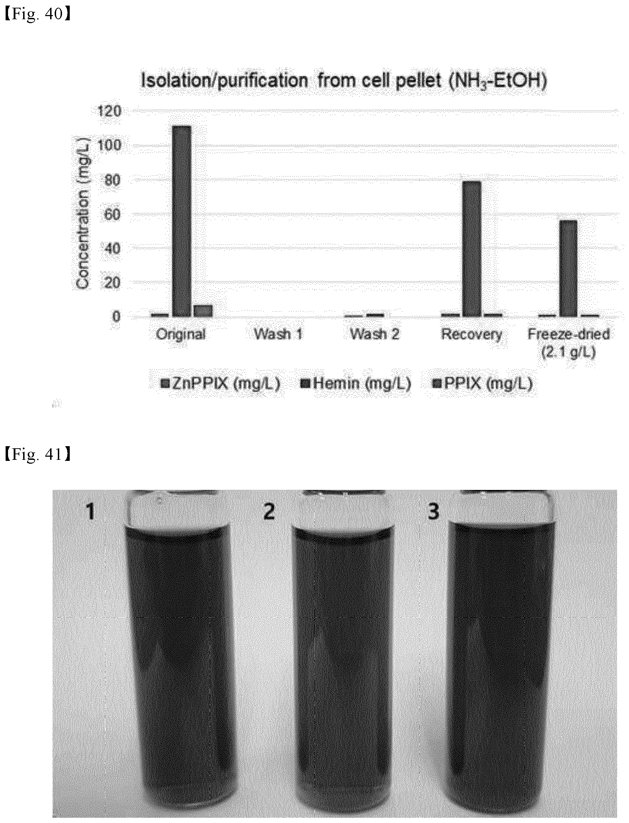

FIG. 40 shows the results of analyzing the concentrations of hemin, PPIX and ZnPPIX in the supernatants (Wash 1 and Wash 2) and precipitate (Recovery) obtained by adding 2 mL of concentrated hydrochloric acid (HCl) to 10 mL of a NH₃-EtOH-based HAEM7 cell extract (Original), recovering a precipitate therefrom, and washing the precipitate twice with 5 mL DW. It also includes the concentrations of hemin, PPIX, and ZnPPIX when the obtained precipitate was freeze-dried and then re-dissolved at a concentration of 2.1 g/L [Freeze-dried (2.1 g/L)].
FIG. 41 shows the appearances of hemin-NaOH-water solution (1), hemin-NH₃-water solution (2), and hemin-NH₃-EtOH solution (3), prepared by dissolving about 10 mg of hemin in about 50 mL of dilute NaOH aqueous solution, dilute aqueous ammonia, and dilute ammonia-ethanol solution, respectively.

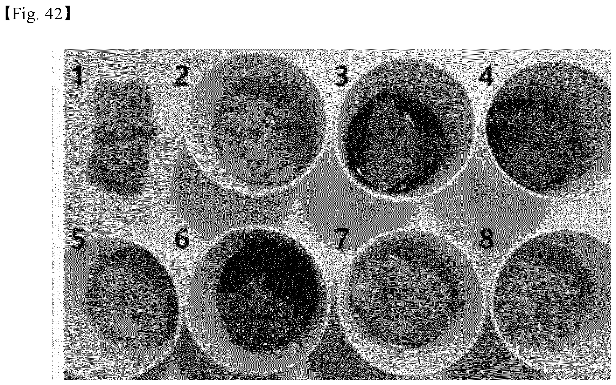

FIG. 42 shows the appearances of unsoaked soy meat (1), soy meat soaked in water (2), soy meat soaked in hemin-NaOH-water solution (3), soy meat soaked in hemin-NH₃-water solution (4), soy meat soaked in ethanol after soaking in water (5), soy meat soaked in hemin-NH₃-EtOH solution after soaking in water (6), soy meat soaked in water after soaking in ethanol (7), and soy meat soaked in water after soaking in hemin-NH₃-EtOH solution (8), sequentially from the left in the upper row.

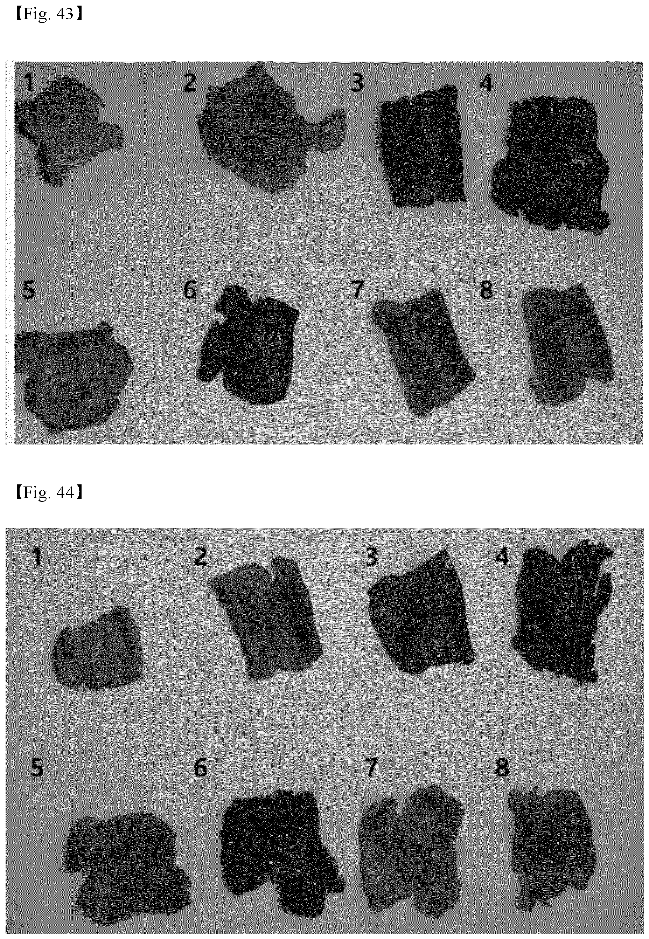

FIG. 43 shows the appearances after cooking of unsoaked soy meat (1), soy meat soaked in water (2), soy meat soaked in hemin-NaOH-water solution (3), soy meat soaked in hemin-NH₃-water solution (4), soy meat soaked in ethanol after soaking in water (5), soy meat soaked in hemin-NH₃-EtOH solution after soaking in water (6), soy meat soaked in water after soaking in ethanol (7), and soy meat soaked in water after soaking in hemin-NH₃-EtOH solution (8), sequentially from the left in the upper row.
FIG. 44 shows the appearances after cooking of unsoaked soy meat (1), soy meat soaked in water (2), soy meat soaked in hemin-NaOH-water solution (3), soy meat soaked in hemin-NH₃-water solution (4), soy meat soaked in ethanol after soaking in water (5), soy meat soaked in hemin-NH₃-EtOH solution after soaking in water (6), soy meat soaked in water after soaking in ethanol (7), and soy meat soaked in water after soaking in hemin-NH₃-EtOH solution (8), sequentially from the left in the upper row.

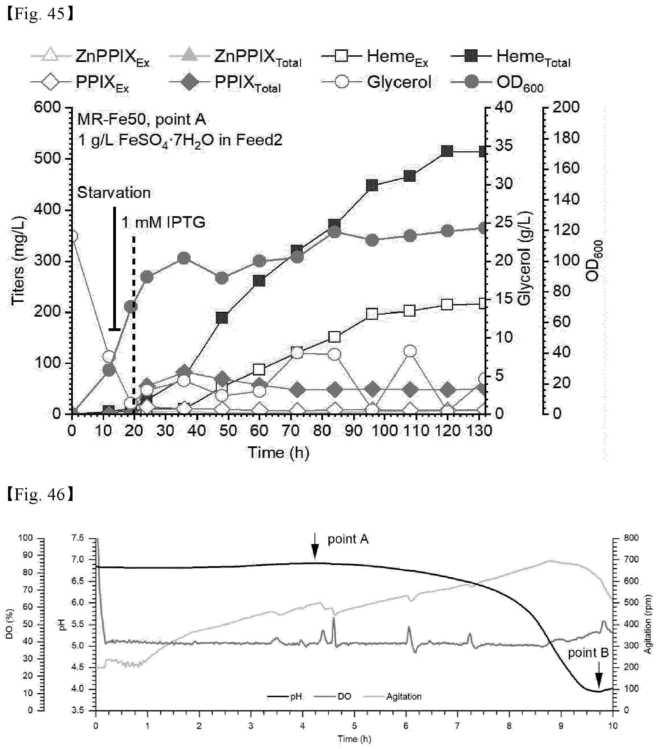

FIG. 45 shows the growth curve by fed-batch culture of the HAEM7 strain using MR-Fe50 medium and the amounts of heme, PPIX and ZnPPIX produced by the fed-batch culture, and shows the results of IPTG induction when the OD₆₀₀ reached about 70 after temporarily blocking the supply of a feeding solution between 13 hours and 14 hours.
FIG. 46 shows the pH, DO and agitation profiles observed when fermentation proceeded without adjusting the pH by the supply of aqueous ammonia during fed-batch culture of the HAEM7 strain using MR-Fe50 medium.

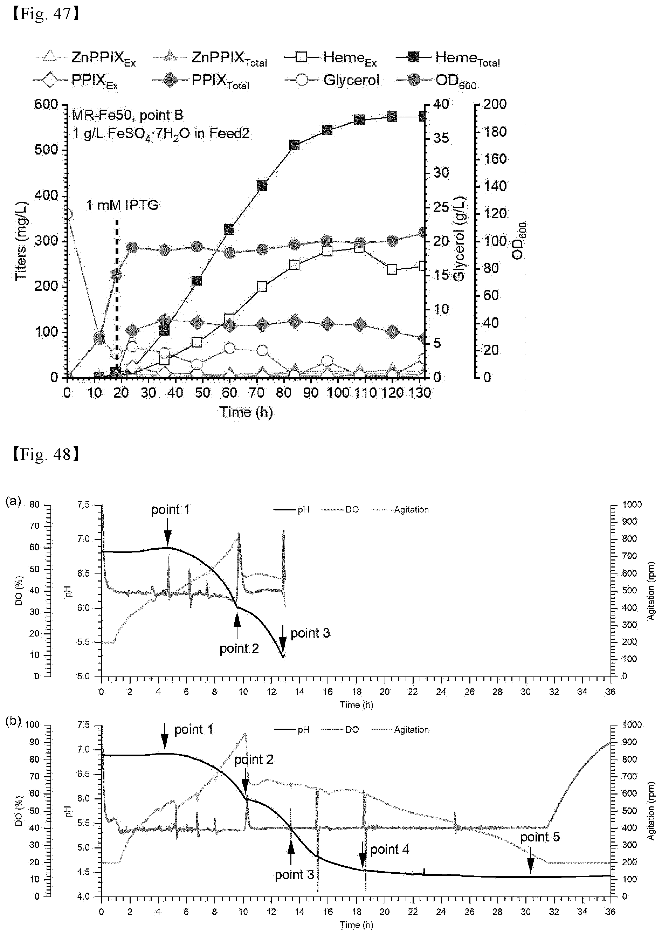

FIG. 47 shows the growth curve by fed-batch culture of the HAEM7 strain using MR-Fe50 medium and the amounts of heme, PPIX and ZnPPIX produced by the fed-batch culture, and shows the results of IPTG induction when the OD₆₀₀ reached about 70 after the start of pH control at point B in FIG. 46.
FIG. 48 shows the pH, DO and agitation profiles observed when fermentation proceeded without adjusting the pH by the supply of aqueous ammonia during fed-batch culture of the HAEM7 strain using R-Fe100 medium. FIG. 48(a) shows the profiles observed when a feeding solution was not supplied before the start of pH control, and FIG. 48(b) shows the profiles observed when a feeding solution was supplied whenever a rapid increase in the DO value was observed.

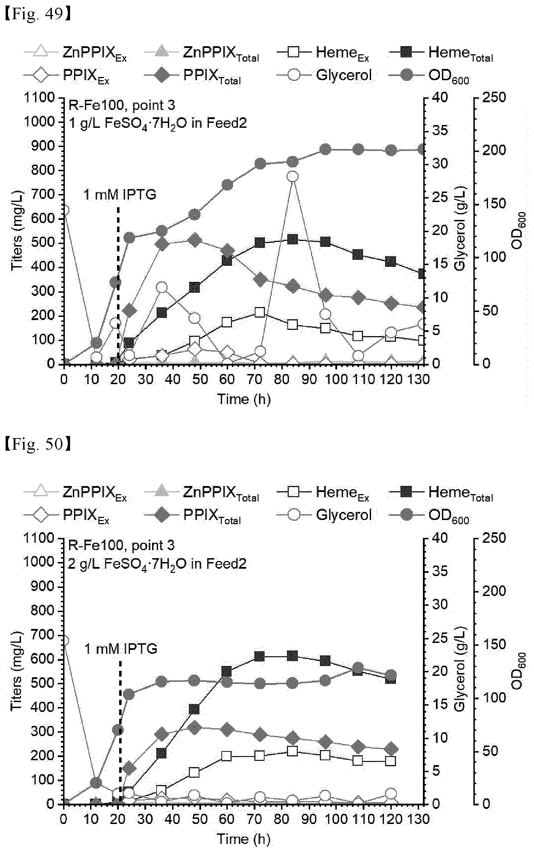

FIG. 49 shows the growth curve by fed-batch culture of the HAEM7 strain using MR-Fe50 medium and the amounts of heme, PPIX and ZnPPIX produced by the fed-batch culture, and shows the results of supplying a feeding solution supplemented with 1 g/L FeSO₄·7H₂O after IPTG induction when the OD₆₀₀ reached about 70 after the start of pH control at point 3 in FIG. 48.
FIG. 50 shows the growth curve by fed-batch culture of the HAEM7 strain using R-Fe100 medium and the amounts of heme, PPIX and ZnPPIX produced by the fed-batch culture, and shows the results of supplying a feeding solution supplemented with 2 g/L FeSO₄·7H₂O after IPTG induction when the OD₆₀₀ reached about 70 after the start of pH control at point 3 in FIG. 48.

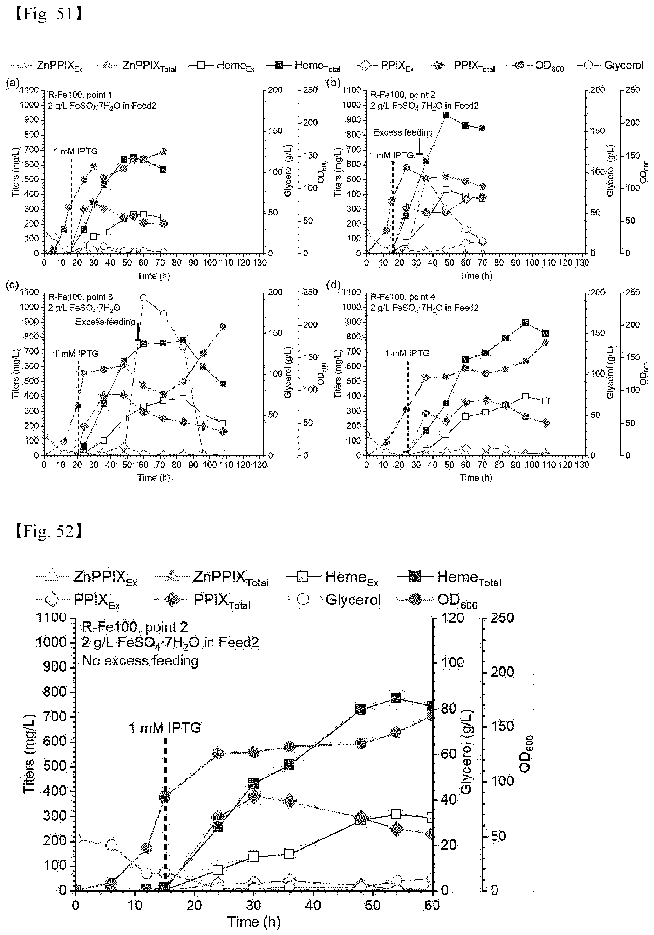

FIG. 51 shows the growth curve by fed-batch culture of the HAEM7 strain using R-Fe100 medium and the amounts of heme, PPIX and ZnPPIX produced by the fed-batch culture, and shows the results of supplying a feeding solution supplemented with 2 g/L FeSO₄·7H₂O after IPTG induction when the OD₆₀₀ reached about 70 to 90 after the start of pH control at point 1 (a), point 2 (b), point 3 (c), and point 4 (d) in FIG. 48.
FIG. 52 shows the growth curve by fed-batch culture of the HAEM7 strain using R-Fe100 medium and the amounts of heme, PPIX and ZnPPIX produced by the fed-batch culture, and shows the results of supplying a feeding solution supplemented with 2 g/L FeSO₄·7H₂O while preventing excessive supply, after IPTG induction when the OD₆₀₀ reached about 70 to 90 after the start of pH control at point 2 in FIG. 48.

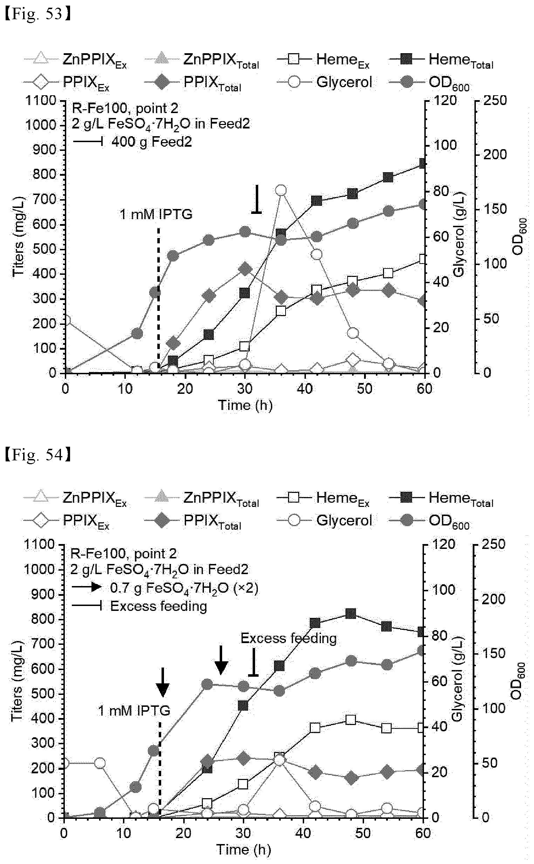

FIG. 53 shows the growth curve by fed-batch culture of the HAEM7 strain using R-Fe100 medium and the amounts of heme, PPIX and ZnPPIX produced by the fed-batch culture, and shows the results of supplying a feeding solution supplemented with 2 g/L FeSO₄·7H₂O after IPTG induction when the OD₆₀₀ reached 70 to 90 after the start of pH control at point 2 in FIG. 48, and oversupplying 400 g of the feeding solution between 30.5 hours and 33.5 hours.
FIG. 54 shows the growth curve by fed-batch culture of the HAEM7 strain using R-Fe100 medium and the amounts of heme, PPIX and ZnPPIX produced by the fed-batch culture, and shows the results of supplying a feeding solution supplemented with 2 g/L FeSO₄·7H₂O after IPTG induction when the OD₆₀₀ reached 70 to 90 after the start of pH control at point 2 in FIG. 48, and supplying an iron solution at 15 hours and 26.5 hours.

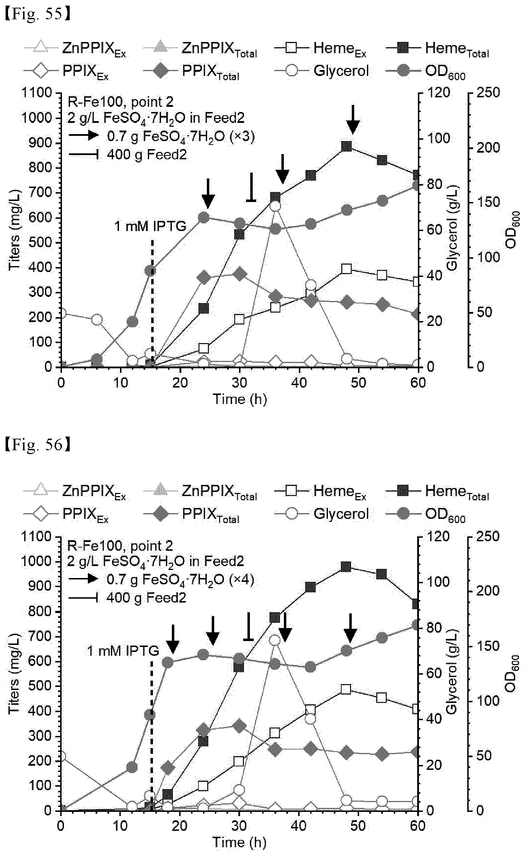

FIG. 55 shows the growth curve by fed-batch culture of the HAEM7 strain using R-Fe100 medium and the amounts of heme, PPIX and ZnPPIX produced by the fed-batch culture, and shows the results of supplying a feeding solution supplemented with 2 g/L FeSO₄·7H₂O after IPTG induction when the OD₆₀₀ reached 70 to 90 after the start of pH control at point 2 in FIG. 48, supplying an iron solution at 24.3, 36.5 and 48.5 hours, and oversupplying 400 g of the feeding solution between 30 hours and 33 hours.
FIG. 55 shows the growth curve by fed-batch culture of the HAEM7 strain using R-Fe100 medium and the amounts of heme, PPIX and ZnPPIX produced by the fed-batch culture, and shows the results of supplying a feeding solution supplemented with 2 g/L FeSO₄·7H₂O after IPTG induction when the OD₆₀₀ reached 70 to 90 after the start of pH control at point 2 in FIG. 48, supplying an iron solution at 24.3, 36.5 and 48.5 hours, and oversupplying 400 g of the feeding solution between 30 hours and 33 hours.
FIG. 56 shows the growth curve by fed-batch culture of the HAEM7 strain using R-Fe100 medium and the amounts of heme, PPIX and ZnPPIX produced by the fed-batch culture, and shows the results of supplying a feeding solution supplemented with 2 g/L FeSO₄·7H₂O after IPTG induction when the OD₆₀₀ reached 70 to 90 after the start of pH control at point 2 in FIG. 48, supplying an iron solution at 18.7, 24.3, 36.8 and 48.5 hours, and oversupplying 400 g of the feeding solution between 30.5 hours and 33.5 hours.

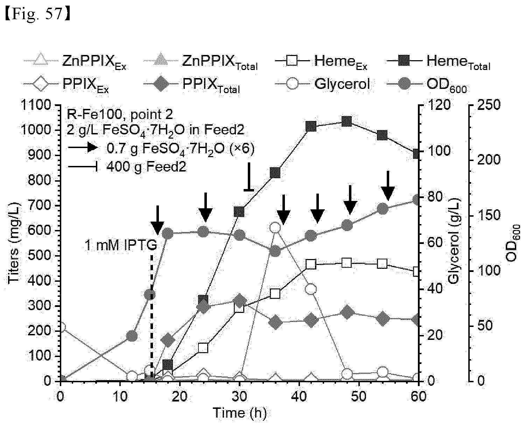

FIG. 57 shows the growth curve by fed-batch culture of the HAEM7 strain using R-Fe100 medium and the amounts of heme, PPIX and ZnPPIX produced by the fed-batch culture, and shows the results of supplying a feeding solution supplemented with 2 g/L FeSO₄·7H₂O after IPTG induction when the OD₆₀₀ reached 70 to 90 after the start of pH control at point 2 in FIG. 48, supplying an iron solution at 16.5, 24.1, 36.5, 42.5, 48 and 54.5 hours, and oversupplying 400 g of the feeding solution between 30.3 hours and 33.3 hours.

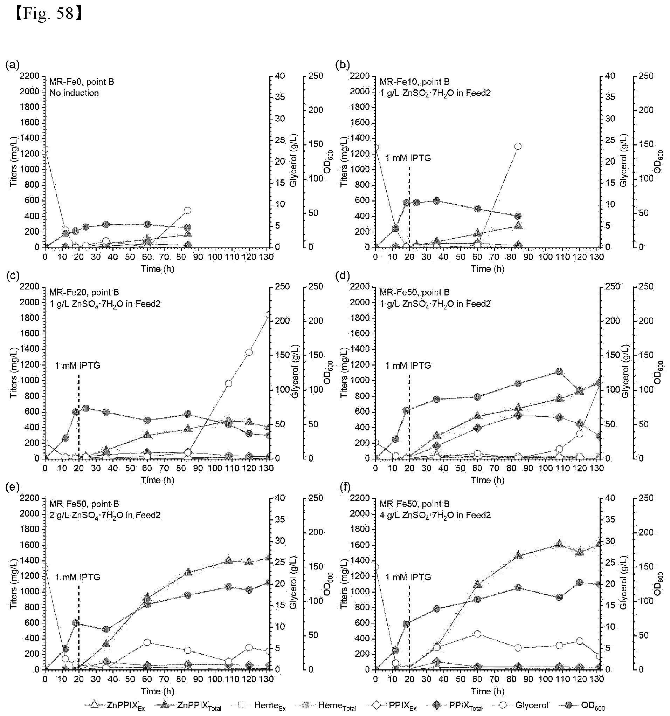

FIG. 58 shows the growth curve by fed-batch culture of the HAEM7 strain using MR-Fe0 (a), MR-Fe10 (b), MR-Fe20 (c) or MR-Fe50 (d) medium and the amounts of heme, PPIX and ZnPPIX produced by the fed-batch culture, and shows the results of supplying a feeding solution supplemented with 1 g/L ZnSO₄·7H₂O after IPTG induction when the OD₆₀₀ reached 70 to 80 after the start of pH control at point B in FIG. 46. It also shows the results of performing fed-batch culture of the HAEM7 strain using MR-Fe50 medium under the same conditions while supplying a feeding solution supplemented with 2 g/L ZnSO₄·7H₂O (e) or 4 g/L ZnSO₄·7H₂O (f).

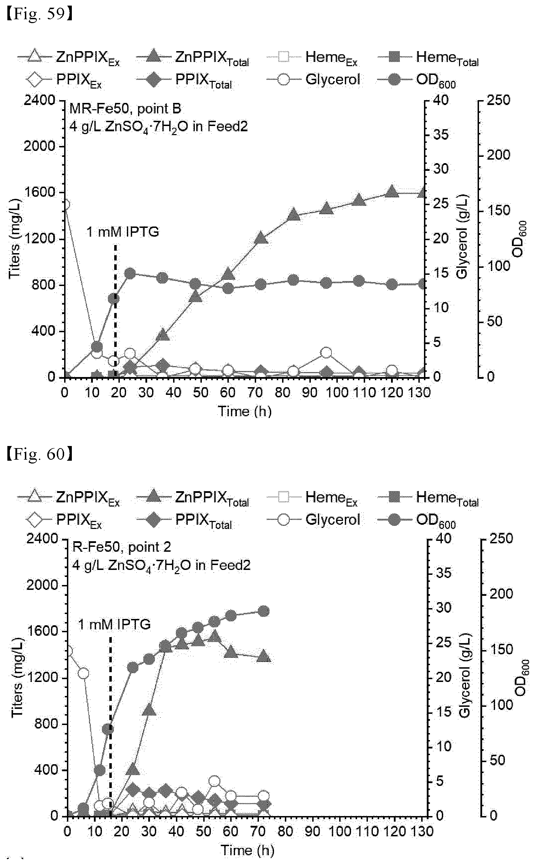

FIG. 59 shows the growth curve by fed-batch culture of the HAEM7 strain using R-Fe100 medium and the amounts of heme, PPIX and ZnPPIX produced by the fed-batch culture, and shows the results of supplying a feeding solution supplemented with 4 g/L ZnSO₄·7H₂O after IPTG induction when the OD₆₀₀ reached 70 to 80 after the start of pH control at point B in FIG. 46.
FIG. 60 shows the growth curve by fed-batch culture of the HAEM7 strain using R-Fe50 medium and the amounts of heme, PPIX and ZnPPIX produced by the fed-batch culture, and shows the results of supplying a feeding solution supplemented with 4 g/L ZnSO₄·7H₂O after IPTG induction when the OD₆₀₀ reached 70 to 80 after the start of pH control at point 2 in FIG. 48.

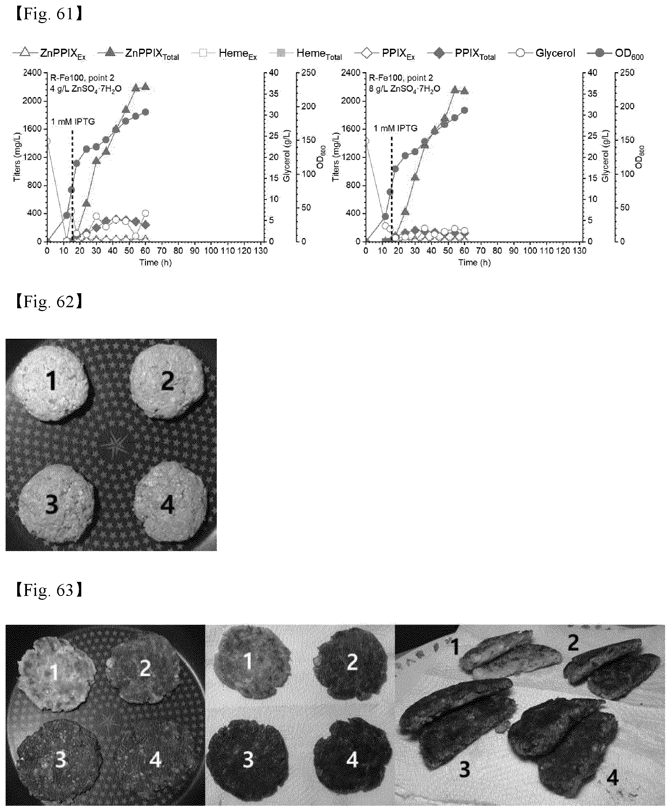

FIG. 61 shows the growth curve by fed-batch culture of the HAEM7 strain using R-Fe100 medium and the amounts of heme, PPIX and ZnPPIX produced by the fed-batch culture, and shows the results of supplying a feeding solution supplemented with 4 g/L ZnSO₄·7H₂O (a) or 8 g/L ZnSO₄·7H₂O (b) after IPTG induction when the OD₆₀₀ reached 70 to 80 after the start of pH control at point 2 in FIG. 48.
FIG. 62 shows the appearances of vegetable patty doughs prepared based on texturized soy protein (TSP) and tofu, which are a negative control group (1) to which heme, hemoglobin, or ZnPPIX was not added, a positive control group (2) to which hemoglobin was added, an experimental group (3) to which heme was added, and an experimental group (4) to which ZnPPIX was added.
FIG. 63 shows the appearances after cooking of vegetable patty doughs prepared based on texturized soy protein (TSP) and tofu, which are a negative control group (1) to which heme, hemoglobin, or ZnPPIX was not added, a positive control group (2) to which hemoglobin was added, an experimental group (3) to which heme was added, and an experimental group (4) to which ZnPPIX was added.

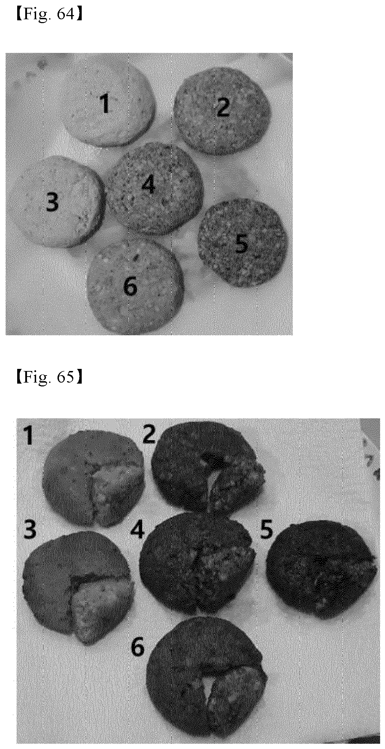

FIG. 64 shows the appearances of vegetable patty doughs prepared based on pressed tofu, which are a negative control group (1) to which dilute NaOH aqueous solution was added, an experimental group (2) to which heme suspended in dilute NaOH aqueous solution was added, a negative control group (3) to which dilute aqueous ammonia was added, an experimental group (4) to which heme suspended in dilute aqueous ammonia was added, an experimental group (5) to which a larger amount of heme suspended in dilute aqueous ammonia was added, and a positive control group (6) to which hemoglobin suspended in dilute aqueous ammonia was added.
FIG. 65 shows the appearances after cooking of vegetable patty doughs prepared based on pressed tofu, shown in FIG. 64, which are a negative control group (1) to which dilute NaOH aqueous solution was added, an experimental group (2) to which heme suspended in dilute NaOH aqueous solution was added, a negative control group (3) to which dilute aqueous ammonia was added, an experimental group (4) to which heme suspended in dilute aqueous ammonia was added, an experimental group (5) to which a larger amount of heme suspended in dilute aqueous ammonia was added, and a positive control group (6) to which hemoglobin suspended in dilute aqueous ammonia was added.

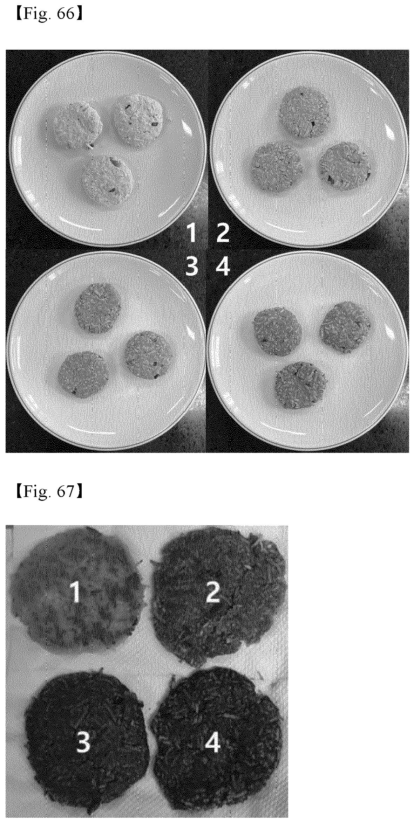

FIG. 66 shows the appearances of vegetable patty doughs prepared based on dried tofu and dehydrated tofu, which are a negative control group (1) to which heme was not added, and experimental groups (2 to 4) to which gradually higher concentrations of heme were added.
FIG. 66 shows the appearances after cooking of vegetable patty doughs prepared based on dried tofu and dehydrated tofu, shown in FIG. 65, which are a negative control group (1) to which heme was not added, and experimental groups (2 to 4) to which gradually higher concentrations of heme were added.

### Detailed Description and Preferred Embodiments of the Invention

It is known that, when heme-containing peptides or proteins such as leghemoglobin, hemoglobin or myoglobin are added to food, they can improve flavor, nutrition, and color of the food. In particular, when these peptides or proteins are added to meat substitutes, they can improve the flavor of the meat substitutes, increase iron absorption, and also simulate the red color of meat. Methods have been developed to purify heme-containing peptides/proteins for addition to food from animal blood or produce and purify the same from recombinant microorganisms or recombinant plants. However, these methods have a problem in that, because the ratio of the molecular weight of heme (0.6 kDa) relative to the molecular weights of heme-containing peptides or proteins (leghemoglobin, ~16 kDa; hemoglobin, ~65 kDa; and myoglobin, ~17 kDa) is low, it is necessary to produce and purify a relatively large amount of a heme-containing peptide/protein in order to obtain the effect of enhancing the flavor, nutrition or color of food by heme. Moreover, there are disadvantages in that the proteins are less stable than simple organic compounds, and thus purification and storage processes therefor are relatively difficult and incur higher costs.

On the other hand, various porphyrin, metalloporphyrin, or porphyrin analog compounds, including heme, have significantly lower molecular weights than heme-containing peptides/proteins, and thus contain a larger number of molecules of key compounds even if the same mass is produced and added. In addition, since these compounds are chemically more stable, they have the advantage of being easier to separate/purify and store, thereby lowering the production cost.

Accordingly, the present inventors have developed a microbial mutant and a microbial fermentation method, which have enhanced production of various porphyrins, metalloporphyrins, or porphyrin analog compounds, including heme. In addition, the present inventors have developed a method capable of effectively isolating and purifying various porphyrin, metalloporphyrin, and porphyrin analog compounds from microorganisms cultured by fermentation and fermentation broths. In addition, the present inventors have developed a method of preparing a solution or suspension by dissolving or suspending various isolated/purified porphyrin, metalloporphyrin, and porphyrin analog compounds to facilitate the addition of the compounds to food.

Furthermore, the present inventors have developed a method and a composition for adding the prepared solution or suspension of porphyrin, metalloporphyrin, and porphyrin analog compounds to food. In addition, in the present invention, in order to determine whether the flavor and color of food supplemented with various porphyrin, metalloporphyrin, or porphyrin analog compounds would be improved, food supplemented with porphyrin, metalloporphyrin or porphyrin analog compounds was cooked, the change in appearance of the food was evaluated, and the flavor change of the food was evaluated through tasting.

Meanwhile, when porphyrin, metalloporphyrin, or porphyrin analog compounds are isolated/purified from microorganisms that produce these compounds, the yield may be reduced due to losses during the isolation/purification process. In addition, there is a problem in that the production cost increases due to the materials and energy used in the isolation/purification process. On the other hand, when ingestible microorganisms themselves that produce porphyrin, metalloporphyrin, or porphyrin analog compounds, including heme, are used as food additives, loss of the produced compounds may be reduced, and the isolation/purification process cost may be reduced. Furthermore, useful nutrients such as proteins and vitamins contained in the biomass of the microorganisms may also be added to food, and thus can contribute to enhancing the nutritional components of the food.

The present inventors have developed a microbial mutant and a microbial fermentation method, which have enhanced production of various porphyrin, metalloporphyrin, or porphyrin analog compounds, including heme, based on an ingestible microorganism. Furthermore, the present inventors have developed a method and composition for adding porphyrin-, metalloporphyrin-, and porphyrin analog-producing microorganisms to food. In addition, in the present invention, food supplemented with microorganisms that produce various porphyrin, metalloporphyrin, or porphyrin analog compounds was cooked, a change in the appearance of the food was evaluated, and a change in the flavor of the food was evaluated through tasting.

Therefore, the present invention is directed to a method for producing a compound selected from the group consisting of heme, porphyrins, metalloporphyrins, and porphyrin analogs, the method comprising a step of producing a compound selected from the group consisting of heme, porphyrins, metalloporphyrins, and porphyrin analogs by culturing a heme-producing microorganism having a heme synthesis pathway gene in a medium containing a carbon source.

Examples of porphyrin compounds and porphyrin analogs that can be biosynthesized in prokaryotes and eukaryotes include, but are not limited to, protoporphyrin IX, coproporphyrin III, uroporphyrin III, coproporphyrin I, uroporphyrin I, sirohydrochlorin, and the like. These porphyrin compounds can form metalloporphyrins by binding various metal ions.

Among metalloporphyrins and analogs thereof that can be biosynthesized in prokaryotes and eukaryotes, Feporphyrin complex analogs include, but are not limited to, heme, Fe-coproporphyrin III, Fe-uroporphyrin III, Fe-coproporphyrin I, Fe-uroporphyrin I, siroheme, and the like. Here, examples of heme include, but are not limited to, heme b, hemin, hematin, heme a, heme c, heme d, heme l, heme m, heme o, heme s, and the like.

Among metalloporphyrins that can be biosynthesized in prokaryotes and eukaryotes, Zn-porphyrin complexes include, but are not limited to, Zn-protoporphyrin IX (ZnPPIX), Zn-coproporphyrin III, Zn-uroporphyrin III, Zn-coproporphyrin I, Zn-uroporphyrin I, and the like.

Among metalloporphyrins that can be biosynthesized in prokaryotes and eukaryotes, Mg-porphyrin complexes include, but are not limited to, Mg-protoporphyrin IX, Mg-coproporphyrin III, Mg-uroporphyrin III, Mg-coproporphyrin I, Mg-uroporphyrin I, chlorophyll, and the like. Here, examples of chlorophyll include, but are not limited to, chlorophyll a, chlorophyll a₂, chlorophyll b₂, bacteriochlorophyll a, bacteriochlorophyll b, bacteriochlorophyll c, bacteriochlorophyll d, bacteriochlorophyll e, and precursors thereof.

Among metalloporphyrins and metalloporphyrin analogs that can be biosynthesized in prokaryotes and eukaryotes, Co-porphyrin complexes and analogs include, but are not limited to, Co-protoporphyrin IX, Co-coproporphyrin III, Co-uroporphyrin III, Co-coproporphyrin I, Co-uroporphyrin I, vitamin B₁₂, cyanocobalamin, and the like.

Among metalloporphyrins and metalloporphyrin analogs that can be biosynthesized in prokaryotes and eukaryotes, Cu-porphyrin complexes and analogs include, but are not limited to, Cu-protoporphyrin IX, Cu-coproporphyrin III, Cu-uroporphyrin III, Cu-coproporphyrin I, Cu-uroporphyrin I, and the like.

Among metalloporphyrins and metalloporphyrin analogs that can be biosynthesized in prokaryotes and eukaryotes, Mn-porphyrin complexes and analogs include, but are not limited to, Mn-protoporphyrin IX, Mn-coproporphyrin III, Mn-uroporphyrin III, Mn-coproporphyrin I, and Mn-uroporphyrin I, and the like.

Therefore, in one aspect, the present invention is directed to a method for producing a compound selected from the group consisting of heme, porphyrins, metalloporphyrins, and porphyrin analogs, the method comprising a step of producing a compound selected from the group consisting of heme, porphyrins, metalloporphyrins, and porphyrin analogs by culturing a heme-producing microorganism having a heme synthesis pathway gene in a medium containing a carbon source.

In the present invention, the heme synthesis pathway gene may be controlled by the T7lac promoter.

In the present invention, when the OD value of the culture reaches 10 to 80 during culturing, expression of the heme synthesis pathway gene may be induced.

In the present invention, induction of expression of the gene may be performed by adding IPTG.

In order to increase the production of the compound selected from the group consisting of heme, porphyrins, metalloporphyrins, and porphyrin analogs, the present inventors checked whether the increase in production occurred through various changes in the medium or in a feeding solution during fed-batch culture.

In the present invention, the culturing may be fed-batch culture, and a solution containing Fe ions (Fe²⁺ or Fe³⁺) may be additionally supplied to increase the production of heme. The Fe ions (Fe²⁺ or Fe³⁺) may be supplied at a concentration of 1 to 30 mM. The final concentration of Fe ions in the medium may be in the range of 0.2 to 6 mM.

The term "fed-batch culture" refers to a culture method in which a medium is continuously or intermittently fed without removing the culture.

The molecular weights and key elements of the main inorganic compounds used in culture and the atomic weights of various elements contained in the fermentation medium are as follows. In Examples and below, concentrations expressed in g/l, mg/ml or g/ml may be expressed in mM or M in consideration of the molecular weights described below, and a person skilled in the art can calculate the concentration in different units through conversion therebetween.

| Compound | Molecular weight (g/mol) | Key element |
|---|---|---|
| FeSO₄·7H₂O | 278.02 | Fe, S |
| KH₂PO₄ | 136.09 | P |
| (NH₄)₂HPO₄ | 132.06 | P, N |
| MgSO₄·7H₂O | 246.48 | Mg, S |
| CaCl₂ | 110.98 | Ca |
| ZnSO₄·7H₂O | 287.6 | Zn, S |
| MnSO₄·5H₂O | 241.08 | Mn, S |
| COCl₂·6H₂O | 129.839 | Co |
| CuSO₄·5H₂O | 249.68 | Cu, S |
| (NH₄)₆Mo₇O₂₄·4H₂O | 1235.86 | Mo, N |
| Na₂B₄O₇·10H₂O | 381.37 | B |

| Element | Atomic weight (g/mol) |
|---|---|
| Fe | 55.85 |
| P | 30.97 |
| N | 14.01 |
| S | 32.07 |
| Mg | 24.31 |
| Ca | 40.08 |
| Zn | 65.38 |
| Mn | 54.94 |
| Co | 58.93 |
| Cu | 63.55 |
| Mo | 95.95 |
| B | 10.81 |

In the present invention, the production of Zn-protoporphyrin (ZnPPIX) among metalloporphyrins may be increased by reducing the concentration of Fe ions (Fe²⁺ or Fe³⁺) in the medium. For example, the production of Zn-protoporphyrin (ZnPPIX) among metalloporphyrins may be increased by reducing the concentration of Fe ions (Fe²⁺ or Fe³⁺) in the medium to 0.15 mM or less. The concentration of Fe ions (Fe²⁺ or Fe³⁺) in the medium may be reduced to a minimum of 0 mM, preferably 0.005 mM.

In the present invention, the production of Zn-protoporphyrin (ZnPPIX) among metalloporphyrins may be increased by increasing the concentration of Zn ions in the medium. For example, the production of Zn-protoporphyrin (ZnPPIX) among metalloporphyrins may be increased by increasing the concentration of Zn ions in the medium to 0.04 mM or more. The concentration of Zn ions in the medium may be increased up to 25 mM.

In the present invention, the culturing may be fed-batch culture, and the production of the compound selected from the group consisting of heme, porphyrins, metalloporphyrins, and porphyrin analogs may be increased by supplying a feeding solution containing a carbon source so that the residual carbon source concentration in the medium is 10 g/L or more. The feeding solution containing a carbon source may be supplied so that the residual carbon source concentration in medium is up to 400 g/L.

Examples of the carbon source include glycerol, glucose, sucrose, lactose, xylose, arabinose, and the like, without being limited thereto. According to the present invention, the carbon source may be glycerol.

In some cases, the feeding solution may contain iron (Fe) .

The culturing may be fed-batch culture, and the production of the compound selected from the group consisting of heme, porphyrins, metalloporphyrins, and porphyrin analogs may be increased by temporary depletion of the residual carbon source in the medium, for example, adjustment of the residual carbon source concentration in the medium to 0 g/L.

Temporary depletion of the residual carbon source in the medium refers to not supplying the carbon source, and the time when the residual carbon source concentration in the medium is 0 g/L may be 10 minutes or longer. The time when the residual carbon source concentration in the medium is 0 g/L may be 6 hours or less.

The culturing may be fed-batch culture, and the production of Zn-protoporphyrin (ZnPPIX) among metalloporphyrins may be increased by increasing the concentration of Zn ions in the feeding solution containing the carbon source to 3 mM or more. The Zn ion concentration in the feeding solution containing the carbon source may be increased up to 100 mM.

The production of the compound selected from the group consisting of heme, porphyrins, metalloporphyrins, and porphyrin analogs may be increased by increasing the concentrations of phosphate ions and trace metals in the medium so that the final cell density is increased to a cell density corresponding to an OD₆₀₀ of 90 or higher.

The term "trace metals" refers to metal elements that are essential for cell growth or promote growth when added, although they are added at a concentration of 5 mM or less in the preparation of minimal medium because their concentration in the biomass of microorganisms is low. The trace metals may be, for example, iron, manganese, boron, molybdenum, cobalt, calcium, zinc, and copper.

Regarding the increase in the concentration of the trace metal ions, the concentration may be adjusted in the following order of priority in order to increase the production of the compound selected from the group consisting of heme, porphyrins, metalloporphyrins, and porphyrin analogs.

### 1) Iron ions

### 2) Manganese ions, boron ions, and molybdenum ions

### 3) Cobalt ions

### 4) Calcium ions, zinc ions, and copper ions.

Regarding the increases in the concentrations of phosphate ions and trace metal ions, the concentrations may be increased to 100 or more based on the residual amounts in the medium. Specifically, when the concentrations are increased, the concentration of phosphate ions (PO₄³⁻) may be increased to 85 mM or more, the concentration of iron ions may be increased to 0.2 mM or more, the concentration of manganese ions to 0.02 mM or more, the concentration of boron ions to 0.002 mM or more, or the concentration of molybdenum ions to 0.003 mM or more.

When the concentrations of phosphate ions and trace metal ions are increased, the final cell density may be increased to 100 or more. The final cell density may be increased to a cell density corresponding to an OD₆₀₀ of 90 or more.

The production of the compound selected from the group consisting of heme, porphyrins, metalloporphyrins, and porphyrin analogs may be increased by increasing the concentrations of phosphate ions and trace metals in the medium so that the final cell density is increased to a cell density corresponding to an OD₆₀₀ of 90 or higher.

In order to increase the production of the compound selected from the group consisting of heme, porphyrins, metalloporphyrins, and porphyrin analogs, the time point at which pH control starts may be adjusted.

The production of the compound selected from the group consisting of heme, porphyrins, metalloporphyrins, and porphyrin analogs may be increased by delaying the start point of pH control to after the point at which the instantaneous change rate of pH increases or decreases by 30% or more compared to the average change rate for 10 minutes. The production of the compound selected from the group consisting of heme, porphyrins, metalloporphyrins, and porphyrin analogs may be increased by delaying the start point of pH control to after the point at which the instantaneous change rate of pH increases or decreases by up to 10,000% compared to the average change rate for 10 minutes.

For example, it can be seen that, when the pH changes (increases) at an average rate of +0.1/min for the last 10 minutes during observation, but suddenly starts to change (decrease) at a rate of -0.05/min, there is a -150% change, and when the pH changes (decrease) at an average rate of - 0.1/min for the last 10 minutes during observation, but suddenly starts to change (decrease) at a rate of -0.05/min, there is a +50% change.

The start point of pH control may be delayed to after 4 hours after inoculation.

As a technology for producing free heme unbound to peptides or proteins, strains obtained by microbial evolutionary breeding were reported, including a *Klebsiella variicola* strain (KR 10-2118083) having an intracellular heme content which is about 3-fold higher (0.12 µM/g DCW) than that of the wild-type *E.coli* strain W3110, and a *C. glutamicum* strain (KR 10-2210764) having a heme content which is about 4-fold higher (0.175 µM/g DCW) than that of the wild-type *Corynebacterium glutamicum* ATCC 13032 strain. In addition, there were reports of an *E. coli* strain (10-1427075) into which the exogenous C4 ALA biosynthesis pathway has been introduced and which produces 6.4 mg/L heme when cultured, and a *C. glutamicum* strain (10-2016-0145740) into which the exogenous C4 ALA biosynthesis pathway has been introduced has been introduced and which has an intracellular heme content of 0.74 mg/g DCW when cultured. In addition, the present inventors previously reported an example in which 225.9 mg/L heme was produced by enhancing the C5 ALA biosynthesis pathway characteristic of *E. coli* and the heme biosynthesis pathway and 132.0 mg/L heme was secreted extracellularly and an example in which 239.2 mg/L heme was produced through additional heme exporter gene overexpression and 151.4 mg/L heme was secreted extracellularly (KR 10-2168039).

In the present invention, the above-described microorganism may be used as the heme-producing microorganism, without being limited thereto.

In another aspect, the present invention is directed to a method for extracting a compound selected from the group consisting of heme, porphyrins, metalloporphyrins and porphyrin analogs, the method comprising steps of:
(a) suspending cells, collected from a fermentation broth of a heme-producing microorganism, in water; and
(b) sonicating the cell suspension, and extracting a compound selected from the group consisting of heme, porphyrins, metalloporphyrins and porphyrin analogs by adding a basic alcohol solution to the sonicated cell suspension.

In the present invention, the volume ratio between water and the basic alcohol solution added in step (b) may be 2 to 4:6 to 8.

In the present invention, the "basic alcohol solution" used is preferably a solution of NH₃ in ethanol (NH₃-EtOH), a solution of KOH in ethanol (KOH-EtOH), a solution of NaOH in ethanol (NaOH-EtOH), or a solution of NaOH in methanol (NaOH-MeOH), more preferably, a solution of NH₃ in ethanol (NH₃-EtOH) or a solution of KOH in ethanol (KOH-EtOH) .

The dissolution of heme, porphyrins, metalloporphyrins and porphyrin analogs into the supernatant may be promoted by increasing the pH of the fermentation broth containing heme, porphyrins, metalloporphyrins and porphyrin analogs to 8 or higher. A basic substance may be added to increase the pH to 8 or higher. The basic material added may be NH₃, NaOH, KOH, Tris, or an aqueous solution thereof. The method may further comprise steps of: separating the supernatant containing heme, porphyrins, metalloporphyrins and porphyrin analogs; and precipitating heme, porphyrins, metalloporphyrins and porphyrin analogs by adding an acidic substance.

The acidic substance may be, for example, acetic acid or hydrochloric acid.

In still another aspect, the present invention is directed to a method for recovering a compound selected from the group consisting of heme, porphyrins, metalloporphyrins and porphyrin analogs, the method comprising a step of: precipitating a compound selected from the group consisting of heme, porphyrins, metalloporphyrins and porphyrin analogs by adding acetic acid or hydrochloric acid (HCl) to an extract containing the compound.

In yet another aspect, the present invention is directed to a method for recovering a compound selected from the group consisting of heme, porphyrins, metalloporphyrins and porphyrin analogs, the method comprising a step of: precipitating a compound selected from the group consisting of heme, porphyrins, metalloporphyrins and porphyrin analogs by adding hydrochloric acid (HCl) to a supernatant separated after adding a basic aqueous solution to a culture of a heme-producing microorganism.

In still yet another aspect, the present invention is directed to a food having a flavor similar to that of meat, the food containing a compound selected from the group consisting of heme, porphyrins, metalloporphyrins and porphyrin analogs, obtained by fermentation of a heme-producing microorganism.

In the present invention, the food may be a meat substitute.

In a further aspect, the present invention is directed to a method for producing a meat substitute having a flavor similar to that of meat, the method comprising immersing a meat substitute in a solution containing: a compound selected from the group consisting of heme, porphyrins, metalloporphyrins, and porphyrin analogs, obtained by fermentation of a heme-producing microorganism; NaOH; and water.

In the present invention, the meat substitute may be a plant-based meat substitute.

In another further aspect, the present invention is directed to a food having a flavor similar to that of meat, the food containing an edible microorganism capable of producing a compound selected from the group consisting of heme, porphyrins, metalloporphyrins, and porphyrin analogs.

In the present invention, the food may be a meat substitute.

In the present invention, the edible microorganism may be *Arthrospira platensis, Arthrospira fusiformis, Arthrospira maxima, Aspergillus awamori, Aspergillus luchuensis, Aspergillus niger, Aspergillus oryzae, Aspergillus sojae, Bacillus amyloliquefaciens, Bacillus licheniformis, Bacillus subtilis, Bifidobacterium bifidum, Bifidobacterium breve, Bifidobacterium longum, Bifidobacterium lactis, Brevibacterium linens, Chlorella minutissima, Chlorella pyrenoidosa, Chlorella variabilis, Chlorella vulgaris, Enterococcus faecium, Escherichia coli, Gluconacetobacter xylinus, Lactobacillus acidophilus, Lactobacillus bulgaricus, Lactobacillus casei, Lactobacillus fermentum, Lactobacillus paracasei, Lactobacillus plantarum, Lactobacillus rhamnosus, Lactobacillus reuteri, Lactococcus* spp., *Leuconostoc* spp., *Penicillium camemberti, Penicillium candidum, Penicillium roqueforti, Propionibacterium freudenreichii, Rhizopus oligosporus, Saccharomyces cerevisiae, Saccharomyces pastorianus, Salinicoccus jeotgali, Schizosaccharomyces pombe, Staphylococcus* spp., *Streptococcus thermophilus, Yarrowia lipolytica,* or the like, without being limited thereto.

Hereinafter, the present invention will be described in more detail with reference to examples. These examples are only for illustrating the present invention, and it will be apparent to those skilled in the art that the scope of the present invention is not to be construed as being limited by these examples.

### Example 1: Establishment of extraction and analysis methods for analysis of heme and various porphyrins, metalloporphyrins, and porphyrin analogs

To establish an effective extraction method for analyzing heme and various porphyrins, metalloporphyrins, and porphyrin analogs, biomass obtained by culturing the *E*. *coli* strain HAEM7 (Zhao et al., Nature Catalysis 1, 721-728, 2018; KR 10-2168039) reported to have the ability to produce and extracellularly secrete heme was used. Here, the *E. coli* strain HAEM7 was constructed by deleting *yfeX, ldhA* and *pta* genes from *E. coli* BL21(DE3) and then introducing a pCDFDuet-1-based plasmid (pCDF-hemAL) expressing *E. coli hemA^{fbr}* and *hemL* genes, a pRSFDuet-1-based plasmid (pRSF-hemBCD) expressing *E. coli hemB, hemC* and *hemD* genes, a pETDuet-1-based plasmid (pET-heEFGH) expressing *E. coli hemE, hemF, hemG* and *hemH* genes, and a pACYCDuet-1-based plasmid (pACYC-ccmABC) expressing *E. coli ccmA, ccmB* and *ccmC* genes (Zhao et al., Nature Catalysis 1, 721-728, 2018; KR 10-2168039) . Using equal amounts of cells recovered from a culture of the *E. coli* strain HAEM7, the cells were suspended in various solutions made of NaOH, water, methanol (MeOH), ethanol (EtOH), DMSO, etc., and sonicated using a bath-type sonicator to facilitate extraction. Subsequently, the supernatant was recovered by centrifugation, and the concentrations of heme and protoporphyrin IX (PPIX) therein were analyzed using HPLC.

Analysis of heme and PPIX was performed based on the absorbance at 400 nm using high-performance liquid chromatography (HPLC, 1260 Infinity II, Agilent) equipped with an ultraviolet (UV) lamp, a visible light (VIS) lamp and a diode-array detector (DAD). An XBridge C18 column (5 µm, 4.6 x 150 mm) was used as the stationary phase, and 20 mM ammonium acetate (pH 9.6; buffer A) and MeOH (buffer B) were used as the mobile phase. The mobile phase was flowed at 0.6 mL/min, and the ratio of buffer A: buffer B was maintained at 90:10 for the first 3 minutes (0 to 3 min), and changed with a linear gradient from 90:10 to 10:90 for the next 17 minutes (3 to 20 min). For the next 5 minutes (20 to 25 min), the ratio of buffer A: buffer B was maintained at 10:90, and for the next 1 minute (25 to 26 min), the ratio of buffer A: buffer B was changed with a linear gradient from 10:90 to 0:100 along a linear gradient, and then maintained at the same ratio for 5 minutes (26-31 min). Finally, the ratio of buffer A:buffer B was changed to 90:10 and the mobile phase was allowed for 6 minutes to stabilize the column. At this time, the concentration of heme was calculated based on the molecular weight of hemin, a form of heme.

The concentrations of heme and PPIX extracted from the cells using various solutions made of NaOH, water, methanol (MeOH), ethanol (EtOH), DMSO, etc. were analyzed according to the above-described analysis method, and the results are shown in Table 1 below. The NaOH-MeOH (No. 2) extraction method that extracts the largest amounts of hemin and PPIX was selected.

**[Table 1]**

| No. | Solution for suspension | Additional solution | Hemin (mg/L) | PPIX (mg/L) |
|---|---|---|---|---|
| 1 | 1,000 µL 1 M NaOH-H₂O | - | 4.39 | 0.13 |
| 2 | 1,000 µL NaOH-MeOH | - | 6.64 | 0.34 |
| 3 | 1,000 µL NaOH-EtOH | - | 1.23 | 0.10 |
| 4 | 1,000 µL DMSO | - | 1.99 | 0.20 |
| 5 | 50 µL H₂O | 950 µL DMSO | 1.41 | 0.20 |
| 6 | 50 µL 1 M NaOH-H₂O | 950 µL EtOH | 3.61 | 0.16 |
| 7 | 500 µL 1 M NaOH | 500 µL MeOH | 3.99 | 0.26 |
| 8 | 500 µL 1 M NaOH | 500 µL EtOH | 5.84 | 0.17 |
| 9 | 500 µL 1 M NaOH | 500 µL DMSO | 4.39 | 0.13 |

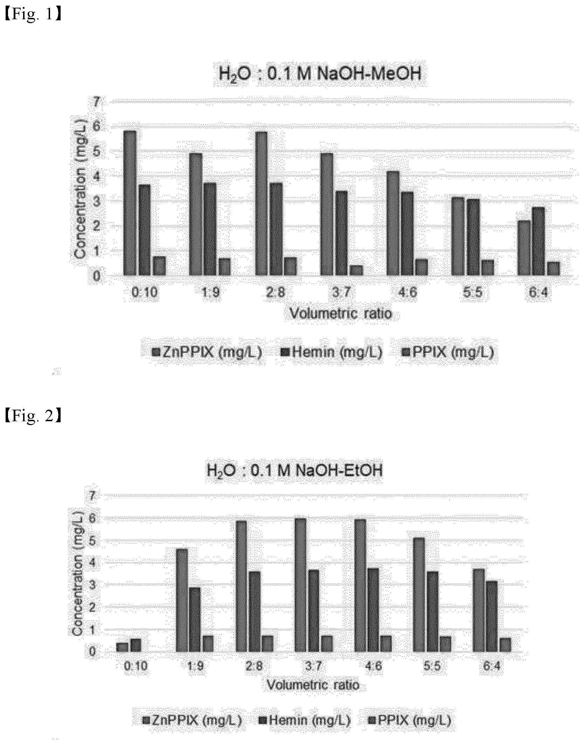

When the amount of recovered cells was large, a phenomenon was observed in which the cells were not well suspended when the NaOH-MeOH solution was added. Thus, for more stable extraction, a method of suspending the cells in water, followed by extraction by adding a 0.1 M NaOH-MeOH solution, was devised. The 0.1 M NaOH-MeOH solution was prepared by dissolving 0.2 g NaOH in 50 mL MeOH and filtering the solution through a 0.22-µm filter. The extraction results depending on the volume ratio between water used for suspension and the 0.1 M NaOH-MeOH solution added for extraction are shown in Table 2 below and FIG. 1. It was confirmed that hemin and PPIX were most effectively extracted when the volume ratio between water used for cell suspension and the 0.1 M NaOH-MeOH solution used for extraction was 2:8. For subsequent analysis, a method of suspending the cells in 200 µL water and adding 800 µL of the 0.1 M NaOH-MeOH solution thereto (condition No. 3), followed by treatment with a bath-type sonicator to facilitate extraction, was used.

**[Table 2]**

| No. | Solution for suspension | Additional solution | ZnPPIX (mg/L) | Hemin (mg/L) | PPIX (mg/L) |
|---|---|---|---|---|---|
| 1 | - | 1,000 µL 0.1 M NaOH-MeOH | 5.8 | 3.6 | 0.8 |
| 2 | 100 µL H₂O | 900 µL 0.1 M NaOH-MeOH | 4.9 | 3.7 | 0.7 |
| 3 | 200 µL H₂O | 800 µL 0.1 M NaOH-MeOH | 5.8 | 3.7 | 0.7 |
| 4 | 300 µL H₂O | 700 µL 0.1 M NaOH-MeOH | 4.9 | 3.4 | 0.4 |
| 5 | 400 µL H₂O | 600 µL 0.1 M NaOH-MeOH | 4.2 | 3.3 | 0.7 |
| 6 | 500 µL H₂O | 500 µL 0.1 M NaOH-MeOH | 3.2 | 3.1 | 0.6 |
| 7 | 600 µL H₂O | 400 µL 0.1 M NaOH-MeOH | 2.2 | 2.7 | 0.6 |

In addition, screening for extraction conditions using EtOH, which is less harmful than MeOH, was also performed. Table 3 below and FIG. 2 show the results of extracting heme from cells, suspended in water, using a 0.1 M NaOH-EtOH solution prepared by dissolving 0.2 g NaOH in 50 mL EtOH and filtering the solution through a 0.22-µm filter. It was confirmed that hemin, ZnPPIX, and PPIX were extracted most effectively when the volume ratio between water used for cell suspension and the 0.1 M KOH-EtOH solution used for extraction was in the range of 2:8 to 4:6.

**[Table 3]**

| No. | Solution for suspension | Additional solution | ZnPPIX (mg/L) | Hemin (mg/L) | PPIX (mg/L) |
|---|---|---|---|---|---|
| 1 | - | 1,000 µL 0.1 M NaOH-EtOH | 0.4 | 0.6 | 0 |
| 2 | 100 µL H₂O | 900 µL 0.1 M NaOH-EtOH | 4.6 | 2.9 | 0.7 |
| 3 | 200 µL H₂O | 800 µL 0.1 M NaOH-EtOH | 5.9 | 3.6 | 0.7 |
| 4 | 300 µL H₂O | 700 µL 0.1 M NaOH-EtOH | 6.0 | 3.7 | 0.7 |
| 5 | 400 µL H₂O | 600 µL 0.1 M NaOH-EtOH | 5.9 | 3.7 | 0.7 |
| 6 | 500 µL H₂O | 500 µL 0.1 M NaOH-EtOH | 5.1 | 3.6 | 0.7 |
| 7 | 600 µL H₂O | 400 µL 0.1 M NaOH-EtOH | 3.7 | 3.2 | 0.6 |

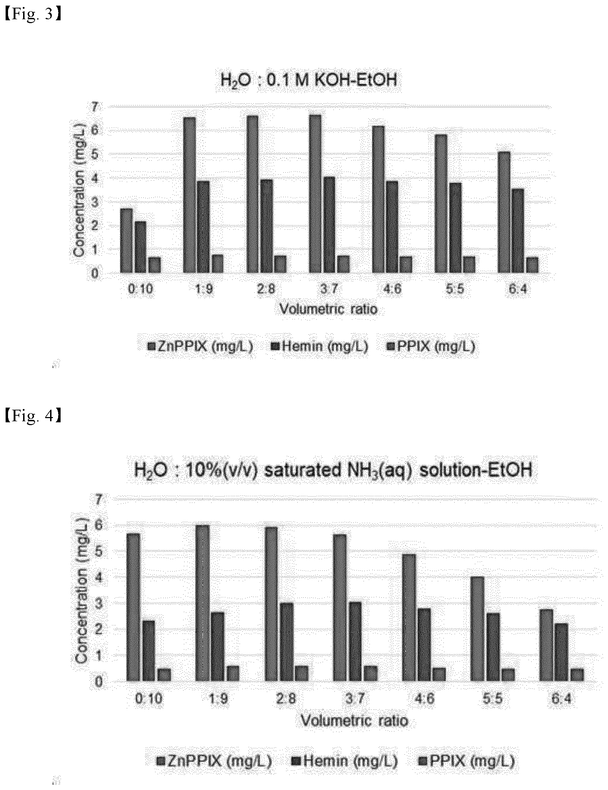

In addition, screening for conditions for mixing EtOH with KOH containing potassium ions (K⁺) known to help excrete Na⁺ when ingested, instead of NaOH containing sodium ions (Na⁺), which can cause an increase in blood pressure when ingested, was also conducted. Table 4 below and FIG. 3 show the results of extracting heme and the like from cells, suspended in water, using a 0.1 M KOH-EtOH solution prepared by dissolving 0.28 g KOH in 50 mL EtOH and filtering the solution through a 0.22-pm filter. It was confirmed that hemin, ZnPPIX, and PPIX were extracted most effectively when the volume ratio between water used for cell suspension and the 0.1 M KOH-EtOH solution used for extraction was in the range of 1:9 to 3:7.

**[Table 4]**

| No. | Solution for suspension | Additional solution | ZnPPIX (mg/L) | Hemin (mg/L) | PPIX (mg/L) |
|---|---|---|---|---|---|
| 1 | - | 1,000 µL 0.1 M KOH-EtOH | 2.7 | 2.2 | 0.7 |
| 2 | 100 µL H₂O | 900 µL 0.1 M KOH-EtOH | 6.5 | 3.9 | 0.8 |
| 3 | 200 µL H₂O | 800 µL 0.1 M KOH-EtOH | 6.6 | 3.9 | 0.7 |
| 4 | 300 µL H₂O | 700 µL 0.1 M KOH-EtOH | 6.6 | 4.0 | 0.7 |
| 5 | 400 µL H₂O | 600 µL 0.1 M KOH-EtOH | 6.2 | 3.9 | 0.7 |
| 6 | 500 µL H₂O | 500 µL 0.1 M KOH-EtOH | 5.8 | 3.8 | 0.7 |
| 7 | 600 µL H₂O | 400 µL 0.1 M KOH-EtOH | 5.1 | 3.5 | 0.7 |

Considering the subsequent extraction, isolation and purification of heme and various porphyrins, metalloporphyrins and porphyrin analogs, the isolation and purification process may be easier if the compound used for extraction has volatility. Thus, the present inventors tested whether a solution (NH₃-EtOH) obtained by mixing EtOH with ammonia (NH₃), a volatile basic compound, instead of NaOH or KOH, could be used for extraction of heme and the like. Table 5 below and FIG. 4 show the results of extracting heme and the like from cells, suspended in water, using an NH₃-EtOH solution prepared by mixing saturated aqueous ammonia and EtOH at a volume ratio of 1:9. It was confirmed that hemin, ZnPPIX, and PPIX were most effectively extracted when the volume ratio between water used for cell suspension and the NH₃-EtOH solution used for extraction was 2:8.

**[Table 5]**

| No. | Solution for suspension | Additional solution | ZnPPIX (mg/L) | Hemin (mg/L) | PPIX (mg/L) |
|---|---|---|---|---|---|
| 1 | - | 1,000 µL NH₃-EtOH | 5.7 | 2.3 | 0.5 |
| 2 | 100 µL H₂O | 900 µL NH₃-EtOH | 6.0 | 2.7 | 0.6 |
| 3 | 200 µL H₂O | 800 µL NH₃-EtOH | 5.9 | 3.0 | 0.6 |
| 4 | 300 µL H₂O | 700 µL NH₃-EtOH | 5.6 | 3.0 | 0.6 |
| 5 | 400 µL H₂O | 600 µL NH₃-EtOH | 4.9 | 2.8 | 0.5 |
| 6 | 500 µL H₂O | 500 µL NH₃-EtOH | 4.0 | 2.6 | 0.5 |
| 7 | 600 µL H₂O | 400 µL NH₃-EtOH | 2.8 | 2.2 | 0.5 |

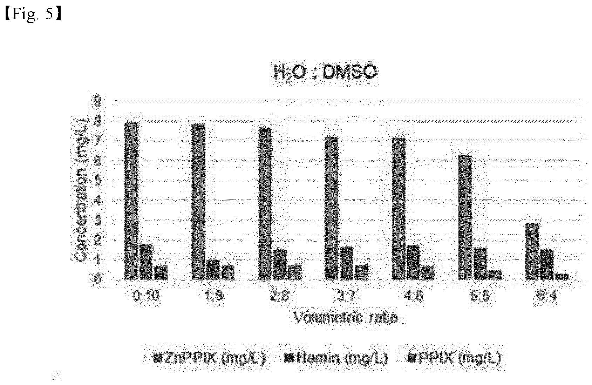

In addition, heme and various porphyrins, metalloporphyrins, porphyrin analogs, etc. were extracted from cells using DMSO, which was confirmed to effectively dissolve commercially available purified heme and various porphyrins, metalloporphyrins, porphyrin analogs, etc. Table 6 below and FIG. 5 show the results of extracting heme and the like from cells, suspended in water, using DMSO. The ZnPPIX extraction efficiency of DMSO was superior to that of other solutions, but the hemin and PPIX extraction efficiency of DMSO was lower than that of other solutions.

**[Table 6]**

| No. | Solution for suspension | Additional solution | ZnPPIX (mg/L) | Hemin (mg/L) | PPIX (mg/L) |
|---|---|---|---|---|---|
| 1 | - | 1,000 µL DMSO | 7.9 | 1.8 | 0.6 |
| 2 | 100 µL H₂O | 900 µL DMSO | 7.8 | 1.0 | 0.7 |
| 3 | 200 µL H₂O | 800 µL DMSO | 7.6 | 1.5 | 0.7 |
| 4 | 300 µL H₂O | 700 µL DMSO | 7.2 | 1.6 | 0.7 |
| 5 | 400 µL H₂O | 600 µL DMSO | 7.1 | 1.7 | 0.6 |
| 6 | 500 µL H₂O | 500 µL DMSO | 6.3 | 1.6 | 0.5 |
| 7 | 600 µL H₂O | 400 µL DMSO | 2.8 | 1.5 | 0.3 |

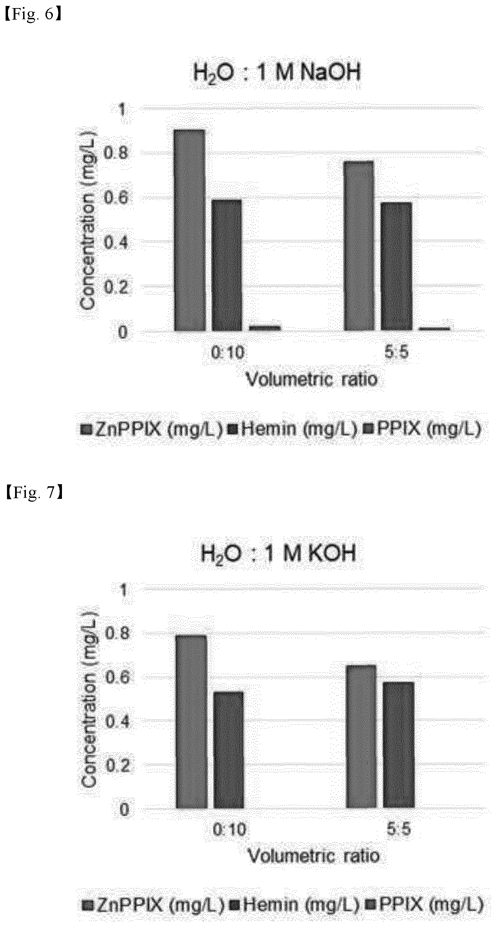

Meanwhile, Table 7 below and FIGS. 6 and 7 show the results of extracting heme and various porphyrins from cells using only aqueous solutions of NaOH or KOH.

**[Table 7]**

| No. | Solution for suspension | Additional solution | ZnPPIX (mg/L) | Hemin (mg/L) | PPIX (mg/L) |
|---|---|---|---|---|---|
| 1 | - | 1000 µL 1 M NaOH | 0.9 | 0.6 | 0.0 |
| 2 | 500 µL H₂O | 500 µL 1 M NaOH | 0.8 | 0.6 | 0.0 |
| 3 | - | 1000 µL 1 M KOH | 0.8 | 0.5 | 0.0 |
| 4 | 500 µL H₂O | 500 µL 1 M KOH | 0.7 | 0.6 | 0.0 |

### Example 2: Increase in heme production by changing carbon source in fermentation medium

Glycerol not only has a function of protecting cells from extreme environments or external stimuli, but also has more reducing power per carbon atom than glucose. In addition, considering that glycerol is produced in large quantities as a by-product of biodiesel production and can be obtained inexpensively, it was determined that glycerol could be a better carbon source than glucose for producing compounds that can stimulate cells, such as heme, or compounds that require a large amount of reducing power, by microbial fermentation. Furthermore, glucose, which is used as a carbon source in conventional fermentation for heme production, can induce catabolite repressor protein (CRP) to bind to the *lac* operator (lacO) located on the promoter that controls the overexpression of heme biosynthetic genes, thereby inhibiting expression of the heme biosynthetic genes. Therefore, it was determined that, when fermentation for heme production is performed using glucose as a carbon source, the expression level of heme biosynthetic genes may not be sufficient even after overexpression of these genes is induced. On the other hand, glycerol does not inhibit the expression of genes from promoters that control the expression of heme biosynthetic genes. Therefore, the present inventors performed flask culture of the HAEM7 strain using each of MR-Fe20 medium containing 20 g/L glucose as a carbon source and MR-Fe20 medium containing 20 g/L glycerol as a carbon source in order to confirm the difference in heme production depending on the carbon source.

For flask inoculation, the HAEM7 strain was inoculated into 5 mL MR-Fe0 medium in a 50-mL conical tube and cultured at 37°C and 200 rpm for about 12 hours. At this time, the MR-Fe0 medium was supplemented with 20 g/L glucose or 20 g/L glycerol as a carbon source and supplemented with 50 mg/L ampicillin, 25 mg/L kanamycin, 50 mg/L streptomycin, and 17 mg/L chloramphenicol as antibiotics. In addition, the composition of MR-Fe0 is shown in Table 8 below, and the composition of Fe-free trace metal solution (Fe0-TMS) is shown in Table 9 below.

**[Table 8]**

| **Component** | **Concentration** | |
|---|---|---|
| Citric acid | | 0.8 g/L |
| Fe-free trace metal solution (Fe0-TMS) | | 5 mL/L |
| FeSO₄.7H₂O | | 0 mg/L |
| KH₂PO₄ | | 6.67 g/L |
| (NH₄)₂HPO₄ | | 4 g/L |
| Yeast extract | | 5 g/L |
| MgSO₄·7H₂O | | 0.8 g/L |
| pH (using KOH) | | 7.0 |

**[Table 9]**

| **Component** | **Concentration** | |
|---|---|---|
| CaCl₂ | | 2 g/L |
| ZnSO₄· 7H₂O | | 2.2 g/L |
| MnSO₄· 5H₂O | | 0.58 g/L |
| CuSO₄· 5H₂O | | 1 g/L |
| (NH₄)₆Mo₇O₂₄· 4H₂O | | 0.1 g/L |
| Na₂B₄O₇· 10H₂O | | 0.02 g/L |
| FeSO₄· 7H₂O | | 0 g/L |
| Concentrated HCl | | 5 mL/L |

For flask culture, 1 mL of the HAEM7 strain cultured in MR-Fe0 medium for inoculation was placed in 50 mL MR-Fe20 medium in a 250-mL Erlenmeyer flask and cultured at 37°C and 200 rpm until the OD₆₀₀ reached 0.6 to 0.7. Then, 1 mM IPTG was added to the culture which was then cultured for 72 hours at 30°C and 200 rpm. At this time, the composition of MR-Fe20 is shown in Table 10 below, and the composition of Fe-free trace metal solution (Fe0-TMS) is shown in Table 9.

**[Table 10]**

| **Component** | **Concentration** | |
|---|---|---|
| Citric acid | | 0.8 g/L |
| Fe-free trace metal solution (Fe0-TMS) | | 5 mL/L |
| FeSO₄*7H₂O | | 20 mg/L |
| KH₂PO₄ | | 6.67 g/L |
| (NH₄)₂HPO₄ | | 4 g/L |
| Yeast extract | | 5 g/L |
| MgSO₄· 7H₂O | | 0.8 g/L |
| pH (using KOH) | | 7.0 |

In order to confirm the culture results of the HAEM7 strain depending on the carbon source, 1 mL of the culture was collected and separated into the supernatant and the cells by centrifugation at 16,100xg for 10 min. The collected supernatant was filtered through a 0.22-µm PVDF filter to remove particles, and then analyzed using the HPLC method described in Example 1 to analyze extracellularly secreted and produced heme, metalloporphyrins, and porphyrins. In addition, the collected cells were suspended in 200 µL distilled water and used to analyze heme, metalloporphyrins, and porphyrins, accumulated inside the cells, according to the extraction method and HPLC analysis method described in Example 1.

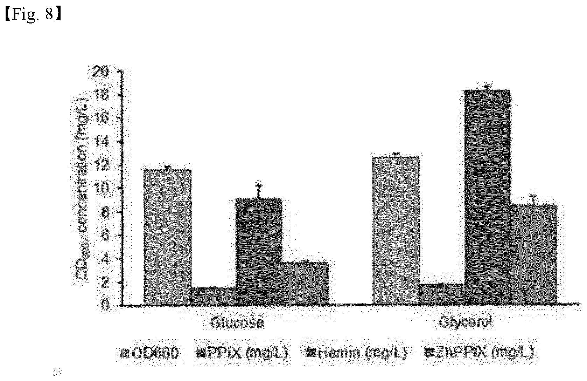

As a result, as shown in FIG. 8, it was confirmed that, when the HAEM7 strain was cultured using each of glucose and glycerol as the carbon source, heme (based on the molecular weight of hemin), PPIX, and ZnPPIX were produced at 9.1 ± 0.2 mg/L, 1.5 ± 0.0 mg/L, and 3.6 ± 0.2 mg/L, respectively, when the HAEM7 strain was cultured in the MR-Fe20 medium supplemented with glucose. On the other hand, it was confirmed that, when the HAEM7 strain was cultured in the MR-Fe20 medium supplemented with glycerol, heme (based on the molecular weight of hemin), PPIX, and ZnPPIX were produced at 18.3 ± 0.5 mg/L, 1.7 ± 0.1 mg/L, and 8.5 ± 0.8 mg/L, respectively, indicating that heme, porphyrins, and metalloporphyrins were produced at higher concentrations when glycerol was used as the carbon source than when glucose was used as the carbon source. In the following Examples, the HAEM7 strain was fermented in a medium supplemented with glycerol as a carbon source.

### Example 3: Increase in heme production by changing Fe concentration in fermentation medium

To evaluate the effect of iron (Fe) concentration in medium on the production of heme, metalloporphyrins, and porphyrins, flask culture of the HAEM7 strain was performed using each of MR-Fe20, MR-Fe50 and MR-Fe100 media, each supplemented with 20 g/L glycerol as a carbon source. The compositions of the MR-Fe20, MR-Fe50 and MR-Fe100 media are shown in Tables 10, 11, and 12, respectively, and the flask culture and the analysis of the production of heme, metalloporphyrins, and porphyrins are performed according to the methods in Example 2 and Example 1. As a result, Heme, metalloporphyrin, and porphyrin production of the HAEM7 strain depending on the Fe concentration in the medium is shown in FIG. 9. It was confirmed that, when the HAEM7 strain was cultured in the MR-Fe20 medium supplemented with glycerol, heme (based on the molecular weight of hemin), PPIX, and ZnPPIX were produced at 18.3 ± 0.5 mg/L, 1.7 ± 0.1 mg/L, and 8.5 ± 0.8 mg/L, respectively, whereas, when the HAEM7 strain was cultured in the MR-Fe50 medium, heme (based on the molecular weight of hemin), PPIX, and ZnPPIX were produced at 21.7 ± 2.7 mg/L, 1.5 ± 0.2 mg/L, and 7.0 ± 1.3 mg/L, respectively, indicating that the production of heme increased as the Fe concentration in the medium increased. In addition, when the HAEM7 strain was cultured in the MR-Fe100 medium, heme (based on the molecular weight of hemin), PPIX, and ZnPPIX were produced at 22.7 ± 1.5 mg/L, 1.5 ± 0.1 mg/L, and 8.3 ± 1.3 mg/L, respectively, and the production of heme in this case did not significantly differ from the production of heme when the strain was cultured in the MR-Fe50 medium.

**[Table 11]**

| **Component** | **Concentration** | |
|---|---|---|
| Citric acid | | 0.8 g/L |
| Fe-free trace metal solution (Fe0-TMS) | | 5 mL/L |
| FeSO₄*7H₂O | | 50 mg/L |
| KH₂PO₄ | | 6.67 g/L |
| (NH₄)₂HPO₄ | | 4 g/L |
| Yeast extract | | 5 g/L |
| MgSO₄· 7H₂O | | 0.8 g/L |
| pH (using KOH) | | 7.0 |

**[Table 12]**

| **Component** | **Concentration** | |
|---|---|---|
| Citric acid | | 0.8 g/L |
| Fe-free trace metal solution (Fe0-TMS) | | 5 mL/L |
| FeSO₄*7H₂O | | 100 mg/L |
| KH₂PO₄ | | 6.67 g/L |
| (NH₄)₂HPO₄ | | 4 g/L |
| Yeast extract | | 5 g/L |
| MgSO₄· 7H₂O | | 0.8 g/L |
| pH (using KOH) | | 7.0 |

### Example 4: Measurement of growth curve during fed-batch fermentation to control time point of heme biosynthetic gene expression, and production of protoporphyrin IX (PPIX)

According to the results of Examples 2 and 3, fed-batch fermentation of the HAEM7 strain was performed using the MR-Fe50 medium supplemented with 20 g/L glycerol as a carbon source, which was confirmed to be effective in increasing heme production. At this time, the effect of the time point of heme biosynthetic gene expression, induced by the addition of 1 mM IPTG, on heme production, was evaluated. Prior to selecting the time point of IPTG induction, fed-batch fermentation was performed without adding IPTG to obtain the growth curve of the HAEM7 strain in the MR-Fe50 medium supplemented with glycerol as a carbon source. First, the HAEM7 strain was inoculated into 5 mL LB medium (10 g/L NaCl, 10 g/L tryptone, and 5 g/L yeast extract) supplemented with 25 mg/L kanamycin, 50 mg/L ampicillin, 50 mg/L streptomycin, 17 mg/L chloramphenicol, and 10 g/L glucose, and then cultured for 12 hours at 37°C and 200 rpm. In order to obtain the HAEM7 inoculum for inoculating the fermenter, 0.4 mL of the culture in which the cultured HAEM7 cells were growing was inoculated into 200 mL of a freshly prepared MR-Fe0 medium (Table 8) supplemented with 25 mg/L kanamycin, 50 mg/L ampicillin, 50 mg/L streptomycin, 17 mg/L chloramphenicol, and 20 g/L glycerol, and then cultured in a 1-L Erlenmeyer flask at 37°C and 200 rpm for 12 hours. Next, the culture was inoculated into a 6.6-L Bioflo 320 fermenter (Eppendorf AG, Hamburg, Germany) containing 1.8 L of MR-Fe50 medium (Table 11) supplemented with 25 mg/L kanamycin, 50 mg/L ampicillin, 50 mg/L streptomycin, 17 mg/L chloramphenicol and 20 g/L glycerol at 30°C with agitation at 200 rpm in an air saturated state by air supply at a flow rate of 2 L/min. During fermentation, the temperature was maintained at 30°C, and the pH was maintained at 7.0 using a solution obtained by diluting a saturated ammonia solution in the same volume of water [-14% (w/v) NH₄OH]. In addition, the air saturation was maintained at 40% of the initial air saturation by increasing the agitation speed up to 1,000 rpm and then increasing the O₂ partial pressure in the supplied air. In addition, for continuous supply of glycerol during fed-batch culture, a feeding solution (800 g/L glycerol, 8 g/L MgSO₄7H₂O, 25 mg/L kanamycin, 50 mg/L ampicillin, 50 mg/L streptomycin, and 17 mg/L chloramphenicol) was supplied using a pH-stat technique. Sampling was conducted at 12-hour intervals to analyze the growth curve and heme, PPIX, and zinc-protoporphyrin IX (ZnPPIX) production. The growth curve was analyzed by measuring the OD₆₀₀ value, and the production of heme, PPIX, and ZnPPIX was analyzed using the method described in Example 1.

As a result, as shown in FIG. 10, it was confirmed that 128 mg/L of PPIX was produced without separate IPTG induction. On the other hand, after heme was produced at 20 mg/L at the beginning of fermentation (24 hours), the concentration thereof gradually decreased as the volume of the culture increased due to the increase in the supply amount of the feeding solution.

### Example 5: Increase in heme production by controlling time point of heme biosynthetic gene expression, and production of protoporphyrin IX (PPIX)

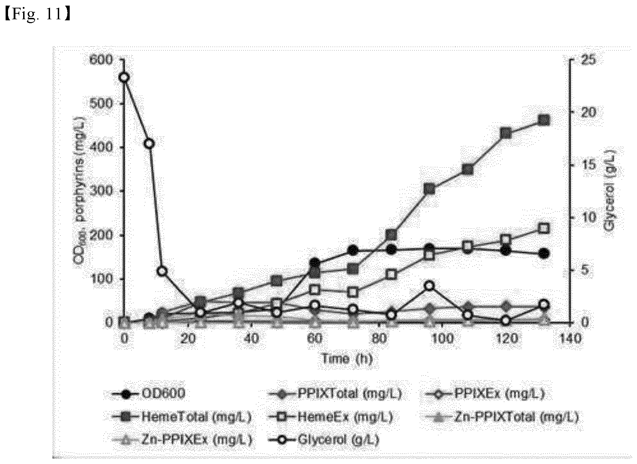

Based on the growth curve obtained in Example 4, heme production depending on the IPTG induction time point during fed-batch fermentation was analyzed. During fed-batch fermentation performed according to the method described in Example 4, when the OD₆₀₀ reached about 10, 25, 70, and 95, 1 mM IPTG was added, and a feeding solution having a changed composition (800 g/L glycerol, 8 g/L MgSO₄·7H₂O, 5 g/L (NH₄)₂SO₄, 1 g/L FeSO₄·7H₂O, 1 mM IPTG, 25 mg/L kanamycin, 50 mg/L ampicillin, 50 mg/L streptomycin, and 17 mg/L chloramphenicol) was supplied. Sampling was conducted at 12-hour intervals to analyze the growth curve and heme, PPIX and zinc-protoporphyrin IX (ZnPPIX) production. The growth curve was analyzed by measuring the OD₆₀₀ value, and the production of heme, PPIX, and ZnPPIX was analyzed using the method described in Example 1. The results are shown in FIGS. 11, 12, 13, and 14. In the case in which IPTG induction was performed when the OD₆₀₀ reached about 10, the largest amount of heme (461 mg/L, at 132 h) was produced, and at this time, ZnPPIX and PPIX were produced at 9.7 mg/L and 36.6 mg/L, respectively (FIG. 11). In the case in which IPTG induction was performed when the OD₆₀₀ reached about 70, the second largest amount of heme (363 mg/L, at 120 h) was produced, and at this time, ZnPPIX and PPIX were produced at 1.6 mg/L and 61.2 mg/L, respectively (FIG. 13). In the case in which IPTG induction was performed when the OD₆₀₀ reached about 95, heme production was the lowest (42.9 mg/L), and PPIX was produced at the highest concentration (120 mg/L) at 84 h, similar to the case where IPTG induction described in Example 4 was not performed (FIG. 14). Then, as heme production increased, the PPIX concentration decreased to 98 mg/L.

### Example 6: Control of iron concentration in feeding solution to increase heme production

Based on the fact that the iron concentration in the culture medium affects heme production, like the results of Example 3, it was determined that the iron concentration in the feeding solution during fed-batch fermentation would affect heme production. Thus, in order to examine the production of heme depending on the iron concentration in the feeding solution, fed-batch fermentation of the HAEM7 strain was performed while supplying a feeding solution containing iron at a lower concentration (0.5 g/L FeSO₄·7H₂O) or higher concentration (2 g/L, 4 g/L, or 8 g/L FeSO·7H₂O) than the iron concentration (1 g/L FeSO₄·7H₂O) used in Example 5. Fermentation conditions were the same as those in the case in which IPTG induction was performed when the OD₆₀₀ reached about 70, among the fermentation conditions described in Example 5, except for the iron concentration in the feeding solution supplied after IPTG induction. Growth curve measurement and the analysis of heme, ZnPPIX, and PPIX production were performed in the same manner as described in Example 4. The results are shown in FIGS. 15, 16, 17, and 18, and it was confirmed that the highest amount of heme was produced when fermentation was performed using the feeding solution containing FeSO₄·7H₂O, described in Example 5 (see FIG. 13).

### Example 7: Improvement of fermentation conditions for production of Zn-protoporphyrin IX (ZnPPIX)

It was confirmed that the production of heme could be increased while lowering the production of ZnPPIX, by increasing the concentration of iron in the culture medium during flask culture in Example 3. In view of this fact, it was expected that, when the iron concentration in the medium was lowered, the production of ZnPPIX could be increased while lowering the production of heme. Furthermore, it was expected that the production of ZnPPIX could be further increased by increasing the concentration of zinc ions (Zn2⁺) in the medium. To verify this expectation, according to the method described in Example 3, the HAEM7 strain was cultured in a flask using MR-Fe0 medium (Table 8) supplemented with 20 g/L glycerol. In addition, flask culture of the HAEM7 strain was also performed in media having increased ZnSO₄·7H₂O concentrations of 25 mg/L or 50 mg/L, respectively, obtained by adding ZnSO₄·7H₂O to the MR-Fe0 medium (Table 8) containing 11 mg/L ZnSO₄·7H₂O. As a result, as hypothesized, 32.7 ± 1.4 mg/L of ZnPPIX was produced in the MR-Fe0 medium, and when flask culture of the strain was performed in the media having increased ZnSO₄·7H₂O concentrations of 25 mg/L and 50 mg/L, 34.4 ± 0.9 mg/L of ZnPPIX and 35.3 ± 1.1 mg/L of ZnPPIX were produced in the media, respectively (Table 13). In addition, it could be confirmed that both heme and PPIX were produced at 3 mg/L or less, and the concentrations of heme and PPIX also decreased as the concentration of ZnSO₄·7H₂O increased.

**[Table 13]**

| Medium | ZnPPIX (mg/L) | Hemin (mg/L) | PPIX (mg/L) |
|---|---|---|---|
| MR-Fe0 (11 mg/L ZnSO₄·7H₂O) | 32.7 ± 1.4 | 2.8 ± 0.4 | 3.0 ± 0.2 |
| MR-Fe0 (25 mg/L ZnSO₄·7H₂O) | 34.4 ± 0.9 | 2.6 ± 0.1 | 1.6 ± 0.1 |
| MR-Fe0 (50 mg/L ZnSO₄·7H₂O) | 35.3 ± 1.1 | 2.5 ± 0.1 | 1.2 ± 0.1 |

### Example 8: Selection of fermentation broth pretreatment conditions for recovery of heme and various porphyrins, metalloporphyrins, and porphyrin analogs through fermentation supernatant

In order to effectively recover and isolate/purify heme and various porphyrins, metalloporphyrins, and porphyrin analogs, produced by microbial fermentation, various separation/purification conditions were investigated. Considering the use of the recovered products as food additives, an intensive investigation was made of ways to utilize ethanol (EtOH), NaOH, KOH, and aqueous ammonia that can be ingested without harm to the human body through the control of the concentration thereof in food and an appropriate neutralization process, among the compounds used for extracting heme and various porphyrins, metalloporphyrins, and porphyrin analogs in Example 1.

Since heme and various porphyrins, metalloporphyrins, and porphyrin analogs contain carboxyl groups in many cases, it was determined that increasing the pH would promote ionization of the carboxyl groups, thereby increasing their solubility in hydrophilic solvents. Thus, in order to evaluate the effect of the pH of the fermentation broth on the recovery of heme and various porphyrins, metalloporphyrins, and porphyrin analogs from the culture medium and cells, 0 mL, 0.1 mL, 0.5 mL or 1 mL of 1 M NaOH aqueous solution was added per 10 mL of the HAEM7 fermentation broth. At this time, the final pHs of the culture media were measured to be 6.9, 7.3, 8.5, and 9.1, respectively. The culture medium to which the NaOH aqueous solution was added was separated into the supernatant and the cells (pellet) by centrifugation at 10,000xg for 10 minutes. The results of analyzing the concentrations of heme (based on the molecular weight of hemin), PPIX, and ZnPPIX in the supernatant using the method described in Example 1 are shown in FIG. 19. It could be confirmed that, when 1 mL of 1 M NaOH was added per 10 mL of the fermentation broth (pH 9.1), the largest amounts of heme (100.6 mg/L), PPIX (3.8 mg/L), and ZnPPIX (0.6 mg/L) were dissolved.

### Example 9: Selection of extraction conditions and fermentation broth pretreatment conditions for recovery of intracellular heme and various porphyrins, metalloporphyrins, and porphyrin analogs

Next, before evaluating the effect of the pH of the fermentation broth on the recovery of heme and various porphyrins, metalloporphyrins, and porphyrin analogs from the recovered cells, the most effective mixing ratio of solutions for purifying the products from a large amount of cells recovered from the fermentation broth was selected. Considering the use of the recovered products as food additives, an intensive investigation was made of ways to utilize ethanol (EtOH), NaOH, KOH, and aqueous ammonia that can be ingested without harm to the human body through the control of the concentration thereof in food and an appropriate neutralization process, among the compounds used for extracting heme and various porphyrins, metalloporphyrins, and porphyrin analogs in Example 1.

To this end, the cells recovered from 1 mL of the fermentation broth to which NaOH was not additionally added were suspended in 100, 200, 300, or 400 µL of deionized water (DW). At this time, vortexing and sonication using a bath-type sonicator were performed to facilitate the suspension of the high-density cells. To the suspended cells, 900, 800, 700, or 600 µL of a solution of 0.1 M KOH in ethanol (KOH-EtOH), confirmed to be effective in extracting heme, PPIX, and ZnPPIX in Example 1, was added, an extraction process was performed through vortexing and sonication. Next, the supernatant separated by centrifugation was analyzed according to the method described in Example 1. As a result, as shown in FIG. 20, it was confirmed that, when the volume ratio between DW and the KOH-EtOH solution was 3:7, the largest amounts of heme (55 mg/L), PPIX (12 mg/L), and ZnPPIX (0.3 mg/L) were extracted.

Next, the extraction ability of a solution obtained by mixing ethanol with ammonia, a basic compound that can be easily used in the subsequent purification process due to its high volatility, was tested, although the effect of the solution on the extraction of heme, PPIX, and ZnPPIX was not the best in Example 1. Cells recovered from 1 mL of the fermentation broth to which additional NaOH was not added were suspended in 100, 200, 300, and 400 µL deionized water (DW) as described above, and then subjected to extraction by adding 900, 800, 700, or 600 µL of a solution (NH₃-EtOH) obtained by mixing saturated aqueous ammonia and ethanol at a volume ratio of 1:9. Next, the supernatant separated by centrifugation was analyzed according to the method described in Example 1. As a result, as shown in FIG. 21, it was confirmed that, when DW and the NH₃-EtOH solution were used at a volume ratio of 3:7, the largest amounts of heme (97 mg/L), PPIX (14 mg/L), and ZnPPIX (1 mg/L) were extracted.

Next, in order to reduce the inconvenience of preparing the NH₃-EtOH solution, the present inventors tested a condition in which cells were suspended in an aqueous ammonia solution and then subjected to extraction by adding pure ethanol. Cells recovered from 1 mL of the fermentation broth to which additional NaOH was not added were suspended in 100, 200, 300, or 400 µL of saturated aqueous ammonia (NH₃) as described above, and then subjected to extraction by adding 900, 800, 700, or 600 µL of ethanol (EtOH). Next, the supernatant separated by centrifugation was analyzed according to the method described in Example. As a result, as shown in FIG. 22, it was confirmed that, when NH₃ and EtOH were used at a volume ratio of 3:7, the largest amounts of heme (74 mg/L), PPIX (13 mg/L), and ZnPPIX (1 mg/L) were extracted. In addition, it was confirmed that, among the above-described three solution mixing methods, the method of suspending cells in DW and then adding the NH₃-EtOH solution had the best extraction efficiency.

Based on the above results, in order to evaluate the effect of the pH of the fermentation broth on the recovery of heme and various porphyrins, metalloporphyrins, and porphyrin analogs from cells, 0 mL (pH 6.9), 0.1 mL (pH 7.3), 0.5 mL (pH 8.5) or 1 mL (pH 9.1) was added per 10 mL of the HAEM7 fermentation broth, and then the collected cells (pellet) was suspended in 3 mL DW and subjected to extraction by adding 7 mL of the NH₃-EtOH solution. Next, the concentrations of heme (based on the molecular weight of hemin), PPIX, and ZnPPIX in the supernatant were analyzed using the method described in Example 1. As a result, as shown in FIG. 23, it was confirmed that, as in the case of the supernatant, when 1 mL of 1 M NaOH was added per 10 mL of the fermentation broth (pH 9.1), the largest amounts of heme (91 mg/L) and ZnPPIX (1 mg/L) were extracted. On the other hand, it could be confirmed that, when 1 M NaOH aqueous solution was added in amounts of 0 mL (pH 6.9), 0.1 mL (pH 7.3), 0.5 mL (pH 8.5), and 1 mL (pH 9.1), PPIX was extracted at concentrations of 11 mg/L, 11 mg/L, 7 mg/L, and 4 mg/L, respectively, indicating that the amount of PPIX extracted decreased as the pH increased due to an increase in the amount of NaOH added. Based on the above results, the fermentation broth pretreated by adding 1 M NaOH aqueous solution corresponding to 1/10 of the volume was used in subsequent recovery and isolation/purification processes.

### Example 10: Selection of precipitation conditions for heme and various porphyrins, metalloporphyrins, and porphyrin analogs contained in fermentation supernatant

Next, the present inventors tested a method of precipitating and recovering various porphyrins, metalloporphyrins, and porphyrin analogs, including heme, from the supernatant of the fermentation broth. Since a number of porphyrins, metalloporphyrins, and porphyrin analogs have carboxyl groups, it was determined that lowering the pH would suppress ionization of the carboxyl groups, thereby reducing their solubility in hydrophilic solvents. Therefore, the present inventors tested a method of precipitating various porphyrins, metalloporphyrins, and porphyrin analogs, including heme, by adding HCl to the HAEM7 fermentation broth to lower the pH. 0 mL, 0.1 mL, 0.5 mL, or 1 mL of 1 M NaOH aqueous solution was added per 10 mL of the HAEM7 fermentation broth, and then 0 µL, 10 µL, 20 µL, 50 µL, 100 µL, or 200 µL of concentrated hydrochloric acid (HCl) was added per mL of the collected supernatant. Next, the concentrations of heme, PPIX, and ZnPPIX in the supernatant collected by centrifugation were analyzed and the results are shown in FIGS. 24, 25, 26 and 27. As a result, it was confirmed that, when 0 mL or 0.1 mL of 1 M NaOH aqueous solution was added per 10 mL of the fermentation broth, the concentrations of heme, PPIX, and ZnPPIX remaining in the supernatant were the lowest when 50 µL of HCl was added per 1 mL of the supernatant. In addition, it was confirmed that, when 0.5 mL or 1 mL of 1 M NaOH aqueous solution was added per 10 mL of the fermentation broth, the concentrations of heme, PPIX, and ZnPPIX remaining in the supernatant were the lowest when 100 µL of HCl was added per 1 mL of the supernatant. Therefore, to precipitate heme and various porphyrins, metalloporphyrins, and porphyrin analogs from the supernatant of the fermentation broth, 50 µL or 100 µL of HCl was added per 1 mL of the supernatant to perform isolation and purification processes.

### Example 11: Selection of precipitation conditions for heme and various porphyrins, metalloporphyrins, and porphyrin analogs extracted from cells

Next, the present inventors tested the addition of acetic acid (Ac) and concentrated hydrochloric acid (HCl) to precipitate and recover heme and various porphyrins, metalloporphyrins, and porphyrin analogs extracted from cells. Cells recovered after flask culture were suspended in 2 mL DW and then subjected to extraction by adding 8 mL of one of a solution of 0.1 M NaOH in EtOH (NaOH-EtOH), s solution of 0.1 M KOH in EtOH (KOH-EtOH), and a solution (NH₃-EtOH) obtained by mixing saturated aqueous ammonia and ethanol at a volume ratio of 1:9. Next, 100 µL, 200 µL or 400 µL of acetic acid (Ac) or concentrated hydrochloric acid (HCl) was added per mL of the extract, followed by centrifugation, and then the supernatant was analyzed according to the method described in Example 1. As a result, as shown in FIGS. 28, 29 and 30, it was confirmed that, in the case of HCl, the concentrations of heme, PPIX, and ZnPPIX remaining in the supernatant without precipitating started to decrease significantly from when 100 µL of HCl was added per 1 mL of the extract, and the residual concentrations gradually decreased as the volume of HCl added increased. On the other hand, in the case of Ac, the concentration of ZnPPIX tended to decrease as the volume of Ac added increased, but no significant decrease in the concentration of heme or PPIX was observed. Therefore, in the following Examples, 200 µL of HCl was added per 1 mL of the extract to precipitate heme and various porphyrins, metalloporphyrins, and porphyrin analogs extracted from cells.

Furthermore, in order to reduce the amount of HCl required to precipitate heme and various porphyrins, metalloporphyrins, and porphyrin analogs extracted from cells, the present inventors tested a strategy of evaporating ethanol from the extract and adding HCl to induce precipitation. To this end, 1 mL of 1 M NaOH was added per 10 mL of the HAEM7 fermentation broth, and the recovered cells were suspended in 3 mL DW (together with vortexing and sonication), and subjected to extraction by adding 7 mL KOH-EtOH, thus obtaining 10 mL of an extract. The obtained extract was placed in a 50-mL conical tube, the bottom of the tube was heated to 40°C, and evaporation was induced by forming a continuous air flow on the surface of the solution. Next, the residue was re-suspending by adding 3 mL of DW, and precipitation was induced by adding 0 µL, 20 µL, 50 µL, 100 µL, or 200 µL of HCl per mL of the suspension. The results of analyzing the supernatant collected by centrifugation according to the method disclosed in Example 1 are shown in FIG. 31. As in the above example in which extraction from the fermentation supernatant was induced, when 100 µL of HCl was added per mL of the supernatant, the lowest amounts of heme (2 mg/L), PPIX (0 mg/L), and ZnPPIX (0 mg/L) remained in the supernatant.

### Example 12: Selection of dissolution conditions for heme and various porphyrins, metalloporphyrins, and porphyrin analogs precipitated from fermentation supernatant

In order to select conditions for re-dissolving various porphyrins and metalloporphyrins precipitated from the fermentation supernatant by applying the conditions selected in Example 10, a precipitate recovered by adding 50 µL of HCl per mL of a supernatant (80 mg/L hemin, 3.6 mg/L PPIX, and 0.3 mg/L ZnPPIX) collected by centrifugation after adding 1 M NaOH per 10 mL of the fermentation broth was used. The precipitate recovered from 1 mL of the supernatant was first dissolved/suspended in 300 µL 0.1 M NaOH, and then further dissolved by adding 700 µL KOH-EtOH solution or adding 700 µL NH₃-EtOH solution [NH₃-EtOH (0.1 M)] or 700 µL 0.1 M NaOH aqueous solution [NaOH (aq) (0.1M)] . At this time, in observation using the naked eye, no residual precipitate was found in all experimental groups (0.1 M) in which 300 µL of 0.1 M NaOH was added for the first dissolution. Thereafter, the supernatant collected by centrifugation was analyzed using the method described in Example 1, and the results are shown in FIG. 32.

In the following Examples, the substances precipitated from the fermentation supernatant were dissolved using the method [KOH-EtOH (0.1 M)] of dissolving/suspending the precipitate 300 µL of 0.1 M NaOH and then extracting the same using 700 µL KOH-EtOH solution, which was confirmed to dissolve the precipitate from which the largest amounts of heme, PPIX, and ZnPPIX (77 mg/L hemin, 2.4 mg/L PPIX, and 0.1 mg/L ZnPPIX) were recovered.

### Example 13: Selection of dissolution conditions for heme and various porphyrins, metalloporphyrins, and porphyrin analogs precipitated from cell extract

In order to select conditions for re-dissolving various porphyrins and metalloporphyrins precipitated from the cell extract by applying the conditions selected in Example 11, a precipitate recovered by adding 200 µL of concentrated hydrochloric acid (HCl) per mL of the extract obtained by adding 1 mL of 1 M NaOH per 10 mL of the fermentation broth, recovering cells therefrom by centrifugation, and suspending the recovered cells in 3 mL DW, followed by extraction by adding 7 mL KOH-EtOH (104 mg/L hemin, 5.6 mg/L PPIX, and 0.4 mg/L ZnPPIX) or adding 7 mL NH₃-EtOH (118 mg/L hemin, 5.6 mg/L PPIX, and 1 mg/L ZnPPIX), was used. The precipitate recovered from 1 mL of the extract was first dissolved/suspended in 300 µL of 0.1 M NaOH, and then further dissolved by adding 700 µL KOH-EtOH solution [KOH-EtOH (0.1 M)] or adding 700 µL NH₃-EtOH solution [NH₃-EtOH (0.1 M)] or 700 µL of 0.1 M NaOH aqueous solution [NaOH (aq) (0.1M)]. At this time, vortexing and sonication were also performed to promote dissolution and suspension. A precipitate of another group was first dissolved/suspended in 300 µL of 10 mM NaOH, and then further dissolved by adding 700 µL KOH-EtOH solution [KOH-EtOH(0.1 M)] or adding 700 µL NH₃-EtOH solution [NH₃-EtOH (0.1M)] or 700 µL 10 mM NaOH aqueous solution [NaOH (aq) (0.1M)]. At this time, in observation using the naked eye, no residual precipitate was found in all experimental groups (0.1 M) in which 300 µL of 0.1 M NaOH was added for the first dissolution. Thereafter, the supernatant collected by centrifugation was analyzed using the method described in Example 1, and the results are shown in FIGS. 33 and 34.

In the case in which precipitation was performed after extraction from the cells using KOH-EtOH, the largest amounts of heme, PPIX and ZnPPIX were recovered in the experimental group in which the precipitate was suspended in 300 µL of 0.1 M NaOH, followed by the addition of 700 µL KOH-EtOH solution [KOH-EtOH (0.1 M); 75 mg/L hemin, 1.6 mg/L PPIX, and 1.1 mg/L ZnPPIX], or in which the precipitate was suspended in 300 µL of 10 mM NaOH, followed by the addition of 700 µL KOH-EtOH solution [KOH-EtOH (10 mM); 75 mg/L hemin, 1.6 mg/L PPIX, and 1.1 mg/L ZnPPIX].

Likewise, in the case in which precipitation was performed after extraction from the cells using NH₃-EtOH, the largest amounts of heme, PPIX and ZnPPIX were recovered in the experimental group in which the precipitate was suspended in 300 µL of 0.1 M NaOH, followed by the addition of 700 µL KOH-EtOH solution [KOH-EtOH (0.1 M); 96 mg/L hemin, 1.6 mg/L PPIX, and 1.2 mg/L ZnPPIX], or in which the precipitate was suspended in 300 µL of 10 mM NaOH, followed by the addition of 700 µL KOH-EtOH solution [KOH-EtOH (10 mM); 100 mg/L hemin, 1.4 mg/L PPIX, and 1.2 mg/L ZnPPIX]. However, a larger amount of heme was recovered when precipitation was performed after extraction from the cells using NH₃-EtOH than when precipitation was performed after extraction from the cells using KOH-EtOH.

In addition, the present inventors tested whether the extraction conditions selected in the above Example could also be applied to the cell extract resuspended in DW after drying. At this time, extracts recovered by adding 0 µL, 20 µL, 50 µL, 100 µL or 200 µL of concentrated hydrochloric acid (HCl) per mL of a suspension obtained by adding 1 M NaOH to 10 mL of the fermentation broth in Example 11, recovering cells therefrom by centrifugation, suspending the recovered cells in 3 mL DW, adding 7 mL KOH-EtOH thereto to obtain an extract, drying 10 mL of the extract, and re-suspending the dried extract in 3 mL DW, were used. Each precipitate was suspended in 300 µL of 0.1 M NaOH aqueous solution and then dissolved by adding 700 µL KOH-EtOH solution.

As a result, as shown in FIG. 35, it was confirmed that, the concentrations of hemin, PPIX, and ZnPPIX in the supernatant remaining after HCl treatment (FIG. 31) decreased, the concentrations of hemin, PPIX, and ZnPPIX dissolved from the precipitate increased, indicating that the two results showed consistency. In addition, it was confirmed that the above-described redissolution conditions were effectively applied to the cell extract resuspended in DW after drying.

### Example 14: Selection of washing conditions for precipitated heme and various porphyrins, metalloporphyrins, and porphyrin analogs

In order to select conditions for washing the precipitate while minimizing the loss of heme and various porphyrins, metalloporphyrins, and porphyrin analogs from the precipitate recovered by applying the methods of Examples 10 and 11, a washing method using DW or ethanol was selected. To this end, the precipitate recovered by adding 50 µL of concentrated hydrochloric acid (HCl) to 1 mL of the supernatant (Original) separated from the fermentation broth to which 1 mL of 1 M NaOH was added per 10 mL was used. The washing operation was performed by suspending the precipitate recovered from 1 mL of the supernatant in 1 mL of ethanol (EtOH) or 1 mL of deionized water (DW) (together with vortexing and sonication) and separating the suspension into the supernatant and the pellet by centrifugation. First, the supernatant obtained in each washing step by sequential washing with 1 mL EtOH (Wash 1), 1 mL EtOH (Wash 2) and 1 mL DW (Wash 3), and the sample (Extraction) obtained by suspending the final precipitate in 300 µL of 0.1 M NaOH and dissolving the same by adding 700 µL KOH-EtOH were analyzed according to the method described in Example 1 (FIG. 36), and as a result, it was confirmed that significant amounts of heme, PPIX, and ZnPPIX were lost during the washing process performed using EtOH. In addition, it was confirmed that, even when the precipitate was washed sequentially with 1 mL DW (Wash 1), 1 mL DW (Wash 2) and 1 mL EtOH (Wash 3) (FIG. 37), significant amounts of heme, PPIX and ZnPPIX were lost in the washing step performed using EtOH. Therefore, it was confirmed that washing with DW rather than EtOH is preferable to minimize loss of various porphyrins, metalloporphyrins, and porphyrin analogs, including heme, during washing.

### Example 15: Separation and purification of heme and various porphyrins, metalloporphyrins, and porphyrin analogs from fermentation supernatant under selected conditions

Under the conditions selected in Example 8, Example 10, Example 12, and Example 14, heme and various porphyrins, metalloporphyrins, and porphyrin analogs were separated and purified from the fermentation supernatant of the HAEM7 strain. First, 1 mL of 1 M NaOH aqueous solution was added to 10 mL of the HAEM7 fermentation broth, and then the supernatant was collected therefrom by centrifugation. Thereafter, 0.5 mL concentrated hydrochloric acid (HCl) was added to 10 mL of the collected supernatant, and the formed precipitate was collected by centrifugation and washed twice (Wash 1 and Wash 2) with 5 mL DW. The precipitate remaining after washing was suspended in 3 mL of 0.1 M NaOH aqueous solution to quantify the recovered material, and then subjected to extraction (recovery) by adding 7 mL KOH-EtOH solution. In addition, in order to measure the mass ratio of heme and various porphyrins, metalloporphyrins, and porphyrin analogs in the collected precipitate, the precipitate washed in the same way was freeze-dried for about 6 hours, and then 6.0 mg was suspended in 300 µL of 0.1 M NaOH, followed by extraction by adding 700 µL KOH-EtOH [Freeze-dried (6.0 g/L)]. Heme and various porphyrins, metalloporphyrins, and porphyrin analogs in the supernatant and lysate obtained in each step were analyzed using the method described in Example 1, and as a result, as shown in FIG. 38, it was confirmed that about 91% of heme was recovered, and the mass specific gravity of heme (based on the molecular weight of hemin) in the freeze-dried sample was about 0.7%. Also, about 89% of ZnPPIX and about 78% of PPIX were recovered.

### Example 16: Separation and purification of heme and various porphyrins, metalloporphyrins, and porphyrin analogs from cell extract under selected conditions

Under the conditions selected in Example 9, Example 11, Example 13, and Example 14, heme and various porphyrins, metalloporphyrins, and porphyrin analogs were separated and purified from the cells of the HAEM7 strain. First, 1 mL of 1 M NaOH aqueous solution was added to 10 mL of the HAEM7 fermentation broth, and cells were collected therefrom by centrifugation. The collected cells were suspended in 3 mL DW, and a cell extract (Original) was prepared by adding 7 mL KOH-EtOH solution or 7 mL NH₃-EtOH solution to the cell suspension. Subsequently, 2 mL concentrated hydrochloric acid (HCl) was added to 10 mL of the cell extract, and the formed precipitate was collected by centrifugation and washed twice (Wash 1 and Wash 2) with 5 mL DW. The precipitate remaining after washing was suspended in 3 mL of 0.1 M NaOH aqueous solution to quantify the recovered material, and then subjected to extraction (recovery) by adding 7 mL KOH-EtOH solution. In addition, in order to measure the mass ratio of heme and various porphyrins, metalloporphyrins, and porphyrin analogs in the collected precipitate, the precipitate washed in the same way was freeze-dried for about 6 hours, and then 1.8 mg (sample derived from KOH-EtOH solution-based cell extract) or 2.1 mg (sample derived from NH₃-EtOH solution-based cell extract) was suspended in 300 µL of 0.1 M NaOH, followed by extraction by adding 700 µL KOH-EtOH [Freeze-dried (1.8 g/L) and [Freeze-dried (2.1 g/L)]. Heme and various porphyrins, metalloporphyrins, and porphyrin analogs in the supernatant and lysate obtained in each step were analyzed using the method described in Example 1. As a result, as shown in FIGS. 39 and 40, it was confirmed that, when separation/purification from the KOH-EtOH solution-based cell extract was performed, about 68% of heme was recovered, and the mass specific gravity of heme in the freeze-dried sample (based on the molecular weight of hemin) was about 6.6%. In addition, it was confirmed that, when separation/purification from the NH₃-EtOH solution-based cell extract was performed, about 71% of heme was recovered, and the mass specific gravity of heme (based on the molecular weight of hemin) in the freeze-dried sample was about 2.7%. About 100% of ZnPPIX and 27% of PPIX were recovered.

### Example 17: Preparation of solutions/suspensions of various porphyrins, metalloporphyrins, and porphyrin analogs for addition to food

In order to add heme and various porphyrins, metalloporphyrins, and porphyrin analogs, which are not bound to peptides and proteins, to food, it is necessary to prepare the same in the form of solutions or suspensions that can be easily mixed with food. A hemin reagent (hemin) purified from pig blood to secure a method for dissolving/suspending heme and various porphyrins, metalloporphyrins, and porphyrin analogs that can be easily added to food and to secure a solution/suspension that can be used for food addition (hemin) reagent ( >96%, BioXtra, Sigma-Aldrich) and solution/suspension were prepared. In order to secure a method for dissolving/suspending heme and various porphyrins, metalloporphyrins, and porphyrin analogs that can facilitate the addition of the same to food and to secure a solution/suspension that can be used for the addition of the same to food, a method for dissolving//suspending hemin reagent (>96%, BioXtra, Sigma-Aldrich) purified from porcine blood and a solution/suspension of the hemin reagent were prepared.

A hemin solution for addition to food was prepared by dissolving about 10 mg of hemin powder in 1 mL of 50 mM NaOH aqueous solution, followed by dilution in 50 mL of drinking water (hemin-NaOH-water solution; about 200 mg/L hemin). Another form of hemin solution was prepared by dissolving about 10 mg of hemin powder in 1 mL of dilute aqueous ammonia prepared by mixing 0.1 mL of saturated aqueous ammonia and 0.9 mL of water, followed by dilution in 50 mL of drinking water (hemin-NH₃-water solution; about 200 mg/L hemin). Still another form of hemin solution was prepared by dissolving about 10 mg of hemin powder in 1 mL of dilute aqueous ammonia prepared by mixing 0.1 mL of saturated aqueous ammonia and 0.9 mL of water, followed by dilution in 50 mL of ethanol (hemin-NH₃-EtOH solution; about 200 mg/L hemin). At this time, a greenish dark brown color was observed in the hemin-NaOH-water solution and the hemin-NH₃-water solution, and a dark reddish brown color was observed in the hemin-NH₃-EtOH solution (FIG. 41).

### Example 18: Addition of heme and various porphyrins, metalloporphyrins, and porphyrin analogues to food

In order to evaluate the color and flavor after cooking of food to which heme unbound to peptides and proteins was added, soybean meat to which hemin was added was prepared using soy meat (Hyokyung Tech) made of hemin reagent (>96%, BioXtra, Sigma-Aldrich) purified from porcine blood and texturized soy protein (TSP).

In order to hydrate soy meat in the prepared solution and absorb hemin at the same time, the texturized soy protein (TSP) soy meat product (Hyokyung Tech) prepared for bulgogi was rinsed with running water, and then two pieces of the soy meat product were hydrated by soaking for 20 minutes or more in each of the hemin-NaOH-water, hemin-NH₃-water, and hemin-NH₃-EtOH solutions prepared in Example 17 (FIG. 42, Nos. 3, 4, and 8, respectively) . In addition, for use as a control group, two pieces of soy meat rinsed in water were soaked in water (No. 2) for 20 minutes or more, and two other pieces were soaked in ethanol (No. 7) for 20 minutes or more (FIG. 42). At this time, since it was confirmed that the soy meat soaked in each of ethanol and the hemin-NH₃-EtOH solution was not hydrated and was maintained in a hard state, the soy meat was transferred to water and subjected to second hydration (FIG. 42, Nos. 7 and 8, respectively). Conversely, other groups were also prepared by subjecting 2 pieces of the soy meat, subjected to first hydration by soaking in water, to second hydration by soaking in each of ethanol (No. 5) and the hemin-NH₃-EtOH solution (No. 6) (FIG. 42). At this time, it was confirmed that the groups, subjected to first hydration by soaking in water and subjected to second hydration by soaking in each of ethanol (No. 5) and the hemin-NH₃-EtOH solution (No. 6), absorbed water during the first hydration process, and the increased volume was partially reduced due to dehydration by ethanol during the second hydration process (FIG. 42).

After completion of hydration, it could be confirmed that the soy meat soaked in the hemin-NaOH-water solution (No. 3) was slightly reddish brown compared to the control group (No. 2) hydrated by soaking in water (FIG. 42). In addition, it was confirmed that the soy meat hydrated in the hemin-NH₃-water solution (No. 4) was brownish compared to the control group (No. 2) hydrated by soaking in water, and was significantly less reddish compared to the soy meat (No. 3) hydrated in the hemin-NaOH-water solution (FIG. 42). In particular, it was confirmed that the soy meat hydrated in the hemin-NH₃-water solution absorbed a larger amount of water and the volume thereof significantly was expanded, compared to the control group (No. 2) hydrated in water or the soy meat (No. 3) hydrated in the hemin-NaOH-water solution (FIG. 42) . It was confirmed that the brown color of the hydrated soy meat was in sharp contrast with the green color of the hemin aqueous solution (FIGS. 41 and 42).

The soy meat (No. 6) soaked in the hemin-NH₃-EtOH solution after being hydrated with water had a more reddish brown color compared to the soy meat (No. 5) soaked in EtOH after being hydrated with water (FIG. 42). In addition, it was confirmed that the soy meat (No. 6) had a more reddish dark brown color compared to the soy meats (Nos. 3 and 4) soaked in other aqueous solutions containing hemin (FIG. 42). On the other hand, the soy meat (No. 8) soaked in water after soaking in the hemin-NH₃-EtOH solution had a slightly reddish color compared to the soy meat (No. 7) soaked in water after soaking in EtOH, but no significant color change thereof was observed (FIG. 42).

### Example 19: Cooking and tasting of food supplemented with heme and various porphyrins metalloporphyrins, and porphyrin analogs

The hydrated soy meat was cooked by removing the solution therefrom and then baking the same in a heated frying pan with grapeseed oil. After sufficiently removing the solution from the hydrated soy meat and roasting the same with only minimal oil, the soy meat soaked in the hemin solution had a dark brown color similar to that of grilled beef (FIG. 43). Thereamong, the soy meat (No. 3) hydrated in the hemin-NaOH-water solution had a brighter color, and the soy meat (No. 6) soaked in the hemin-NH₃-EtOH solution after hydration by soaking in water had the darkest color. In addition, it could be confirmed that the soy meats (Nos. 5 to 8) subjected to first or second soaking in ethanol had a relatively smaller volume and stiffer tissue than the soy meat not soaked in ethanol. During cooking, no special smell was detected regardless of the processing method, and the smell of the soy meat after cooking was not significantly different from the smell of soaked rice/grains. However, it was confirmed that the soy meat soaked in the hemin solution had a relatively small smell of soaked rice/grains. However, in the case of soy meats (FIG. 43, No. 5 and No. 6) that were soaked in an ethanol solution immediately before cooking, the ethanol contained in the soy meat was not sufficiently vaporized during the cooking process, and the smell of ethanol was detected when smelled close.

It could be confirmed that, even after the hydrated soy meat was rinsed with running water to wash off the excess solution from the surface and remove the minimum amount of water, and then cooked by baking in a generous amount of oil, the soy meat soaked in the hemin solution had a dark brown color similar to that of grilled beef (FIG. 44). It could be confirmed that, compared to the case where cooking was performed after sufficient removal of the solution and moisture (FIG. 43), the soy meat looked moist overall, but the overall color tone thereof was similar (FIGS. 43 and 44). Thereamong, the soy meat (No. 3) hydrated in the hemin-NaOH-water solution had a relatively bright color, and the soy meat (No. 6) soaked in the hemin-NH₃-EtOH solution after hydration in water had the darkest color (FIG. 44). In addition, the soy meats (Nos. 5 to 8) cooked after first or second soaking in ethanol had a relatively smaller volume and stiffer tissue than the soy meat not soaked in ethanol (FIG. 44). During cooking, no special smell was detected regardless of the processing method, and it was confirmed that the smell of the soy meat was not significantly different from the smell of soaked rice/grains even after cooking. However, it could be confirmed that the soy meat soaked in the hemin solution had a relatively small smell of soaked rice/grains. However, it could be confirmed that, in the case of the soy meats (Nos. 5 and 6) soaked in the ethanol solution immediately before cooking, the ethanol contained in the soy meat did not vaporize sufficiently during cooking, and thus the smell of ethanol was detected when smelled closely.

As a result of tasting a portion of the cooked soy meat without swallowing, it was evaluated that the soy meat (FIG. 44, No. 3) cooked after being soaked in the hemin-NaOH-water solution was somewhat salty upon contact with the tongue, compared to the soy meat (FIG. 44, No. 2) cooked after hydration only in water, and that the smell of smoke, which can be detected when eating grilled meat, was detected by spreading from the mouth to the nasal cavity. A similar flavor could also be detected in the soy meat (FIG. 44, No. 4) cooked after soaking in the hemin iron-NH₃-water solution, but the degree thereof was evaluated to be weaker than that of the soy meat (FIG.44, 3) cooked after hydration in the hemin-NaOH-water solution. However, there was an evaluation that the first impression of being salty was not felt. It was evaluated that the soy meats (FIG. 44, Nos. 5 and 6) soaked in ethanol before cooking had a bitter taste and a tingling sensation that lasted on the tongue due to the remaining ethanol that was not vaporized during cooking. It was evaluated that the soy meats (FIG. 44, Nos. 7 and 8) subjected to second hydration in water after soaking in the ethanol solution did not show a clear difference from the soy meat soaked only in water.

### Example 20: Increase in heme production by temporary starvation

In Example 5, fed-batch fermentation was performed as described in Example 4, but when the OD₆₀₀ reached about 70, 1 mM IPTG was added and a feeding solution (800 g/L glycerol, 8 g/L MgSO₄·7H₂O, 5 g/L (NH₄)₂SO₄, 1 g/L FeSO₄·7H₂O, 1 mM IPTG, 25 mg/L kanamycin, 50 mg/L ampicillin, 50 mg/L streptomycin, and 17 mg/L chloramphenicol) supplemented with 1 g/L FeSO₄·7H₂O was supplied, and as a result, 363 mg/L of heme was produced at 120 h (FIG. 13). As a result of stopping the supply of the feeding solution for about 1 hour between 13 h and 14 h under the same fermentation conditions, it was confirmed that heme production was increased (514.8 mg/L, at 120 h) (FIG. 45). Thereby, it was confirmed that heme production could be increased by temporarily stopping the supply of the feeding solution during fed-batch fermentation to induce starvation.

### Example 21: Increase in heme production by controlling start point of pH control

In Example 5, fed-batch fermentation was performed as described in Example 4, but when the OD₆₀₀ reached about 70, 1 mM IPTG was added and a feeding solution (800 g/L glycerol, 8 g/L MgSO₄·7H₂O, 5 g/L (NH₄)₂SO₄, 1 g/L FeSO₄·7H₂O, 1 mM IPTG, 25 mg/L kanamycin, 50 mg/L ampicillin, 50 mg/L streptomycin, and 17 mg/L chloramphenicol) supplemented with 1 g/L FeSO₄·7H₂O was supplied, and as a result, 363 mg/L of heme was produced at 120 h (FIG. 13). Under these conditions, the pH was not controlled at the beginning of fermentation, and pH control started from the time point (point B in FIG. 46) when the pH value reached about 4 and began to increase naturally. As a result, it was confirmed that heme production dramatically increased (574.5 mg/L, at 120 h) (FIG. 47).

### Example 22: Increase in heme production by increase in cell density

As a result of analyzing the results of fermentation performed in Examples 5, 6, 20 and 21, it was confirmed that, when IPTG induction was performed after the early stationary phase (OD₆₀₀ = 70 to 80), the cell density during fermentation remained at an OD₆₀₀ of about 100. Since it was considered that heme production could be increased if the cell density during fermentation was increased, R-Fe100 medium (Table 14) obtained by further adding phosphate and trace metal salts to the MR-Fe50 medium (Table 11) was applied to fermentation for heme production. The composition of the trace metal solution used to prepare the R-Fe100 medium is shown in Table 15 below.

**[Table 14]**

| **Component** | **Concentration** |
|---|---|
| Citric acid | 1.7 g/L |
| Fe-free trace metal solution for R-FeX | 5 mL/L |
| FeSO₄*7H₂O | 100 mg/L |
| KH₂PO₄ | 13.3 g/L |
| (NH₄)₂HPO₄ | 4 g/L |
| Yeast extract | 5 g/L |
| MgSO₄·7H₂O | 1.2 g/L |
| pH (using KOH) | 7.0 |

**[Table 15]**

| **Component** | **Concentration** |
|---|---|
| CaCl₂ | 2 g/L |
| ZnSO₄·7H₂O | 2.2 g/L |
| MnSO₄·5H₂O | 2.56 g/L |
| CoCl₂·6H₂O | 0.50 g/L |
| CuSO₄·5H₂O | 1 g/L |
| (NH₄)₆Mo₇O₂₄·4H₂O | 0.36 g/L |
| Na₂B₄O₇·10H₂O | 0.93 g/L |
| FeSO₄·7H₂O | 0 g/L |
| Concentrated HCl | 5 mL/L |

In order to evaluate the effect of the R-Fe100 medium on the fermentation of the HAEM7 strain, when the pH increased at about 13 h during culture performed without supplying the feeding solution [point 3 in FIG. 48(a)], pH control started, and then the supply of a feeding solution (800 g/L glycerol, 12 g/L MgSO₄·7H₂O, 25 mg/L kanamycin, 50 mg/L ampicillin, 50 mg/L streptomycin, and 17 mg/L chloramphenicol) also started, and when the OD₆₀₀ value reached about 78, 1 mM IPTG was added. Next, fermentation was performed while feeding a feeding solution (800 g/L glycerol, 12 g/L MgSO₄·7H₂O, 5 g/L (NH₄)₂SO₄, 1 g/L FeSO₄·7H₂O, 1 mM IPTG, 25 mg/L kanamycin, 50 mg/L ampicillin, 50 mg/L streptomycin, and 17 mg/L chloramphenicol) supplemented with 1 g/L FeSO₄·7H₂O, and as a result, 515.0 mg/L of heme was produced at 84 h (FIG. 49). Although this is a lower value than the concentration of heme produced in Example 21 (574.5 mg/L, 120 h, 4.79 mg/L/h, FIG. 47), 515.0 mg/L of heme was produced within a shorter time (84 h), and thus higher productivity (6.13 mg/L/h) could be achieved.

### Example 23: Increase in heme production by increasing iron concentration in feeding solution during heme fermentation with increased cell density

It was considered that, as the iron consumption increased due to the increased cell density during fermentation in Example 23, more iron supply would be required for heme production. Thus, fermentation was performed while supplying a feeding solution (800 g/L glycerol, 12 g/L MgSO₄·7H₂O, 5 g/L (NH₄)₂SO₄, 2 g/L FeSO₄·7H₂O, 1 mM IPTG, 25 mg/L kanamycin, 50 mg/L ampicillin, 50 mg/L streptomycin, and 17 mg/L chloramphenicol) supplemented with an increased concentration of FeSO₄·7H₂O (2 g/L) after IPTG addition. As a result, heme production was increased, and thus 612.6 mg/L of heme was produced at 84 h, and a productivity of 7.29 mg/L/h was achieved (FIG. 50).

### Example 24: Increase in heme production by controlling start point of pH control during heme fermentation with increased cell density

It was considered that heme production could be increased if the start point of pH control during fermentation in Example 23 was controlled. In fermentation in Example 23 in which the feeding solution was not supplied before the start of pH control, the pH, agitation, and DO profiles were obtained as shown in FIG. 48 (a), and pH control started at point 3 where the pH suddenly increased. However, it was suspected that the rapid increase in DO value and rapid decrease in agitation with the increase in the pH at point 3 could be caused by the depletion of residual glycerol in the medium. In fact, as a result of supplying a feeding solution (800 g/L glycerol, 12g/L MgSO₄·7H₂O, 25 mg/L kanamycin, 50 mg/L ampicillin, 50 mg/L streptomycin, and 17 mg/L chloramphenicol) at point 3, it was confirmed that the pH decrease continued as shown in FIG. 48(b). In addition, as a result of supplying the feeding solution whenever a rapid increase in DO was observed, the pH, agitation, and DO profile shown in FIG. 48(b) could be obtained. Then, in order to confirm the change in heme production depending on the start point of pH control, the start of pH control was attempted at the time point corresponding to points 1 to 4 in FIG. 48. As a result, as shown in FIG. 51(b), when pH control started at point 2, the largest amount of heme (934.0 mg/L, 48 h, 19.5 mg/L/h) was produced. However, in this case, about 400 g of the feeding solution was oversupplied between 32.5 h and 36 h due to an unexpected rise in the pH. Next, when pH control started at point 4, as shown in FIG. 51(d), 896.0 mg/L of heme (96 h, 9.33 mg/L/h) was produced, and when pH control started at point 3, as shown in FIG. 51(c), 778.8 mg/L/h of heme (84 h, 9.27 mg/L/h) was produced. Finally, when pH control started at point 1, as shown in FIG. 51(a), 649.9 mg/L of heme (54 h, 12.0 mg/L/h) was produced.

### Example 25: Increase in heme production by oversupply of feeding solution

In the case of the condition in which pH control started at point 2 where the largest amount of heme produced during fermentation in Example 24 [FIG. 51(b)], about 400 g of a feeding solution (800 g/L glycerol, 12 g/L MgSO₄·7H₂O, 5 g/L (NH₄)₂SO₄, 2 g/L FeSO₄·7H₂O, 1 mM IPTG, 25 mg/L kanamycin, 50 mg/L ampicillin, 50 mg/L streptomycin, and 17 mg/L chloramphenicol) was oversupplied between 32.5 h and 36 h due to an unexpected rise in the pH. In consideration of the possibility that the oversupply of the feeding solution inhibited heme production, an attempt was made to prevent oversupply of the feeding solution. However, a smaller amount of heme (775.7 mg/L, 54 h, 14.4 mg/L/h) than in Example 24 was produced (FIG. 52).

In order to re-evaluate the effect of oversupply of the feeding solution on heme production, 400 g of a feeding solution (800 g/L glycerol, 12g/L MgSO₄·7H₂O, 5 g/L (NH₄)₂SO₄, 2 g/L FeSO₄·7H₂O, 1 mM IPTG, 25 mg/L kanamycin, 50 mg/L ampicillin, 50 mg/L streptomycin, and 17 mg/L chloramphenicol) was oversupplied between 30.5 h and 33.5 h at a constant flow rate. As a result, it was confirmed that a larger amount of heme (844.9 mg/L, 60 h, 14.1 mg/L/h) than when oversupply was blocked (FIG. 52) was produced (FIG. 53).

### Example 26: Increase in heme production by oversupply of feeding solution and supply of iron solution

In order to evaluate the effect of the increase in the iron concentration in culture medium by the supply of an iron solution on heme production during fermentation, an iron solution obtained by dissolving 0.7 g of FeSO₄·7H₂O in 50 mL of 60 mM HCl aqueous solution was supplied at 15 h and 26.5 h. As a result, it was confirmed that 821.4 mg/L of heme (17.1 mg/L/h) was produced at 48 h, and at this time, about 100 g of a feeding solution (800 g/L glycerol, 12 g/L MgSO₄·7H₂O, 5 g/L (NH₄)₂SO₄, 2 g/L FeSO₄·7H₂O, 1 mM IPTG, 25 mg/L kanamycin, 50 mg/L ampicillin, 50 mg/L streptomycin, and 17 mg/L chloramphenicol) was oversupplied between 31.2 h and 32.5 h due to an unexpected rise in the pH (FIG. 54).

Since it was considered that the production of heme could be further increased if the oversupply of the feeding solution and the supply of the iron solution were performed, 400 g of a feeding solution (800 g/L glycerol, 12 g/L MgSO₄·7H₂O, 5 g/L (NH₄)₂SO₄, 2 g/L FeSO₄·7H₂O, 1 mM IPTG, 25 mg/L kanamycin, 50 mg/L ampicillin, 50 mg/L streptomycin, and 17 mg/L chloramphenicol) was oversupplied at a constant flow rate between 30 h and 33 h, and at the same time, an iron solution obtained by dissolving 0.7 g of FeSO₄·7H₂O in 50 mL of 60 mM HCl aqueous solution was additionally supplied at 24.3 h, 36.5 h, and 48.5 h. As a result, it was confirmed that 885.4 mg/L of heme (18.4 mg/L/h) was produced at 48 h (FIG. 55), indicating that the production of heme was further increased when both the oversupply of the feeding solution and the supply of the iron solution were performed.

Next, it was expected that, if the time point of the first supply of the iron solution was advanced, the total production of heme could be increased by increasing heme production in the early stage of fermentation. To test this hypothesis, 400 g of a feeding solution (800 g/L glycerol, 12 g/L MgSO₄·7H₂O, 5 g/L (NH₄)₂SO₄, 2 g/L FeSO₄·7H₂O, 1 mM IPTG, 25 mg/L kanamycin, 50 mg/L ampicillin, 50 mg/L streptomycin, and 17 mg/L chloramphenicol) was supplied at a constant flow rate between 30.5 h and 33.5 h, and at the same time, 50 mL of the iron solution was supplied at 18.7 h, 24.3 h, 36.8 h, and 48.5 h. As a result, it could be confirmed that, as hypothesized, a larger amount of heme (978.4 mg/L, 48 h, 20.4 mg/L/h) was produced (FIG. 56).

In addition, as a result of supplying 50 mL of the iron solution at 16.5 h, 24.1 h, 36.5 h, 42.5 h, 48 h, and 54.5 h (bringing the time point of the first supply of the iron solution forward) and oversupplying 400 g of a feeding solution (800 g/L glycerol, 12 g/L MgSO₄·7H₂O, 5 g/L (NH₄)₂SO₄, 2 g/L FeSO₄·7H₂O, 1 mM IPTG, 25 mg/L kanamycin, 50 mg/L ampicillin, 50 mg/L streptomycin, and 17 mg/L chloramphenicol) between 30.3 h and 33.3 h, it could be confirmed that a larger amount of heme (1,034.3 mg/L, 48 h, 21.5 mg/L/h) was produced (FIG. 57).

### Example 27: Optimization of iron concentration in medium for ZnPPIX production by fed-batch culture

In order to produce ZnPPIX by fed-batch culture of the HAEM7 strain, fermentation was performed using MR-Fe0 medium, which was confirmed to be suitable for ZnPPIX production in Example 7. First, the HAEM7 strain was inoculated into 5 mL of LB medium (10 g/L NaCl, 10 g/L tryptone, and 5 g/L yeast extract) supplemented with 25 mg/L kanamycin, 50 mg/L ampicillin, 50 mg/L streptomycin, 17 mg/L chloramphenicol and 10 g/L glucose, and then cultured at 37°C and 200 rpm for 12 hours. In order to obtain the HAEM7 inoculum for inoculating the fermenter, 0.4 mL of the culture in which the cultured HAEM7 cells were growing was inoculated into 200 mL of a freshly prepared MR-Fe0 medium (Table 8) supplemented with 25 mg/L kanamycin, 50 mg/L ampicillin, 50 mg/L streptomycin, 17 mg/L chloramphenicol, and 20 g/L glycerol, and then cultured in a 1-L Erlenmeyer flask at 37°C and 200 rpm for 12 hours. Next, the culture was inoculated into a DasGip parallel bioreactor system (Eppendorf AG, Hamburg, Germany) equipped with a 1.3-L vessel containing 0.27 L of MR-Fe0 medium (Table 8) supplemented with 25 mg/L kanamycin, 50 mg/L ampicillin, 50 mg/L streptomycin, 17 mg/L chloramphenicol and 20 g/L glycerol at 30°C with agitation at 200 rpm in an air saturated state by air supply at a flow rate of 0.3 L/min. During fermentation, the temperature was maintained at 30°C, and the pH was maintained at 7.0 using a solution obtained by diluting a saturated ammonia solution in the same volume of water [-14% (w/v) NH₄OH]. In addition, the air saturation was maintained at 40% of the initial air saturation by increasing the agitation speed up to 1,000 rpm and increasing the O₂ partial pressure in the supplied air to 100%. In addition, for continuous supply of glycerol during fed-batch culture, a feeding solution (800 g/L glycerol, 8 g/L MgSO₄7H₂O, 25 mg/L kanamycin, 50 mg/L ampicillin, 50 mg/L streptomycin, and 17 mg/L chloramphenicol) was supplied using a pH-stat technique. In addition, after IPTG addition, a feeding solution (800 g/L glycerol, 8 g/L MgSO₄7H₂O, 5 g/L (NH4)₂SO₄, 1 g/L ZnSO₄·7H₂O, 1 mM IPTG, 25 mg/L kanamycin, 50 mg/L ampicillin, 50 mg/L streptomycin, 17 mg/L chloramphenicol) supplemented with 5 g/L (NH₄)₂SO₄, 1 g/L ZnSO₄·7H₂O and 1 mM IPTG was supplied. Sampling was conducted at 12-hour intervals to analyze the growth curve and heme, PPIX, and zinc-protoporphyrin IX (ZnPPIX) production. The growth curve was analyzed by measuring the OD₆₀₀ value, and the production of heme, PPIX, and ZnPPIX was analyzed using the method described in Example 1.

As a result, as shown in FIG. 58(a), it was confirmed that the HAEM7 strain did not grow normally. It was determined that cell growth did not occur properly because the concentration of iron contained in the MR-Fe0 medium was insufficient to reach high cell density during the fed-batch culture process. Thus, fed-batch culture was performed using each of MR-Fe10, MR-Fe20 (Table 10), and MR-Fe50 media (Table 11) having increased FeSO₄·7H₂O concentrations of 10, 20, and 50 mg/L, respectively. As a result, it was confirmed that, when the MR-Fe10 and MR-Fe20 media were used, cell growth was still inhibited [FIG. 58(b) and 58(c)], but when the MR-Fe50 medium was used, cell growth was restored to normal levels [FIG. 58(d)]. In addition, it was confirmed that 989.0 mg/L of ZnPPIX was produced at 132 h.

### Example 28: Increase in ZnPPIX production by increasing zinc concentration in feeding solution

In order to improve the ZnPPIX production performance of the HAEM7 strain using MR-Fe50 medium, which was determined to be suitable for fed-batch culture for ZnPPIX production in Example 27, an attempt was made to increase the zinc concentration in the feeding solution. As a result of increasing the ZnSO₄·7H₂O concentration in the feeding solution, supplied after IPTG addition, to 2 g/L (800 g/L glycerol, 8 g/L MgSO₄7H₂O, 5 g/L (NH₄)₂SO₄, 2 g/L ZnSO₄·7H₂O, 1 mM IPTG, 25 mg/L kanamycin, 50 mg/L ampicillin, 50 mg/L streptomycin, and 17 mg/L chloramphenicol) or 4 g/L (800 g/L glycerol, 8 g/L MgSO₄7H₂O, 5 g/L (NH₄)₂SO₄, 4 g/L ZnSO₄·7H₂O, 1 mM IPTG, 25 mg/L kanamycin, 50 mg/L ampicillin, 50 mg/L streptomycin, and 17 mg/L chloramphenicol), as shown in FIGS. 58(e) and 58(f), it could be confirmed that the production of ZnPPIX increased as the zinc concentration increased. When the feeding solution was supplemented with 2 g/L ZnSO₄·7H₂O, 1441.6 mg/L of ZnPPIX was produced [FIG. 58(e)], and when the feeding solution was supplemented with 4 g/L ZnSO₄·7H₂O, 1615.7 mg/L of ZnPPIX was produced [FIG. 58(f)].

Furthermore, in order to confirm the ZnPPIX production ability in fed-batch culture with an increased standard, fed-batch culture of the HAEM7 strain was performed using MR-Fe50 medium according to the method described in Example 4. However, pH control started at a point when the pH value increased (point B in FIG. 46), and a feeding solution (800 g/L glycerol, 8 g/L MgSO₄7H₂O, 5 g/L (NH4)₂SO₄, 4 g/L ZnSO₄·7H₂O, 1 mM IPTG, 25 mg/L kanamycin, 50 mg/L ampicillin, 50 mg/L streptomycin, and 17 mg/L chloramphenicol) supplemented with 4 g/L ZnSO₄·7H₂O was supplied after IPTG addition. As a result, as shown in FIG. 59, 1597.9 mg/L of ZnPPIX (120 h, 13.3 mg/L/h) was produced.

### Example 29: Increase in ZnPPIX production by increasing cell density

Based on the fact that heme production was increased by increasing cell density in Examples 22, 23 and 24, it was considered that ZnPPIX production could also be increased by increasing cell density. However, since iron contained excessively in the medium can promote the production of heme and inhibit the production of ZnPPIX, fed-batch culture was performed using R-Fe50 medium having a FeSO₄·7H₂O content lower by 50 mg/L than the R-Fe100 medium (Table 14). In addition, pH control started at point 2 (FIG. 48), and a feeding solution (800 g/L glycerol, 12 g/L MgSO₄7H₂O, 5 g/L (NH4)₂SO₄, 4 g/L ZnSO₄·7H₂O, 1 mM IPTG, 25 mg/L kanamycin, 50 mg/L ampicillin, 50 mg/L streptomycin, and 17 mg/L chloramphenicol) supplemented with 4 g/L ZnSO₄·7H₂O, 1 mM IPTG, and 5 g/L (NH₄)₂SO₄ was supplied after IPTG addition. As a result, as shown in FIG. 60, 1,547.2 mg/L of ZnPPIX (54 h, 28.7 mg/L/h) was produced. This is a value that was not increased compared to the ZnPPIX production obtained in Example 28 in which fed-batch culture was performed using MR-Fe50 medium. Although 1547.2 mg/L of ZnPPIX was produced within a shorter time (54 h) and relatively high productivity (28.7 mg/L/h) was achieved, it was considered that fermentation conditions would need to be improved to increase ZnPPIX production.

Based on the fact that the production of ZnPPIX decreased significantly at around 36 h (FIG. 60), depletion of residual iron in the fermentation medium was suspected. Therefore, fed-batch fermentation was performed using R-Fe100 medium (Table 14) containing more iron than R-Fe50 medium. As a result of supplying a feeding solution (800 g/L glycerol, 12 g/L MgSO₄7H₂O, 5 g/L (NH4)₂SO₄, 4 or 8 g/L ZnSO₄·7H₂O, 1 mM IPTG, 25 mg/L kanamycin, 50 mg/L ampicillin, 50 mg/L streptomycin, and 17 mg/L chloramphenicol) supplemented with 4 g/L or 8 g/L ZnSO₄·7H₂O after IPTG addition, as shown in FIG. 61, it could be confirmed that the production of ZnPPIX was dramatically increased. More specifically, when the feeding solution was supplemented with 4 g/L ZnSO₄·7H₂O, 2,179.2 mg/L of ZnPPIX (40.4 mg/L/h) within 54 h or 2,195.5 mg/L of ZnPPIX (36.6 mg/L/h) within 60 h was produced [FIG. 61(a)], and when the feeding solution was supplemented with 8 g/L ZnSO₄·7H₂O, 2,154.1 mg/L of ZnPPIX (39.9 mg/L/h) was produced within 54 h [FIG. 61(b)].

### Example 30: Preparation of TSP- and tofu-based vegetable patties supplemented with heme and ZnPPIX

Vegetable patties were prepared to evaluate the color and flavor after cooking of foods supplemented with heme and ZnPPIX unbound to peptides and proteins. To make vegetable patties, soy meat (Hyokyung Tech) made of texturized soy protein (TSP) was minced, and tofu, seasoned salt, cooking wine, soy sauce, minced garlic, green onion, flour, grapeseed oil, sesame oil, etc. were added thereto, thus preparing patty dough. Then, to add heme and ZnPPIX to vegetable patties, about 17 mg of purified hemin reagent (>96%, BioXtra, Sigma-Aldrich) or about 16 mg of ZnPPIX reagent (>92%, Sigma-Aldrich) was suspended in 2 mL of 50 mM NaOH aqueous solution and added to 100 g of the patty dough. As a negative control, 2 mL of 50 mM NaOH aqueous solution in which nothing was suspended was added to the same amount of the dough, and as a positive control, 100 mg of hemoglobin (from bovine blood, Sigma-Aldrich), a kind of heme-containing protein (well known to improve color and flavor when added to plant-based meat substitutes), was suspended in 50 mM NaOH solution and added to the dough. As a result, FIG. 62 shows the appearances before cooking of the negative control group (No. 1), the positive control group to which hemoglobin was added (No. 2), the experimental group to which heme was added (No. 3), and the experimental group to which ZnPPIX was added (No. 4). The negative control group had a light yellow color, whereas the dough supplemented to which hemoglobin or heme was added had a more grayish brown than the negative control group, and was more reddish than the dough to which ZnPPIX was added.

The appearances of the above doughs after being cooked on low heat are shown in FIG. 63, and the negative control group had a color similar to that of tofu pancakes or Korean-style meatballs (1 in FIG. 63). The vegetable patty to which hemoglobin was added had a light brown color after cooking (2 in FIG. 63), and the vegetable patty to which heme was added had a darker brown color similar to that of patties with actual beef and pork added (3 in FIG. 63). The vegetable patty to which ZnPPIX was added was more reddish brown like undercooked beef (4 in FIG. 63). When the above vegetable patties were tested, a flavor similar to that of Korean-style meatballs without added meat was detected in the negative control group, and the flavor of animal patties was detected in the vegetable patty to which hemoglobin, a kind of heme-containing protein well known to help mimic the color and flavor of meat when added to vegetable patties, was added. In the vegetable patty to which heme was added, the flavor of animal patties was detected more strongly than that in the vegetable patty to which hemoglobin was added, and in the vegetable patty to which ZnPPIX was added, the flavor of animal patties was insignificant.

### Example 31: Preparation of vegetable patties based on pressed tofu with heme added

In order to evaluate color and flavor after adding heme unbound to peptides and proteins to various plant-based foods and cooking the foods, vegetable patties were prepared by pressing tofu. In order to improve the texture of vegetable patties, tofu was heated and pressed to remove water equivalent to 50% or more of the original mass of the tofu, and minced, and then minced garlic, minced green onion, seasoned salt, cooking wine, sesame oil, flour, sugar, and pepper were added thereto, thus preparing a dough. Then, in order to add heme to the dough, about 10 mg of purified hemin reagent (>96%, BioXtra, Sigma-Aldrich) was suspended in 50 mM NaOH aqueous solution, or about 10 mg or 18.5 mg of purified hemin reagent (>96%, BioXtra, Sigma-Aldrich) was suspended in 1.5 mL of 2.8% (w/v) aqueous ammonia, and the suspension was added to 50 g of the vegetable patty dough. As a negative control, 1.5 mL of 50 mM NaOH aqueous solution or 2.8% (w/v) aqueous ammonia in which nothing was suspended was added to the same amount of the dough, and as a positive control, 100 mg of hemoglobin (from bovine blood, Sigma-Aldrich), a kind of heme-containing protein (well known to improve color and flavor when added to plant-based meat substitutes), was suspended in 1.5 mL of 2.8% aqueous ammonia and added to the dough. Thereafter, 0.5 mL of vinegar was added for neutralization, and then grapeseed oil was additionally added. As a result, FIG. 64 shows the appearances before cooking of the negative control group to which NaOH aqueous solution was added (No. 1), the experimental group to which 10 mg heme suspended in NaOH aqueous solution was added (No. 2), the negative control group to which aqueous ammonia was added (No. 3), the experimental group to which 10 mg heme suspended in aqueous ammonia was added (No. 4), the experimental group to which 18.5 g heme suspended in aqueous ammonia was added (No. 5), and the experimental group to which 100 mg hemoglobin suspended in aqueous ammonia was added (No. 6). The negative control group had a light yellow color, whereas the dough to which hemoglobin or heme was added had a darker grayish brown than the negative control group and was more reddish than the dough to which ZnPPIX was added.

The appearances of the above doughs after being cooked using an air fryer are shown in FIG. 65, and the two negative controls had a color similar to that of cookies (1 and 3 in FIG. 64). The vegetable patties to which 10 mg of heme was added had a dark brown color after cooking (2 and 4 in FIG. 64), and the vegetable patty to which 18.5 mg of heme was added had a darker color (5 in FIG. 64). The vegetable patty to which 0.1 g of hemoglobin was added had a lighter color lighter than that of the vegetable patty to which 10 mg of heme was added (6 in FIG. 64). When the above vegetable patties were tasted, they had a texture similar to that of animal patties, and there was no significant difference between the use of NaOH aqueous solution and the use of aqueous ammonia in the experimental groups and the control groups. In the negative control group, a flavor similar to that of tofu or dewatered bean curd dregs (kong biji) was detected, but in vegetable patties to which 10 mg of heme was added, the flavor of meat was detected without the flavor of tofu or bean curd soup, and in the vegetable patty to which 18.5 mg of heme was added, a stronger beef flavor was detected. On the other hand, in the vegetable patty to which 100 mg of hemoglobin was added, the flavor of meat was initially detected, but the flavor of dewatered bean curd dregs was detected in the aftertaste.

### Example 32: Preparation of dried tofu and tofu-based vegetable patties with heme added

In order to evaluate color and flavor after adding heme unbound to peptides and proteins to various plant-based foods and cooking the foods, vegetable patties were prepared by pressing tofu. In addition, the present inventors tested whether the same effect as the addition of high-purity hemin reagent can be obtained when heme samples extracted from microorganisms containing relatively low-purity heme are added. However, in the case of heme samples extracted from microorganisms, a safety verification procedure is required to add the samples to food, and thus a commercially available heme supplement containing relatively low-purity heme was added instead of the sample, thus preparing plant-based meat substitutes. However, in order to minimize the effects of unnecessary additives, among commercially available heme supplement products, products produced using only heme raw materials themselves obtained through pretreatment such as hydrolysis and drying of hemoglobin without adding additives such as vitamins were selected.

The heme supplement product was dissolved in a solution obtained by mixing 28% (w/v) aqueous ammonia and ethanol at a volume ratio of 1:9, and then the heme content in the product was analyzed using HPLC. As a result, it was confirmed that the mass percentage of heme therein was about 7.9%. In order to improve the texture of vegetable patties, tofu was heated to remove water equivalent to about 33% of the original tofu mass, and then mashed, and heme supplement powder was added thereto in an amount of 0 mg, 860 mg, 1,720 mg or 2,580 mg per 1,100 g of the mass of the final dough. Thereafter, 450 g of shredded tofu or tofu noodles were added, and seasoned salt, sugar, cooking wine, minced green onion, minced garlic, pepper, flour, sesame oil, and grapeseed oil were added, thus preparing a dough. The prepared dough was subdivided into about 50 g each and pressed to a thickness of about 10 mm. The prepared dough was subdivided into pieces each having a weight of about 50 g, and then pressed to a thickness of about 10 mm. FIG. 66 shows the appearances of the vegetable doughs to which the heme supplement powder was added in an amount of 0 mg (0 mg heme/kg vegetable dough; No. 1), 860 mg (about 62 mg heme/kg vegetable dough; No. 2), 1,720 mg (about 124 mg heme/kg vegetable dough; No. 3), or 2,580 mg (about 185 mg heme/kg vegetable dough; No. 4). The negative control group (1 in FIG. 68) having a heme content of 0 mg/kg had a grayish white color, whereas the grayish brown color similar to that of cooked pork and beef gradually deepened as the heme content increased.

FIG. 67 shows the appearances of the above doughs after being cooked by baking on low heat using a frying pan with oil. The negative control had a color similar to that of Korean-style meatballs (1 in FIG. 67). The vegetable patty to which about 62 mg/kg of heme was added had a yellowish brown color after cooking (2 in FIG. 67), and the vegetable patty to which about 124 mg/kg of heme was added had a dark brown color (3 in FIG. 67), and the vegetable patty to which about 185 mg/kg of heme was added had a darker brown color (4 in FIG. 67). When the above vegetable patties were tasted, they had a texture similar to that of animal patties. In the negative control group, a flavor similar to that of Korean-style meatballs was detected, but in the vegetable patties to which heme was added, the flavor of meat was detected, and a stronger meat flavor was detected as the heme content increased.

### Industrial Applicability

According to the present invention, it is possible to improve the flavor, nutrition, and color of food by producing heme and various porphyrins, metalloporphyrins, and porphyrin analogs with high efficiency by fermentation of a microbial mutant, isolating/purifying the products, and adding the isolated/purified products to food.

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

## Claims

1. A method for producing a compound selected from the group consisting of heme, porphyrins, metalloporphyrins, and porphyrin analogs, the method comprising a step of producing a compound selected from the group consisting of heme, porphyrins, metalloporphyrins, and porphyrin analogs by culturing a heme-producing microorganism having a heme synthesis pathway gene in a medium containing a carbon source.

2. The method according to claim 1, wherein expression of the heme synthesis pathway gene is induced when an OD₆₀₀ value of the culture reaches 10 to 80 during the culturing.

3. The method according to claim 1, wherein a concentration of iron ions in the medium is 0.2 to 6 mM.

4. The method according to claim 1, wherein the production of zinc-protoporphyrin (ZnPPIX) among metalloporphyrins is increased by reducing a concentration of iron ions in the medium to 0.15 mM or less.

5. The method according to claim 1, wherein the production of zinc-protoporphyrin (ZnPPIX) among metalloporphyrins is increased by increasing a concentration of zinc ions in the medium to 0.04 mM or more.

6. The method according to claim 1, wherein the production of the compound selected from the group consisting of heme, porphyrins, metalloporphyrins, and porphyrin analogs is increased by supplying a feeding solution containing a carbon source so that the residual carbon source concentration in the medium is 10 g/L or more.

7. The method according to claim 6, wherein the carbon source is glycerol.

8. The method according to claim 6, wherein the feeding solution contains iron.

9. The method according to claim 1, wherein the production of the compound selected from the group consisting of heme, porphyrins, metalloporphyrins, and porphyrin analogs is increased by adjusting the residual carbon source concentration in the medium to 0 g/L.

10. The method according to claim 9, wherein a time period when the residual carbon source concentration in the medium is 0 g/L is 10 minutes or longer.

11. The method according to claim 1, wherein the production of zinc-protoporphyrin (ZnPPIX) among metalloporphyrins is increased by increasing a concentration of zinc ions in a feeding solution containing a carbon source to 3 mM or more.

12. The method according to claim 1, wherein the production of the compound selected from the group consisting of heme, porphyrins, metalloporphyrins, and porphyrin analogs is increased by increasing concentrations of phosphate ions and trace metals in the medium so that a final cell density is increased to a cell density corresponding to an OD₆₀₀ of 90 or higher.

13. The method according to claim 1, wherein the production of the compound selected from the group consisting of heme, porphyrins, metalloporphyrins, and porphyrin analogs is increased by delaying a start point of pH control to after a point at which an instantaneous change rate of pH increases or decreases by 30% or more compared to an average change rate for 10 minutes.

14. The method according to claim 13, wherein the start point of pH control is delayed to after 4 hours after inoculation.

15. The method according to claim 1, further comprising steps of:
suspending the microorganism, which contains the produced heme, porphyrins, metalloporphyrins and porphyrin analogues, in water; and
extracting the compound selected from the group consisting of heme, porphyrins, metalloporphyrins, and porphyrin analogs by adding a basic alcohol solution to the suspension.

16. The method according to claim 15, wherein the extracting is performed by suspending cells, collected from a fermentation broth of the heme-producing microorganism, in water, and adding water and the basic alcohol solution at a volume ratio of 2:8 to 4:6.

17. The method according to claim 15, wherein the basic alcohol solution is a solution of NH₃ in ethanol (NH₃-EtOH), a solution of KOH in ethanol (KOH-EtOH), a solution of NaOH in ethanol (NaOH-EtOH), or a solution of NaOH in methanol (NaOH-MeOH).

18. The method according to claim 15, further comprising a step of precipitating the compound selected from the group consisting of heme, porphyrins, metalloporphyrins, and porphyrin analogs by adding an acidic substance.

19. The method according to claim 18, wherein the acidic substance is acetic acid or hydrochloric acid.

20. The method according to claim 1, wherein dissolution of heme, porphyrins, metalloporphyrins and porphyrin analogs into a supernatant is promoted by increasing a pH of a fermentation broth containing the produced heme, porphyrins, metalloporphyrins, and porphyrin analogs to 8 or higher.

21. The method according to claim 1, further comprising steps of:
separating a supernatant containing the produced heme, porphyrins, metalloporphyrins, and porphyrin analogs; and
precipitating the heme, porphyrins, metalloporphyrins, and porphyrin analogs by adding an acidic substance.

22. The method according to claim 21, wherein the acidic substance is acetic acid or hydrochloric acid.

23. The method according to claim 18, wherein, after the compound selected from the group consisting of heme, porphyrins, metalloporphyrins, and porphyrin analogs is precipitated, the compound is suspended and dissolved in basic aqueous solution and a basic alcohol solution for addition to food.

24. The method according to claim 23, wherein the basic aqueous solution is an aqueous NH₃ solution, an aqueous NaOH solution, or an aqueous KOH solution.

25. A food having a flavor similar to that of meat, the food containing:
a compound selected from the group consisting of heme, porphyrins, metalloporphyrins and porphyrin analogs, obtained by fermentation of a heme-producing microorganism; or
an edible microorganism having an ability to produce the compound selected from the group consisting of heme, porphyrins, metalloporphyrins, and porphyrin analogs.
